(19) Europäisches Patentamt
European Patent Office
Office européen des brevets

(11) EP 4 455 667 B1

(12) **EUROPEAN PATENT SPECIFICATION**

(45) Date of publication and mention of the grant of the patent:
**13.08.2025 Bulletin 2025/33**

(21) Application number: **24172222.2**

(22) Date of filing: **24.04.2024**

(51) International Patent Classification (IPC):
***G01N 33/543*** *(2006.01)* ***G01N 33/94*** *(2006.01)*

(52) Cooperative Patent Classification (CPC):
**G01N 33/54388; G01N 33/946**

(54) **DEVICE FOR DETECTING MOLECULES**

VORRICHTUNG ZUM NACHWEIS VON MOLEKÜLEN

DISPOSITIF DE DÉTECTION DE MOLÉCULES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **25.04.2023 LU 504068**

(43) Date of publication of application:
**30.10.2024 Bulletin 2024/44**

(73) Proprietor: **Viscera Technologies Ltd**
**London W1G 8TB (GB)**

(72) Inventors:
- **HOLLAMBY, Henry**
  **London, W1G 8TB (GB)**
- **QUIGLEY, Ryan**
  **London, SW6 3LF (GB)**

(74) Representative: **Marks & Clerk LLP**
**15 Fetter Lane**
**London EC4A 1BW (GB)**

(56) References cited:
**EP-A1- 0 203 238 WO-A1-03/105899**

- **SHU HWANG ANG ET AL: "A colloidal gold-based lateral flow immunoassay for direct determination of haemoglobin A1c in whole blood", ANALYTICAL METHODS, vol. 7, no. 9, 1 January 2015 (2015-01-01), GB, pages 3972 - 3980, XP055275230, ISSN: 1759-9660, DOI: 10.1039/C5AY00518C**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 455 667 B1

## Description

Field of Invention

The present invention relates to methods and devices for conducting lateral flow tests, and to kits including the devices. In particular, the devices and methods are for conducting quantitative lateral flow tests.

Background

Lateral flow tests (LFTs) can be used to detect a large range of molecules and are useful for rapid diagnosis of diseases. A LFT is typically made up of a nitrocellulose membrane with biological molecules immobilised at different zones (Figure 1a). The sample is applied onto a sample pad, dissolves the deposited conjugate and migrates along the membrane by capillary action. As the sample migrates along the membrane, the conjugate or target analyte will be captured by binding molecules (typically proteins) at the test region (e.g. a test line), resulting in a visible coloured line that indicates the presence or absence of the target analyte in the sample.

There are several types of LFT and the two most common types are sandwich assays and competitive assays. In sandwich assays, the conjugate pad comprises mobilisable labelled conjugate molecules, which comprise an antibody specific for the target analyte conjugated to a detectable label, the test line comprises immobilised antibodies specific for the target analyte, and the control line comprises immobilised secondary antibodies which bind the detectable conjugate (Figure 1a). A test sample is applied to the sample pad of the test device, and target analyte in the test sample form an analyte-conjugate complex with the detectable conjugate mobilisable on the conjugate pad. The analyte-conjugate complex moves from the conjugate pad to the test line, where the analyte-conjugate complex is caught by the anti-analyte antibodies immobilised thereon, thereby producing a visible line indicative of a positive response (Figure 1b). Any detectable conjugate which has not bound the target analyte binds the control line (Figure 1b). However, sandwich assays are useful only for large analyte molecules which have two or more binding sites, allowing the binding of both the conjugate and the anti-analyte antibody at the first test region. Sandwich assays are not suitable for small molecules, which do not permit the simultaneous binding of two other molecules.

Competitive assays are designed to work with small molecules, which have only one binding site. In competitive assays, the conjugate pad comprises a labelled conjugate which specifically binds the target analyte in the test sample, and the test line comprises an immobilised capture molecule specific for the labelled conjugate. When a test sample which does not contain target analyte is applied to the sample pad of the test device, the labelled conjugate flows up the membrane and binds the test line, thereby producing a detectable signal at the test line. When target analyte is present in a test sample, the labelled conjugate binds the target analyte, which inhibits the binding of the labelled conjugate to the immobilised capture molecule of the test line. Thus, presence of target analyte in the sample is indicated by the absence of a detectable signal at the test line.

Both sandwich and competitive LFTs have a control line, after the test line in the direction of flow of the sample, which indicates that flow of the labelled conjugate has occurred correctly. Thus, the control line always produces a detectable signal in a functioning, viable test, regardless of the result of the test. In other words, the signal intensity of the control line is independent of the signal intensity of the test line, and serves only to indicate that the LFT is viable.

Both types of LFT can be used to provide a quantitative result, to quantify the concentration of analyte in the test sample. For example, Insudex® uses a sandwich assay and quantitation is achieved by the intensity of the visible line at the first test region. A greater intensity indicates a higher concentration of analyte in the sample. The user must use a special reader to analyse the test line once it has developed.

US 8,137,984 discloses a competitive LFT device for detecting the presence of caffeine. The device uses a conjugate which comprises an anti-caffeine antibody and a second antibody conjugated to a particle, and a test line which comprises a molecule which binds to the anti-caffeine antibody. Thus, the molecule on the first test line competes with caffeine in the test sample for binding to the anti-caffeine antibody. The device includes a control line which comprises an antibody which binds the second antibody of the conjugate.

US 2022/0146507 discloses a semi-quantitative, barcode-type sandwich LFT device, which has two test lines, for the detection of β-trace protein (βTP). Each test line comprises binding moieties which bind a complex formed between βTP and a detectable conjugate, wherein the detectable conjugate comprises a detectable label and a βTP-binding molecule (e.g. an antibody).

One disadvantage of a competitive LFT is that the signal intensity observed is inversely proportional to the concentration of the target analyte present (Delmulle et al., J. Agric. Food

Chem., 2005, 53, 3364-3368). This means that a detectable signal is present for all but the most concentrated samples. This causes confusion in the interpretation of the results, requires extensive calibration for accurate quantitative results and results in poor sensitivity. In addition, the negative readout associated with the competitive format implies that the presence of a target analyte is detected in the least sensitive region of the dose response curve (i.e. near saturation (i.e.

maximum signal intensity) of the test line). This makes it difficult to increase the sensitivity of a competitive LFT.

Both types of LFTs (i.e. sandwich assays and competitive assays) have the disadvantage that, when used as a quantitative assay, the first test region can become saturated. In a sandwich assay, this occurs when the immobilised antibodies of the first test region become saturated with conjugated analyte, when the analyte in the test sample is present at concentrations greater than the amount of immobilised antibody. This means that, above a certain amount of test sample analyte, the signal intensity of the first test region, indicating the presence of the test sample analyte, cannot increase further. In competitive assays, saturation occurs when the amount of target analyte in the sample is the same or greater than the amount of immobilised analyte on the test line.

One solution to prevent saturation is to increase the range of concentrations that the lateral flow test can measure, by increasing the amount of antibody immobilised at the first test region in a sandwich LFT. However, this negatively impacts the quantitative resolution of the LFT.

In particular, in both sandwich and competitive LFTs, quantitation is achieved by deducing the concentration of the analyte in the test sample from the visual response produced by the conjugate (i.e. a second degree of inference). However, an increase in the range of detection of the LFT (i.e. increasing the amount of antibody immobilised at the first test region of a sandwich assay) means that any change in the visual response will account for a smaller percentage of the visual response. For example, one analyte-conjugate complex binding to a first test region having 100 antibodies in a sandwich assay will cause a 1% change in the intensity of the visible line at the first test region. However, one analyte-conjugate complex binding to a first test region having 1000 antibodies will cause a 0.1% change in intensity of the visible line. Thus, an increase in the detection range of a quantitative lateral flow test is accompanied by a decrease in the resolution, and therefore, the accuracy, of the lateral flow test.

Quantitative LFTs are also subject to errors in reading the intensities of the visible lines produced by binding, or absence of binding, of the conjugate at the first test region. In particular, errors can arise due to deterioration of the antibodies used in the assay, which leads to sandwich LFTs being skewed towards a negative result and competitive LFTs being skewed towards a positive result, due to antibodies conjugated to the detectable label failing to bind to the test line. There can also be systemic errors in the LFT reader, or can arise due to ambient light affecting the measured signal intensity at the test region. For example, Insudex® detects C-peptide (for the diagnosis of diabetes) in a range of 0.17ng/ml to 12ng/ml, and begins experiencing the hook effect - that is, the phenomenon which causes falsely low results when analytes are present in very high concentrations - at levels of C-peptide of about 80ng/ml. When measuring concentrations of C-peptide of about 0.17ng/ml, Insudex® has a variability of about 0.02ng/ml, which is equivalent to a coefficient of variation of about 11%, which means that quantitation readings could lead to misdiagnosis, and there is a limit of the ability of the product to be used in mass screening.

A further disadvantage of competitive LFTs is that their quantitative capabilities are severely limited by the presence of the control line. The presence of the control line means that excess labelled conjugate must be added to the sample, in order for sufficient labelled conjugate to flow to the control line. However, this means that information about the amount of conjugate not binding to the test line is lost, thereby impeding the test from being able to accurately quantify outlying concentrations of the target analyte.

While ELISA tests can be performed to quantify a target analyte in a test sample, ELISAs take a substantial amount of time, and are costly and require specialist equipment and personnel to conduct.

Le *et al.* ("Dual recognition element lateral flow assay toward multiplex strain specific influenza virus detection", Anal. Chem, 2017, 89, 6781-6786) discloses a lateral flow test for detecting specific strains of the influenza virus, which comprises a nucleic acid aptamer with an antibody. The lateral flow test device has a first test line comprising streptavidin and a control line comprising an anti-mouse antibody. An anti-virus antibody is conjugated to a gold nanoparticle, while an aptamer is conjugated to biotin. The aptamer can be chosen to be highly strain specific. When the test sample contains the target virus, both the aptamer and the conjugated antibody bind to the virus, and the biotin conjugated to the aptamer enables the complex to bind the streptavidin on the test line. The gold nanoparticles can then be detected on the test line. In both the absence and presence of the target virus, the gold nanoparticle-conjugated antibody binds the anti-mouse antibody on the control line. However, this LFT is not quantitative.

WO 03/105899 discloses a lateral flow strip test for the detection and diagnosis of cerebrospinal fluid (CSF) leakage and associated conditions using antibodies that specifically bind to CSF proteins. The conjugate release pad contains a detector antibody conjugated to a detectable reagent.

Ang et al. ("A colloidal gold-based lateral flow immunoassay for direct determination of haemoglobin Alc in whole blood", Analytical Methods, 1 January 2015, vol. 7, no. 9, p.3972-3980) discloses a sandwich lateral flow test for detecting haemoglobin A1c (HbA1c). HbA1c is captured at the test line due to the immunoreaction with anti-HbA1c antibodies. Subsequently, colloidal gold-functionalised anti-haemoglobin antibodies generate visible test lines.

EP 0203238 discloses an apparatus for use in semi-quantitatively determining the presence of a test ligand, by comparing the amount of a detectable label at the test pad with the amount of a detectable label at a reference pad, to provide a relative concentration of the test ligand.

Thus, there is a need to provide quantitative lateral flow tests which provide more accurate quantification of a

target analyte in a sample. In particular, there is a need to provide quantitative LFTs which address the problems of saturation and resolution, and which reduce error in reading signal intensities. In addition, such LFTs would reduce the need for ELISA testing kits, which, as mentioned above, are costly and time-consuming.

## Summary of Invention

The present invention arises from the surprising finding that a more accurate quantitative or semi-quantitative lateral flow test can be achieved by pre-loading a lateral flow test with a known amount of a detectable conjugated analyte, to allow for calculation of the concentration of a target analyte based on a signal produced by the detectable conjugated analyte, and by having a first test region to which both the conjugated analyte and target analyte molecules can bind, and a second test region to which only the conjugated analyte can bind. The detectable conjugated analyte comprises an analyte molecule conjugated to a detectable label. The number of binding sites at the second test region is the same as or greater than the number of molecules of conjugated analyte. In the absence of target analyte molecules in the test sample, the conjugated analyte binds to the first test region, and optionally the second test region, to produce a detectable signal at the test regions to which the conjugated analyte has bound. When a target analyte molecule is present in the test sample, competition between the target analyte molecule and the conjugated analyte reduces the amount of conjugated analyte which binds to the first test region, thereby reducing the detectable signal at the first test region. The unbound conjugated analyte (i.e. not bound to the first test region) can then bind to the second test region, thereby increasing the intensity of a detectable signal (either increasing from non-existence or increasing the intensity above the signal intensity in the absence of target analyte) at the second test region. Thus, the competition between target analyte molecules in a sample and conjugated analyte pre-loaded into the device means that the signal intensity at the first test region is inversely related to the signal intensity at the second test region (i.e. the signal intensities of the first and second test regions are dependent on one another), such that the response at the test regions is graded against the number of conjugated analyte. The signal intensity of the second test region thus helps to confirm the signal intensity of the first test region.

The present invention also arises because it has been surprisingly found that a more accurate quantitative lateral flow test can be achieved when the signal intensity at the first test region is inversely related to the signal intensity of the second test region. In particular, the signal intensities at each of the first and second test regions can be compared to produce a ratio of signal intensities, which can be used to more accurately calculate the concentration of target analyte in the sample. This ratio also means that systemic errors caused by the device used to read the signal intensity of the test regions are reduced, as well as errors due to ambient light. In contrast, conventional quantitative LFTs consider only the presence or absence of a detectable signal at the first test region when calculating the concentration of target analyte in a sample, but not a detectable signal at the second test region. The second test region, in conventional LFTs, is used only as a control, to show that the conjugate thereof is functional and has migrated up the test strip. However, in the lateral flow test device of the present invention, the change in signal intensity is measured at both the first test region and the second test region, thereby providing two parameters for the calculation of the concentration of target analyte in the sample.

The present invention also arises because it has been surprisingly found that a more accurate quantitative lateral flow test can be achieved by providing a ratio of the signal intensity of the first test region to the signal intensity at the second test region. In particular, by calculating the ratio of signal intensities of the first and second test regions, and using this ratio to calculate the concentration of target analyte in the sample, particularly when the sum of the signal intensities of the first and second test regions is considered to be 100%, errors due to background ambience can be reduced. In addition, if the ratio of signal intensities is calculated more than once, and the multiple ratios are used to calculate an average ratio, the errors are even further reduced.

Errors in quantitation due to ambient light can be also reduced by comparing an average ratio of signal intensities of the first and second test regions calculated based on predicted signal intensities with a ratio of signal intensities of the first and second test regions which have been measured. In particular, the signal intensity of the first test region can be measured, and then used to predict the signal intensity of the second test region on the basis that the sum of the signal intensities is 100% (e.g. of a maximum signal intensity), and a first ratio of signal intensities of the first and second test regions is determined. The signal intensity of the second test region is then measured, and used to predict the signal intensity of the first test region, on the same basis, and a second ratio of signal intensities of the first and second test regions is determined. An average ratio of signal intensities is then determined from the first and second ratios. Then, a ratio of the measured signal intensities is determined. The ratio of measured signal intensities provides a ratiometric calibration for the average ratio of signal intensities. If the average ratio of signal intensities does not correspond to the ratio of measured signal intensities, then the average ratio of signal intensities can be re-determined, using fresh measurements of the signal intensities recalibrated based on an adjusted scaling (or calibration) factor.

Thus, in a first aspect of the invention, there is provided a lateral flow test device comprising a solid support structure comprising a sample-receiving region, a conjugate pad, a first test region and a second test region, wherein the solid support structure is configured to permit liquid to flow sequentially from the sample-receiving region via the conjugate pad to the first test region and then the second test region, wherein

i) the conjugate pad comprises a mobilisable conjugated analyte comprising one or more analyte molecules conjugated to a detectable label;

ii)

a) the first test region comprises an immobilised analyte-binding molecule defining a first binding site,

b) the conjugate pad comprises a mobilisable analyte-binding molecule and the first test region comprises an immobilised capture molecule for immobilising the analyte-binding molecule, wherein the immobilised capture molecule defines a first binding site, or

c) the conjugate pad comprises a mobilisable analyte-binding molecule and the first test region comprises an immobilised analyte-binding molecule defining a first binding site, and

iii) the second test region comprises an immobilised conjugated-analyte-binding molecule which binds the conjugated analyte and does not bind unconjugated analyte molecules, and which defines a second binding site,

and wherein the number of molecules of the conjugated analyte is less than or equal to the number of second binding sites.

Conveniently, the number of molecules of the conjugated analyte is less than the number of second binding sites on the second test region.

Advantageously, the number of first binding sites is equal to the number of molecules of the conjugated analyte.

Preferably, the conjugated analyte of i), the immobilised analyte-binding molecule of ii)a) and the conjugated-analyte-binding molecule of iii) are present in a ratio of 1:1:1; or the conjugated analyte of i), the mobilisable analyte-binding molecule of ii)b) the capture molecule of ii)b) and the conjugated-analyte-binding molecule of iii) are present in a ratio of 1:1:1:1.

Conveniently, the detectable label is a nanoparticle, preferably a gold nanoparticle.

Preferably, each analyte molecule of the conjugated analyte is conjugated to the detectable label via a linking molecule, preferably wherein the linking molecule is biotin-BSA.

Advantageously, the conjugated-analyte-binding molecule is specific for, and binds, the linking molecule, preferably wherein the conjugated-analyte-binding molecule is avidin, streptavidin or polystreptavidin.

Conveniently, one of the analyte and the analyte-binding molecule is an antibody specific for the other of the analyte and the analyte-binding molecule.

Preferably, the analyte-binding molecule is an anti-analyte antibody.

In a second aspect of the invention, there is provided a method for detecting the presence of an analyte molecule in a test sample, the method comprising:

i) providing the lateral flow test device according to the first aspect,

ii) applying the test sample to the sample-receiving region of the lateral flow test device and allowing the test sample to migrate to the conjugate pad and to mix with the conjugated analyte, and optionally the mobilisable analyte-binding molecule,

iii) allowing the test sample and conjugated analyte, and optionally the mobilisable analyte-binding molecule, to migrate sequentially to the first test region and second test region and to contact the immobilised molecules in each of the first and second test regions,

iv) detecting a signal at each of the first and second test regions, wherein a change in signal intensity at both of the first and second test regions indicates the presence of the analyte molecule in the test sample.

Preferably, the signal is an optical signal.

Advantageously, the method further comprises v) quantifying a concentration of analyte molecule in the test sample based on a ratio of the signal intensity at the first test region to the signal intensity at the second test region.

Preferably, in step iv), a decrease of the signal intensity at the first test region and an increase of the signal intensity at the second test region indicates the presence of the analyte molecule in the test sample.

Conveniently, the signal intensities are measured as a percentage of a maximum signal intensity and wherein step iv) comprises measuring the signal intensity at one of the first and second test regions and predicting the signal intensity at the other of the first and second test regions based on the sum of the signal intensity of the first and second test regions being 100% of the maximum signal intensity.

Preferably, step iv) comprises:

a) measuring the intensity of the signal at the first test region and predicting the intensity of the signal at the second test region based on the sum of the signal intensity of the first and second test regions being 100% of the maximum signal intensity, thereby calculating a first measured:predicted signal intensity ratio,

b) measuring the intensity of the signal at the second test region and predicting the intensity of the signal at the first test

region based on the sum of the signal intensity of the first and second test regions being 100% of the maximum signal intensity, thereby calculating a second measured:predicted signal intensity ratio,
c) calculating an average signal intensity at each of the first and second test regions based on the first and second measured:predicted signal intensity ratios, to determine an average first:second test region signal intensity ratio,
d) calculating a ratio of the measured signal intensities of the first and second test regions,
e) comparing the average first:second test region signal intensity ratio to the ratio of the measured signal intensities, and
f) when the average first:second test region signal intensity ratio matches the ratio of the measured signal intensities, quantifying the concentration of analyte molecule in the test sample based on the average first:second test region signal intensity ratio,

wherein steps a) and b) can be carried out in either order.

Advantageously, the intensity of the signal at the first test region and the intensity of the signal at the second test region are scaled using a calibration factor when measured; and step iv) further comprises:
g) when the average first:second test region signal intensity ratio does not match the ratio of the measured signal intensities calculated in step d), adjusting the calibration factor and repeating steps a) to f) using the adjusted calibration factor.

When scaling using the calibration factor, a default or initial calibration factor may be utilised for one or more initial intensity measurements before the calibration factor is adjusted. This default or initial calibration factor may be equal to one (although, other values may also be used). When scaling using a calibration factor equal to one, the scaled intensities may be equal to the unscaled intensities.

Conveniently, adjusting the calibration factor comprises:

determining whether the measured signal intensities of the first and second test regions add up to greater than the maximum signal intensity;
in response to the measured signal intensities of the first and second test regions adding up to greater than the maximum signal intensity, reducing the calibration factor; and
in response to the measured signal intensities of the first and second test regions not adding up to greater than the maximum signal intensity, increasing the calibration factor.

Preferably, at least step a) of step iv) is carried out using an optical signal reader, preferably a smartphone.

The test sample may have been obtained from a human, animal or plant subject, or may be an environmental sample, or may be a food or drink sample.

In a third aspect of the invention, there is provided a method of diagnosing a disease or condition comprising carrying out the method according to the second aspect, wherein the test sample has been obtained from a human, animal or plant subject.

In a fourth aspect of the invention, there is provided a computer-implemented method of detecting the presence of an analyte molecule in a test sample, the method comprising:

obtaining a signal intensity measurement at each of the first and second test regions of a lateral flow test device according to the first aspect after the test sample has been applied to the lateral flow test device;
determining whether there has been a change in signal intensity at both of the first and second test regions;
in response to determining that there has been a change in signal intensity at both the first and second test regions, outputting an indication of the presence of the analyte molecule in the test sample.

The obtaining of the signal intensity measurement may comprise receiving a signal intensity measurement from an external system. For instance, the method may be implemented by a first computing system. The signal intensity measurement may be measured by a second computing system (e.g. a mobile device, light sensor, or camera). The signal intensity measurement may be obtained by the second computing system, and sent to the first computing system for further analysis to determine whether there has been a change in signal intensity at both of the first and second test regions. Alternatively, the signal intensity measurement may be determined by the first computing system from one or more light measurements of each of the first and second test regions, and/or one or more images showing the first and second test regions of the lateral flow test device. The one or more light measurements and/or one or more images may be obtained by the first computing system (e.g. through the use of a light sensor and/or a camera). Alternatively, the one or more light measurements and/or one or more images may be obtained by a second computing system (e.g. a mobile device, light sensor, or camera) and sent to the first computing system to determine whether there has been a change in signal intensity at both of the first and second test regions.

Preferably, determining whether there has been a change in signal intensity at both of the first and second test

regions comprises:

determining that there has been a change in one of the first and second test regions in response to the signal intensity for the one of the first and second test regions being less than a maximum signal intensity; and
determining that there has been a change in the other of the first and second test regions in response to the signal intensity for the other of the first and second test regions being greater than a minimum signal intensity.

Conveniently, the signal intensity measurements are obtained from one or more light measurements of each of the first and second test regions, and preferably the signal intensity measurements are obtained from one or more images showing the first and second test regions of the lateral flow test device.
In a fifth aspect of the invention, there is provided a kit comprising a lateral flow test device according to the first aspect.
In some embodiments, the conjugated analyte comprises two analyte molecules, or one analyte molecule, conjugated to a detectable label.
The term "immobilised", as used herein, means that the molecule is retained or fixed on the relevant part of the device using any suitable means known to the person skilled in the art. The immobilised molecule is not released or mobilised upon contact with the test sample or by the flow of the test sample. In particular, an "immobilised" analyte-binding molecule or capture molecule is one which will normally be retained in the test region in use, and will hence bind to relevant complementary molecules contained within the liquid contacting the test region.
The term "mobilisable", as used herein, means that the molecule in question is releasably retained or fixed on the relevant part of the device prior to use of the device, and is released from the part of the device upon contact with the test sample. The mobilisable molecule can be carried away from the original point of retention/fixation by the lateral flow of the test sample.
The term "binding site", as used herein, refers to an area or spot to which a molecule can be become fixed, in particular by complementary binding to a binding or capture molecule.
The term "unconjugated", as used herein, with reference to an analyte molecule, means that the analyte molecule is not linked to a conjugate molecule. For example, an unconjugated analyte molecule may be an analyte molecule from the test sample.
The term "polypeptide", as used herein, refers to a polymer of amino acid residues. The term also applies to amino acid polymers in which one or more amino acid residues is a modified residue, or a non-naturally occurring residue, such as an artificial chemical mimetic of a corresponding naturally-occurring amino acid, as well as to naturally occurring amino acid polymers.
The term "polynucleotide", as used herein, refers to a polymer of nucleic acid residues, and includes DNA and RNA.
The term "antigen", as used herein, refers to a molecule which is capable of eliciting an immune response in an individual.
The term "environmental sample" as used herein refers to a sample take from a natural or man-made environment. For example, an environmental sample may be a water sample taken from a natural river of man-made water source, a soil sample or a sample swabbed from natural or man-made surface.

## Brief Description of the Figures

Figure 1 is a schematic of a typical lateral flow assay test strip (a) and of the results obtained using a standard sandwich (b) test format.
Figure 2 is a schematic showing the format of the capture ELISA used to test the binding of the BSA-biotin-caffeine conjugate to both the anti-caffeine antibody and avidin.
Figure 3 is a schematic representation of the modified design of a LFT with two test lines: an anti-caffeine antibody test line and an avidin test line.
Figure 4 is a schematic representation of the reaction for the synthesis of BSA-biotin-caffeine conjugates used in Example 1.
Figure 5 is a graph showing the inhibition curve for the competitive ELISA with BSA-biotin-caffeine conjugate. The data points were obtained in triplicates and the error bars show the standard deviations about the mean values.
Figure 7 is a schematic representation of the initial design of the LFA with two test lines: an anti-mouse antibody test line and an avidin test line.
Figure 8 is a schematic representation of the working principle of the modified LFT design when samples (a) without caffeine and (b) with caffeine are run. Expected test results corresponding to the absence or presence of caffeine in the

samples are shown in Figure 8(c).

Figure 9 is a photograph of LFT strips according to the modified design run with different concentrations of glycerol in the buffer, in the presence (100μg/ml) or absence (0μg/ml) of caffeine.

Figure 10 is a photograph of LFT strips according to the modified design using different concentrations of caffeine. The running buffer was 5% (v/v) glycerol, 2% (w/v) BSa in PBS buffer containing 0.05% (w/v) Tween-20.

Figure 11 is a semi-log graph showing the signal intensities of Test Lines 1 and 2 of the LFT strips shown in Figure 10, analysed using ImageJ software. Results were measured in triplicates.

Figure 12 is a photograph of MFT strips run with different concentrations of caffeine after one week of storage. The running buffer was 5% glycerol, 2% BSA in PBS buffer containing 0.05% Tween-20.

Figure 13 is a schematic representation of the LFT design used to test the performance of lateral flow strips in buffered solution.

Figure 14 is a photograph of LFT strips run with synthetic urine containing different concentrations of NHPA. Solution volume was 200μl.

Figure 15 is a graph showing an ImageJ analysis of the grey level of the pixels in each test line of the strips of Figure 14 (a), and a graph showing the line intensity of each test line (upper line: streptavidin line; lower line: antibody line) at each concentration of NHPA, calculated by subtracting the peak minimum from the background (b).

Figure 16 is a photograph of LFT strips run with synthetic urine containing different concentrations of NHPA. For each concentration, three test strips prepared from the same batch of conjugates were assembled and tested.

Figure 17 is a graph showing the ImageJ analysis of signal intensity of the antibody lines for the different concentrations of NHPA used in Figure 16. Each data point is the average of five LFT strips.

Figure 18 is a photograph of LFT test strips run in the absence (a) and presence (b) of synthetic urine containing NHPA, and a schematic representation of the position of the analyte-binding line (A) and the streptavidin line (S).

Figure 19 is a photograph of LFT test strips run with different concentrations of creatinine.

Figure 20 is a semi-log graph showing the signal intensities of Test Lines 1 and 2 of the LFT strips shown in Figure 19, analysed using ImageJ software. Results were measured in triplicates.

Figure 21 shows a schematic representation of the mechanism of saturation of a sandwich LFT. There is twice as much analyte as each of conjugate and anti-analyte antibody on the test line, so some of the analyte cannot bind the test line, resulting in unquantified analyte.

Figure 22 shows a schematic representation of an example LFT of the present invention in which the amount of analyte molecules in the test sample is the same as the amount of each of the conjugated analyte and the binding sites on the first test line.

Figure 23 shows a schematic representation of an example LFT of the present invention in which the amount of analyte molecules in the test sample is greater than the amount conjugated analyte and binding sites on the first test line.

Figure 24 shows a schematic representation of a conventional sandwich LFT in which the number of analyte molecules in the test sample increases from 400 (A) to 415 (B), and the respective results obtained therefrom (C and D).

Figure 25 shows a schematic representation of an example LFT of the present invention in which the number of analyte molecules in the test sample increases from 400 (A) to 415 (B), and the respective results obtained therefrom (C and D).

Figure 26 shows a schematic representation of a computing system for quantifying a concentration of analyte molecules in a test sample based on signal intensities of first and second test regions of an LFT according to an embodiment.

Figure 27 is a graph of absorbance against concentration of BSA-biotin-creatinine. The data points were taken in triplicate, and the error bars represent standard deviation.

Figure 28 is a graph of absorbance against concentration of free creatinine. The data points were obtained in triplicate and the error bars represent standard deviation.

## Detailed Description

The invention relates, in general terms, to a lateral flow test device which contains a detectable conjugated analyte, comprising an analyte molecule conjugated to a detectable label, and two test regions. The first test region has one or more first binding sites which can bind both the conjugated analyte and the analyte molecules from a test sample, and the second test region has one or more second binding sites which can bind the conjugated analyte but not the target analyte. The number of molecules of the conjugated analyte in the device is less than or equal to the number of second binding sites of the second test region. In the absence of analyte in the test sample, the signal intensity of the second test region is never 100%, in order to ensure a change in the signal intensity of the second test region when analyte is present in the test sample.

*Solid support structure*

The lateral flow test device comprises a solid support structure, upon or within which the other components and reagents of the lateral flow test are placed. For example, the solid support structure may be a backing card upon which are arranged the other components and reagents for the lateral flow test. The backing card may then be comprised within a housing. In some instances, the solid support structure may itself comprise a housing, within which are arranged the other components and reagents for the lateral flow test. The solid support structure may be made of any suitable material known to the person skilled in the art, such as plastic.

The solid support structure is configured to allow liquid (e.g. a test sample, and any other liquid reagents or components added to the device) to flow sequentially from the sample-receiving region to the conjugate pad, then to the first test region and then the second test region. The device is configured to allow the liquid to contact and mix with the conjugated analyte, and, when present, the analyte-binding molecule, of the conjugate pad, and to contact the various molecules of the first and second test regions.

*Sample-receiving region*

The sample-receiving region is configured to receive a test sample, which is preferably in liquid form. The sample-receiving region is also configured to receive any other liquids, such as liquid reagents which may be added to the device of the invention.

The sample-receiving region may comprise any other components required or beneficial for LFTs, which may be mobilisable or immobilised thereon. In some embodiments, the sample-receiving region, before use of the device, comprises buffer components for the lateral flow test. These buffer components are mobilisable upon addition of the test sample, and may be mobilisably fixed to the sample-receiving region by any suitable means known to the person skilled in the art. For example, the buffer components may be dried onto the sample-receiving region, such that addition of a liquid, such as a liquid test sample, causes the buffer components to be released from the sample-receiving region and to mix with the test sample. Any suitable buffer components known to the person skilled in the art, such as sucrose and glycerol, may be used.

The sample-receiving region may be made of any suitable material known to the person skilled in the art. For example, the sample-receiving region may be paper-based, may be a woven mesh or made of cellulose, nitrocellulose or glass fibre. Preferably, the sample-receiving region is made of nitrocellulose or glass fibre. The sample-receiving region may be a part of a single structure comprising the conjugate pad, and optionally also the first and second test regions, or the sample-receiving region may be a separate structure from each of the conjugate pad and the first and second test regions. In some embodiments, the sample-receiving region may be a region of the conjugate pad, such that there is no distinction between the sample-receiving region and the conjugate pad (i.e. the test sample is added to the conjugate pad).

*Conjugate pad*

The conjugate pad comprises mobilisable conjugated analyte, which is disposed on or in the conjugate pad so that the conjugated analyte is released by, and mixes with, a test sample which contacts the conjugate pad. For example, the conjugated analyte may be dried onto the conjugate pad, so that it is released or mobilised by, and mixes with, the test sample upon contact therewith.

In some embodiments, the conjugate pad also comprises a mobilisable analyte-binding molecule and the first test region comprises an immobilised capture molecule for immobilising the analyte-binding molecule, wherein each molecule of immobilised capture molecule defines a first binding site. These embodiments provide the advantage that the effect of any difference in flow speed between the conjugated analyte and the analyte molecule in the test sample is reduced, thereby improving the accuracy of the LFT. In particular, the flow speed of molecules is determined, at least in part, by their size. When there is a large difference in size between the conjugated analyte and the unconjugated analyte (i.e. the analyte molecule in the test sample), the conjugated analyte and the unconjugated analyte flow at different speeds, resulting in one of the conjugated analyte and unconjugated analyte reaching the first test region before the other (usually the unconjugated analyte flows faster). This means that the conjugated analyte may not optimally compete with the analyte molecule in the test sample (i.e. unconjugated analyte) for binding to the analyte-binding molecule, thereby potentially resulting in an inaccurate quantitation of the analyte molecule in the test sample. By mixing the analyte-binding molecule with the test sample and conjugated analyte at the conjugate pad, the analyte-binding molecule is exposed to both the conjugated analyte and the analyte molecule in the test sample at the same time, thereby reducing the effect of the difference in flow speed of the conjugated analyte and unconjugated analyte (from the test sample).

In some embodiments, the conjugate pad comprises a mobilisable analyte-binding molecule as well as the conjugated analyte, and the first test region comprises an immobilised analyte-binding molecule. These embodiments, provide the advantage that the mobilisable analyte-binding molecule acts as a concentration buffer thereby introducing a

minimum threshold concentration before a positive result is shown on the LFT.

In some embodiments, the conjugate pad is a region of the same structure comprising the first and second test regions, or it may be a separate structure from the first and second test regions. When the conjugate pad is a separate structure from the test regions, it may be attached to the structure comprising the first and second test regions by any suitable means known to the person skilled in the art.

The conjugate pad may be made of any suitable material known to the person skilled in the art. For example, the conjugate pad may be paper-based, may be a woven mesh or made of cellulose, nitrocellulose or glass fibre. In some embodiments, the conjugate pad is made of nitrocellulose or glass fibre.

*First and second test regions*

The first and second test regions may be different areas of a single structure or may each be separate structures or areas of separate structures. When the first and second test regions are separate structures or areas of separate structures, the structures may be fixed to one another using any suitable means known to the person skilled in the art to allow liquid flow from the structure comprising the first test region to the structure comprising the second test region. In all embodiments, the first and second test regions are arranged such that, during use of the LFT, liquid flowing through/along the lateral flow test device reaches the first test region before the second test region.

Preferably, the first and second test regions are separate regions of a single structure, which may be a permeable or semi-permeable membrane which permits liquid flow on or through it. In some embodiments, the first and second test regions are separate areas of a single nitrocellulose membrane.

In some embodiments, the first test region comprises an immobilised analyte-binding molecule. Each immobilised analyte-binding molecule defines a first binding site on the first test region.

In some embodiments, the conjugate pad comprises a mobilisable analyte-binding molecule as well as a mobilisable conjugated analyte, and the first test region comprises an immobilised capture molecule for binding to and immobilising the analyte-binding molecule. Each immobilised capture molecule defines a first binding site on the first test region. These embodiments provide the advantage that the effect of any difference in flow speed between the conjugated analyte and the analyte molecule in the test sample is reduced, thereby improving the accuracy of the LFT, as discussed above.

In some embodiments, the conjugate pad comprises a mobilisable conjugated analyte and a mobilisable analyte-binding molecule, and the first test region comprises an immobilised analyte-binding molecule. Each immobilised analyte-binding molecule defines a first binding site on the first test region. These embodiments provide the advantage that the mobilisable conjugated analyte acts as a concentration buffer, thereby introducing a minimum threshold concentration before a positive result is shown on the LFT. In particular, the analyte molecules in the test sample compete with the conjugated analyte for the mobilisable analyte-binding molecule on the conjugate pad. Once an analyte molecule has bound to an analyte-binding molecule, the analyte molecule cannot then also bind to an immobilised analyte-binding molecule on the first test region. At the first test region, there is then competition between the conjugated analyte and any analyte molecules not bound to the mobilisable analyte-binding molecules.

In each of the configurations of the conjugate pad and first test region, the second test site comprises an immobilised conjugated-analyte-binding molecule, which binds to the conjugated analyte, but not to unconjugated analyte (i.e. analyte molecules from the test sample). Each immobilised conjugated-analyte-binding molecule defines a second binding site. Thus, any conjugated analyte which does not bind to the first test region binds to the second test region. As the concentration of analyte molecules in the test sample increases, fewer conjugated analyte molecules are able to bind to the first test region, thereby decreasing the signal intensity produced at the first test region, and more conjugated analyte molecules bind to the second test region, thereby increasing the signal intensity produced at the second test region. The signal intensity of the second test region is therefore dependent on, and inversely related to, the signal intensity of the first test region. In order for there to be an increase in signal intensity upon the presence of one or more analyte molecules in the test sample, the number of second binding sites at the second test region must be the same as or more than the number of conjugated analyte molecules in the lateral flow test device. In particular, as there will always be at least one first binding site at the first test region, there will always be at least one second binding site at the second test region which is not bound to a conjugated analyte molecule once the lateral flow test has been run in the absence of analyte molecules in the test sample - thus, the presence of an analyte molecule in a test sample will be able to increase the amount of conjugated analyte bound to the second test region by at least one molecule, thereby resulting in an increase in the signal intensity at the second test region.

Each of the first and second test regions may be any suitable shape, such as a square, rectangle, circle, triangle, oval or line. Preferably, each of the first and second test regions is a line, formed by immobilising the relevant analyte-binding molecule or capture molecule, and the conjugated-analyte-binding molecule, in a line. For example, when the first and second test regions are areas on a membrane, each of the first and second test regions may be a line on the membrane. Preferably, each of the first and second test regions stretches across the entire width of the flow path of the

liquid along/through the membrane (i.e. perpendicular to the direction of liquid flow). This provides the advantage that analyte molecules and conjugated analyte must contact the first test region before the second test region, thereby preventing a false positive response at the second test region, due to binding of the conjugated analyte. In addition, this configuration also provides that the test sample and the conjugated analyte molecules cannot circumvent the test regions, which would also falsify the results of the test.

*Conjugated analyte*

Each molecule of conjugated analyte used in the devices and methods of the present invention comprises an analyte molecule conjugated to a detectable label. One or more analyte molecules is conjugated to each detectable label. In some embodiments, only one analyte molecule is conjugated to each detectable label.

The analyte molecule conjugated to the detectable label must compete with the analyte molecule of the test sample for the analyte-binding molecule of the conjugate pad and/or the first test region. Preferably, the analyte molecule conjugated to the detectable label is the same type of molecule as the analyte molecule of the test sample. For example, if the analyte molecule to be detected in the test sample is caffeine, then the analyte molecule conjugated to the detectable label may also be caffeine.

The minimum concentration threshold of analyte molecules in the test sample required for a positive result can be altered by changing the number of analyte molecules conjugated to each detectable label. For example, an increase in the number of analyte molecules conjugated to each detectable label results in the conjugated analyte competing more strongly for the analyte-binding molecule, because the ratio of molecules to which the analyte-binding molecule can bind, i.e. the analyte molecules of the test sample and the conjugated analyte, is weighted more heavily in favour of the conjugated analyte. Thus, in some embodiments, each conjugated analyte comprises two or more analyte molecules conjugated to each detectable label, for example, 2, 3, 4, 5, 6, 7, 8, 9 or 10 analyte molecules conjugated to each detectable label.

In some embodiments, there are two analyte molecules conjugated to each detectable label, the conjugate pad comprises a mobilisable analyte-binding molecule and the first test region comprises an immobilised analyte-binding molecule. Preferably, the mobilisable analyte-binding molecule is the same as the immobilised analyte-binding molecule. Embodiments having a mobilisable analyte-binding molecule and an immobilised analyte-binding molecule provide the advantage of reducing the effect of different flow rates of the analyte molecules in the test sample and the conjugated analyte, for the same reasons as discussed above. In addition, the mobilisable analyte-binding molecule acts as a concentration buffer, for the same reason as discussed above. Moreover, the conjugated analyte is capable of binding to both a mobilised analyte-binding molecule and an immobilised analyte-binding molecule, because of the two analyte molecules conjugated to each detectable label, while the analyte molecule from the test sample can bind to only one of the mobilised and immobilised analyte-binding samples. This further contributes to the concentration buffer effect of these embodiments.

In some embodiments, each detectable label is saturated with analyte molecules (i.e. the number of analyte molecules conjugated to each detectable label is the maximum which can be conjugated to the detectable label). The specific number of analyte molecules conjugated to the detectable label will depend on the target analyte molecule in question, due to the size of the target analyte molecule.

The analyte molecule of the conjugated analyte is conjugated to the detectable label using any suitable means known to the person skilled in the art. In particular, the detectable label continues to be detectable while the analyte molecule is conjugated to the detectable label. In some embodiments, the analyte molecule is conjugated to the detectable label via a linker. The linker may be any suitable linker known to the person skilled in the art. For example, the linker may comprise biotin and/or BSA. In some embodiments, the linker is biotin-BSA.

The detectable label may be any suitable molecule known to the person skilled in the art which produces a detectable signal, the intensity of which can be detected or measured. For example, the detectable label may be a dye particle, a carbon particle, a fluorescent label, a latex particle, a gold particle, a magnetic particle, or the like. In some embodiments, the detectable label produces an optical signal, such as a colour change, or the appearance and/or disappearance of a visible mark. The signal intensity can therefore be a change (either positive or negative) in light intensity detected at a given test region. For instance, the detectable label may produce a red colour when bound to a test site. An increase in measured intensity may not in practice relate to an increase in intensity of a red frequency of light, but may instead be an increase in the intensity of a red frequency relative to other frequencies of light (e.g. an increase in the absorption of other frequencies of light). Alternatively, an increased signal intensity may relate to a decrease in intensity of reflected light. For instance, in the example of a red dye being bound to an otherwise white test region, an increased signal intensity may relate to a decrease in the overall intensity of light reflected (e.g. across all visible frequencies, as caused by the absorption of non-red light by the dye) or a decrease in the intensity of one or more non-red frequencies.

In preferred embodiments, the detectable label is a detectable nanoparticle, preferably a gold nanoparticle.

The minimum concentration of analyte molecule in the test sample required to produce a detectable signal at the

second test region can be pre-set by using a pre-determined amount of conjugated analyte and a pre-determined number of binding sites at the first test region. For example, an increase in the amount of conjugated analyte in the lateral flow test device will increase the minimum concentration of target analyte required to cause a change in the signal intensity of the first and second test regions, because the competition between the conjugated analyte and the analyte molecule of the test sample will be weighted in favour of the more numerous conjugated analyte.

*Analyte molecule*

The analyte molecule may be any analyte molecule of interest, particularly those which are associated with a disease or pathological condition. Other analyte molecules of interest include hormones, antibiotics, contaminants, for example food and drink contaminants, and pollutants. The analyte molecule may be a polypeptide, a polynucleotide, or an organic or inorganic compound.

In some embodiments, the analyte molecule is an antigen, such a protein, polypeptide or a fragment thereof. Example antigens include a bacterial or viral protein or a fragment thereof, such as a Coronavirus protein or fragment thereof. Proteins of interest may be, for example, enzymes or protein hormones.

The analyte molecule may be a biomarker for a disease or condition, or a biomarker of immunity to a disease, such that the detection and quantification of the biomarker is useful for the diagnosis and/or monitoring of the associated disease or condition, or immunity thereto. The analyte molecule may be a marker of, for example, pregnancy, kidney function, a bacterial infection or a viral infection. The biomarker may be a metabolite, lipid, steroid, protein, polypeptide, polynucleotide, or a fragment thereof. In some embodiments, the analyte molecule is 3-nitro-hydroxyphenylacetic acid (NHPA). In some embodiments, the analyte molecule is an antibody. In some embodiments, the analyte molecule is creatinine.

The analyte molecule may be a pharmaceutical drug, a nutraceutical drug, or other organic or inorganic compound, the detection and quantification of which is useful. In some embodiments, the analyte molecule is caffeine.

*Analyte-binding molecule*

Any molecule which binds to the analyte molecule of the test sample and of the conjugated analyte may be used as the analyte-binding molecule. Thus, the analyte-binding molecule is suitable for binding to the analyte molecule of the test sample and, separately, the analyte molecule of the conjugated analyte (i.e. the analyte-binding molecule is suitable for binding to the analyte molecule of the test sample and the conjugated analyte, but binds these one at a time). The analyte-binding molecule is preferably specific for the analyte molecule (of the test sample and of the conjugated analyte). In some embodiments, the analyte-binding molecule is an antibody specific for the analyte molecule (i.e. an anti-analyte antibody). For example, if the analyte molecule is NHPA, the analyte-binding molecule may be an antibody specific for 3-Nitrotyrosine (3-NTyr), which is known to cross-react with NHPA (Wisastra et al., "Antibody-free detection of protein tyrosine nitration in tissue sections", ChemBioChem, 2011, 12, 2016-2020). In another example, if the analyte molecule is caffeine, then the analyte-binding molecule may be an anti-caffeine antibody, which are known to the person skilled in the art. In another example, if the analyte molecule is creatinine, then the analyte-binding molecule may be an anti-creatinine antibody.

In some embodiments, the analyte molecule is an antibody, as described above, and the analyte-binding molecule is a molecule to which the antibody specifically binds. For example, the analyte-binding molecule may be an antigen and the analyte molecule may be an antibody specific for the antigen. In other examples, the analyte molecule is an antibody and the analyte-binding molecule is an antibody specific for the analyte molecule (i.e. an anti-antibody).

*Capture molecule*

In embodiments where the first test region comprises a capture molecule, the capture molecule may be any suitable molecule capable of binding to and immobilising the analyte-binding molecule, to retain the analyte-binding molecule at the first test region. In some embodiments, the capture molecule is capable of binding to a complex formed by the analyte-binding molecule and a conjugated analyte or an analyte molecule from the test sample (i.e. the capture molecule is specific for a complex formed by the analyte-binding molecule and the conjugated analyte or a complex formed by the analyte-binding molecule and the analyte molecule of the test sample). Preferably, in these embodiments, the capture molecule does not bind to analyte-binding molecules which have not bound to a conjugated analyte or analyte molecule from the test sample. In some embodiments, the immobilised capture molecule is an antibody specific for the analyte-binding molecule. In embodiments where the analyte-binding molecule is an antibody, the capture molecule may be an anti-antibody-complex antibody, specific for an analyte-binding molecule which has bound to a conjugated analyte or an analyte molecule from the test sample.

*Conjugated-analyte-binding molecule*

The conjugated-analyte-binding molecule of the second test region binds the conjugated analyte, and not unconjugated analyte molecules (i.e. analyte molecules from the test sample). Any suitable conjugated-analyte-binding molecule may be used, for example, a molecule which specifically binds the detectable label, or, when present, the linker of the conjugated analyte.

In some embodiments, the linking linker comprises biotin, preferably biotin-BSA, and the conjugated-analyte-binding molecule is avidin, streptavidin or polystreptavidin.

*Signal detection*

The signal produced by the detectable label of the conjugated analyte may be detected by any suitable means for the type of signal to be detected. In some embodiments, the detectable label produces an optical signal and the signal is detected, and the intensity thereof measured, using an optical signal sensor and/or reader. For example, an optical signal may be detected, and the intensity thereof measured, using a smartphone (e.g. using the camera of a smartphone). Alternatively, the optical signal may be detected, and the intensity thereof measured, using a reader including a lateral flow test holder configured to receive the lateral flow test for measurement. The holder may include one or more walls configured to enclose the lateral flow test (e.g. to block external light). The reader may include a light source to illuminate the lateral flow test during measurement. This may allow the amount of light incident on the lateral flow test to be controlled during measurement. Further discussion of the means of signal detection is provided below with reference to Figure 26.

*Detection of analyte molecules in the test sample*

In devices and methods of the invention, a change in signal intensity at both the first and second test regions is indicative of the presence of one or more analyte molecules in the test sample. In particular, the presence of an analyte molecule in the test sample means that there is competition for binding to the first test region between the analyte molecule and the conjugated analyte molecule. When competition is such that the first binding sites of the first test region are saturated with analyte molecules and/or conjugated analyte, any conjugated analyte which has not been able to bind the first test region will bind the second binding sites of the second test region. The more analyte molecules bind the first binding sites, the more conjugated analyte cannot bind the first binding sites, so the more the signal intensity at the first test region will decrease. The more conjugated analyte cannot bind the first binding sites, the more conjugated analyte will bind the second binding sites, so the more the signal intensity at the second test region will increase.

In some embodiments, the presence of one or more analyte molecules in the test sample is indicated by a decrease in the signal intensity at the first test region to less than 100%. Thus, in these embodiments, the device is configured such that, in the absence of analyte molecules in the test sample, either all of the first binding sites of the first test region are bound to conjugated analyte (either an excess of conjugated analyte, or the same number of first binding sites as there are molecules of conjugated analyte) or all of the conjugated analyte is bound to the first binding sites (either an excess of first binding sites, or the same number of molecules of conjugated analyte as there are first binding sites).

In some embodiments, the presence of one or more analyte molecules in the test sample is indicated by an increase in the signal intensity at the second test region from a signal intensity of less than 100%. In these embodiments, some or none, but not all, of the second binding sites are bound to conjugated analyte in the absence of analyte molecules in the test sample. Thus, when one or more analyte molecules is present in the test sample, the conjugated analyte which does not bind to the first test region due to competition with the analyte molecule(s) in the test sample instead binds the second test region, thereby increasing the signal intensity at the second test region. Thus, the signal intensity at the second test region is inversely related to the signal intensity at the first test region.

In some embodiments, the presence of one or more analyte molecules in the test sample is indicated by an increase in the signal intensity at the second test region from 0%. Thus, in these embodiments, in the absence of analyte molecules in the test sample, all of the molecules of conjugated analyte bind to the first test region, and none (or substantially none, such that no detectable signal is produced) bind to the second test region.

The detection of the signal intensities of the first and second test regions is performed based the signals present at the first and second test regions at a minimum time after the test sample has been applied to the sample-receiving region. It will be understood that the detection of the signal intensities can be performed using the first and second test regions themselves (i.e. detection directly from the physical first and second test regions) or using a reproduction, such as a photograph, of the first and second test regions. In particular, sufficient time must elapse before the detection of the signal intensities in order to permit the test sample to migrate to the conjugate pad, the first test region and then the second test region. The detection of the signal intensities of the first and second test regions may be performed on the signal present at the first and second test regions after any suitable minimum time, for example, after at least 1 minute, at least 2 minutes, at least 3 minutes, at least 4 minutes, at least 5 minutes, at least 10 minutes, at least 15 minutes, at least 20 minutes, at least

25 minutes or at least 30 minutes. Preferably, the detection of the signal intensities of the first and second test regions is based on the signals present at the first and second test regions at least 15 minutes after the test sample has been applied to the sample-receiving region.

The detection of the signal intensities of first and second test regions may be based on the signals present at the first and second test regions before a maximum time has elapsed from the application of the test sample to the sample-receiving region. For example, the detection of the signal intensities may be based on the signals present at the first and second test regions no more than 1 hour, no more than 50 minutes, no more than 45 minutes, no more than 40 minutes, no more than 35 minutes or no more than 30 minutes after the test sample has been applied to the sample-receiving region. In some embodiments, the detection of the signal intensities of the first and second test regions is based on the signals present at the first and second test regions no more than one hour after the test sample has been applied to the sample-receiving region.

*Quantification*

The devices and methods of the present invention provide for a semi-quantitative or a quantitative result. In particular, lateral flow test devices of the invention are manufactured to have a known amount of each component, namely, the conjugated analyte and first and second binding sites, and optionally, mobilisable analyte-binding molecule, in order to manufacture a device with an appropriate detection threshold for the target analyte in question. In particular, each target analyte has a different threshold concentration which indicates an associated condition (e.g. presence of a disease or pathological condition, threshold acceptable levels of a contaminant, etc). The person skilled in the art will know how to adjust the amounts of the conjugated analyte and first and second binding sites, and optionally the mobilisable analyte-binding molecule, to provide a certain threshold for a positive response (i.e. detection of target analyte in the test sample).

Thus, a change in the signal intensity at both the first and second test regions indicates that the analyte molecule is present in the test sample above a threshold concentration. In particular, the change in signal intensity is a decrease in signal intensity at the first test region, due to increased competition between the conjugated analyte and the analyte molecule of the test sample resulting in fewer conjugated analyte molecules binding to the first test region, and an inversely related increase in signal intensity at the second test region, due to an increase in conjugated analytes molecules not binding the first test region and so binding the second test region.

Accordingly, the methods and devices of the present invention provide for quantitation of the concentration of analyte molecule in the test sample by measuring the amount of change of the signal intensities of the first and second test regions. As the threshold concentration of the analyte molecule in the test sample required for a change in the signal intensities is known, as discussed above, further decreases of the signal intensity at the first and further increases of the signal intensity at the second test region correlate with increasing concentration of the analyte molecule in the test sample.

In addition, the devices and methods of the invention provide for more accurate quantitation of the analyte molecule in the test sample because there is no loss of information about the amount of conjugate which does not bind to the first test region and/or which binds the second test region. As mentioned above, conventional LFTs require an excess of labelled conjugate in order to ensure that the control line is an effective control, but this means that information about the amount of labelled conjugate binding to the control line is lost. In contrast, in the devices and method of the present invention, there is the same amount of detectable conjugated analyte as, or less than, the number of binding sites at the second test region, so there is no corresponding loss of information.

It should be noted that the devices and methods of the invention do not require a separate control line because the appearance of a detectable signal at either the first or the second test region indicates a valid test. The absence of both lines indicates an invalid test.

In some embodiments, the quantitation of analyte molecules in the test sample is carried out by measuring the signal intensities at each of the first and second test regions and calculating a ratio of the two signal intensities. The ratio of the two signal intensities is indicative of the number of analyte molecules in the test sample. In particular, and as mentioned above, the devices of the invention are manufactured with a known, pre-determined amount of each component thereof, such that the amount of conjugated analyte binding to each of the first and second test regions in the absence of analyte molecule is known.

The signal intensities at each of the first and second test regions may be measured. This may be achieved by obtaining one or more photographs of the first and second test regions. The location of the first and second test regions within each of the one or more photographs may be obtained through user input or using an image recognition method, such as edge detection and/or machine learning. The signal intensity at each test region may be determined based on the intensity of one or more pixels representing the test region. This may be based on one pixel, or may be an accumulation of intensity values of multiple pixels (e.g. an average intensity).

The pixel intensity values may then be converted into a signal intensity representing an amount of change in intensity (e.g. relative to a calibration section). The signal intensity may be in the form of a percentage relative to one or both of a maximum and minimum signal intensity (e.g. may be normalized). The maximum signal intensity may be a predefined

value (e.g. based on an earlier calibration measurement) or may be determined from a measurement of the signal intensity at a calibration region of the lateral flow test (e.g. a calibration line that has a predefined colour (or discolouration) that corresponds to a test region that is fully saturated (e.g. a signal intensity of 100%)). The signal intensity may be determined as a percentage of the maximum signal intensity. The percentage may also be based on a minimum signal intensity. This minimum signal intensity may be predefined or measured (e.g. a calibration region that has a predefined colour that corresponds to a test region that is fully unsaturated (e.g. a signal intensity of 0%)). Whilst in this embodiment, 100% represents a fully saturated line and 0% represents a fully unsaturated line, it will be appreciated that the opposite may apply, depending on the response of the line to analyte.

In some embodiments, the quantitation of analyte molecules in the test sample is carried out by measuring the signal intensity at one of the first and second test regions and then predicting the signal intensity at the other of the first and second test region based on the sum of the signal intensities of the first and second test regions being 100%. This measurement and subsequent prediction results in a measured:predicted ratio of signal intensities of the first and second test regions.

In some embodiments, the signal intensity of the first test region is measured and, subsequently, the signal intensity of the second test region is predicted based on the sum of the signal intensities of the first and second test regions being 100%. This results in a first measured:predicted signal intensity ratio. The signal intensity of the second test region is measured and, subsequently, the signal intensity of the first test region is predicted, based on the sum of the signal intensities of the first and second test regions being 100%. This results in a second measured:predicted signal intensity ratio. The first and second measured:predicted signal intensity ratios can be calculated in either order. A calibration factor (otherwise known as a scaling factor) may be applied to each of the first measured signal intensity and the second measured signal intensity. This calibration factor may be used to re-calibrate the measurements (discussed later below). The calibration factor may initialise at an initial value (e.g. 1) and may be adjusted based on calibration checks. When scaling using a calibration factor equal to one, the scaled measurements may be equal to (e.g. unchanged from) the unscaled measurements.

Next, the first and second measured:predicted signal intensity ratios are averaged (e.g. the mean of the first and second measured:predicted signal intensity ratios is calculated), to produce an average first:second test region signal intensity ratio. Then, measured signal intensities of both the first and second test regions are used to determine a ratio of the measured signal intensities. The measurements used to determine the ratio of measured signal intensities may be the same as the measurements used in the earlier predictions. Alternatively, different measurements may be taken (e.g. from different samples of the same image, or from different images).

The average first:second test region signal intensity ratio is compared to the ratio of measured signal intensities, which provides a ratiometric calibration to check that the signal intensities used to calculate the average first:second test region signal intensity ratio were accurate.

If the average first:second test region signal intensity ratio does not match the ratio of measured signal intensities (e.g. within a predefined range), then the calibration factor for the measured signal intensities may be adjusted and the quantitation method repeated. That is, the first measured signal intensity and the second measured signal intensity may be recalculated based on the adjusted calibration factor, new measured:predicted signal intensity ratios may be obtained, a new average first:second test region signal may be obtained and the new average first:second test region signal may be compared with a new ratio of the measured signal intensities (based on the adjusted calibration factor). The recalculated first measured signal intensity and second measured signal intensity may be based on the same intensity measurements as previous, or new measurements may be obtained.

If the average first:second test region signal intensity ratio matches the ratio of measured signal intensities, then the average first:second test region signal intensity ratio may be utilised without recalibration as the signal intensity ratio for the test sample.

The method may continue to repeat over multiple iterations until the average first:second test region signal matches the ratio of the measured signal intensities (or until a maximum number of iterations has been reached).

The direction of scaling (the direction of adjustment of the calibration factor) may vary depending on whether the measured signal intensities add up to greater than or less than a maximum signal intensity (e.g. more than 100% of the maximum signal intensity). For instance, where the first and second measured signal intensities add up to more than the maximum signal intensity, then the calibration factor may be reduced (e.g. reduced by a predefined step size, or predefined factor). Similarly, where the first and second measured signal intensities do not add up to more than the maximum signal intensity, or add up to less than the maximum signal intensity, then the calibration factor may be increased (e.g. increased by a predefined step size, or predefined factor). This helps to recalibration the signal intensity measurements to account for varying environmental lighting conditions.

In some embodiments, the average first:second signal intensity ratio matches the measured signal intensities when the average first:second signal intensity ratio is within a predefined range of the ratio of measured signal intensities. The predefined range may be, for example, within 10%, within 9%, within 8%, within 7%, within 6%, within 5%, within 4%, within 3%, within 2%, within 1% or within 0.5% of the ratio of measured signal intensities, i.e. an error margin of no more

than 10% may be permitted. Preferably, the average first:second signal intensity ratio matches the ratio of measured signal intensities when the average first:second signal intensity ratio is within 5% of the ratio of measured signal intensities.

The determined signal intensity ratio for the sample (e.g. either scaled or unscaled) is indicative of the concentration of analyte molecules in the test sample. The signal intensity ratio can therefore be used to determine the concentration of analyte molecules in the test sample (e.g. based on a calibration graph of signal intensity ratio to concentration of analyte). This method of quantifying the analyte molecule in the test sample permits the reduction of systemic errors due to the reader of the LFT device as well as any errors due to ambient lighting. In particular, the first and second measured:predicted signal intensities determine the precision of quantification (i.e. the repeatability of the results), while the ratio of measured signal intensities, and the comparison to the average first:second test region signal intensity ratio, ensures the accuracy of the quantification (i.e. a true quantification of the target analyte in the test sample).

As with existing quantitative LFTs, a calibration curve is used to calculate the concentration of analyte molecules on the basis of the determined ratio of the signal intensities.

The devices and methods of the present invention also address the problem of saturation of the test line of conventional LFTs. In particular, conventional quantitative LFTs use only the test line, and not the control line, to quantify the concentration of analyte molecules in the test sample. Thus, when the test line becomes saturated with analyte molecules from the test sample, any excess analyte molecules from the test sample cannot be quantified. However, in the devices and methods of the present invention, the use of two test regions (e.g. lines), and a ratio of the signal intensities of the two test regions, means that it is possible to quantify the analyte molecules in the test sample beyond the normal saturation point of a corresponding conventional LFT. Figure 21 shows the mechanism of saturation of a conventional sandwich LFT. In particular, as there is more of the analyte molecule in the test sample than available conjugate or anti-analyte antibodies on the test line, there are unbound, and therefore, unquantified analyte molecules. In contrast, the devices and methods of the present invention rely on competition between the analyte molecules in the test sample and the conjugated analyte to produce a ratio of signal intensities of two test regions, which is then used to quantify the analyte in the test sample. Figure 22 shows that, when the amount of analyte molecules in the test sample is the same as the amount of conjugated analyte and the number of binding sites on the first test line (i.e. analogous to the saturation point of a conventional sandwich LFT; in this example, 200 units of each of analyte, conjugated analyte and first binding sites), competition between the analyte molecules from the test sample and the conjugated analyte is equal, resulting in equal binding to the first test line. The remaining conjugated analyte (i.e. 50% of the conjugated analyte originally on the conjugate pad) binds the second test line, resulting in equal signal intensities at each of the first and second test lines (i.e. a signal intensity ratio of 50%:50% (1:1)). Figure 23 shows that, when the amount of analyte molecules in a test sample increases above the amount of conjugated analyte and binding sites on the first test line (i.e. above the saturation point of a conventional sandwich LFT. In this example, twice as many analyte molecules (i.e. 400 units) as each of conjugated analyte and first binding sites (i.e. 200 units of each)), quantification of the analyte molecules in the test sample is still possible. This is because this situation results in a change in signal ratios between the first and second test lines, due to the increased competition for the binding sites on the first test line by the analyte molecules from the test sample. In particular, the ratio of signal intensities of the first test line to second test line in this example LFT is 33%:67% (1:2). Thus, using devices and methods of the present invention, it is still possible to quantify the concentration of analyte molecules in a test sample which are above the normal saturation level of a corresponding conventional LFT.

In conventional LFTs, one solution to overcoming the problem of saturation is to increase the number of binding sites on the test line. However, this results in a decrease in the resolution when quantifying the analyte in the test sample. In particular, when there are more binding sites on the test line, each binding site contributes a smaller percentage of the maximum signal intensity of the test line. For example, if a hypothetical LFT contains 200 binding sites on the test line, each binding site accounts for 0.5% of the maximum signal intensity. When the number of binding sites is increased to 400, each binding site accounts for only 0.25% of the maximum signal intensity. As an example, Figure 24 shows a hypothetical conventional sandwich LFT, in which there are 1000 binding sites on the test line. An increase in the number of analyte molecules in the test sample from 400 (Figure 24a) to 415 (Figure 24b) results in a change of signal intensity at the test line from 40% (Figure 24c) to 41.5% (Figure 24d). Figure 25 shows an example LFT according to the present invention and shows that the same increase from 400 (Figure 25a) analyte molecules in the test sample to 415 (Figure 25b) results in an overall change in the signal intensity ratio of 2% (change from 50% to 49% at the first test line, and from 50% to 51% at the second test line; Figure 25c & d). In particular, the change in signal intensity at the first test line is mirrored by the inverse relationship with the signal intensity at the second test line. This effectively doubles the change in signal intensity compared to a conventional LFT. Larger changes in signal intensity are easier to detect and measure, so the LFT devices of the present invention increase the precision with which analyte molecules can be quantified compared to conventional LFTs.

For the same reasons as discussed in relation to the detection of the signal intensities, the quantitation of the concentration of analyte molecule in the test sample is performed based on the signals present at the first and second test regions after a minimum time has elapsed from the application of the test sample to the sample-receiving region. As with detection of the signal intensities, quantitation can be performed on the first and second test regions themselves or on a

reproduction, such as one or more photographs, of the first and second test regions. Thus, the above-discussed possible minimum times for the detection of the signal intensities apply equally to the quantitation of the concentration of analyte molecule in the test sample.

As with detection of the signal intensities (discussed above), quantitation of the concentration of analyte molecule in the test sample may be performed based on signals present at the first and second test regions before a maximum amount of time has elapsed from the application of the test sample to the sample-receiving region. The maximum amount of time may be the same as discussed in relation to detection of signal intensities, above.

*Ratios of components*

The number of molecules of conjugated analyte in the LFT device of the present invention is the same as or less than the number of second binding sites of the second test region. This ensures that there will always be a change in signal intensity at the test regions when the test sample contains analyte molecule(s).

The specific amount of each of the conjugated analyte, first binding sites and second binding sites is not essential for the functioning of the devices and methods of the present invention, so long as the amount added to the devices during manufacture is known. These amounts can then be used to calibrate the reading devices used to detect, and measure the intensity of, the signals at the first and second test regions. In particular, different analyte molecules (i.e. different target analytes) will require different amounts of each component, as different types of target analyte have different threshold levels and concentrations of interest.

In some embodiments, the ratio of conjugated analyte to second binding sites is from 1:1 to 1:100, 1:1 to 1:50, 1:1 to 1:20, 1:1 to 1:10, 1:1 to 1:5, 1:1 to 1:4, 1:1 to 1:3 or 1:1 to 1:2, or is 1:1. In some embodiments, the number of molecules of conjugated analyte is the same as the number of second binding sites of the second test region (i.e. a ratio of 1:1).

The number of first binding sites may be greater than, the same as or less than the number of second binding sites. In particular, the number of first binding sites can be chosen so as to set a minimum threshold concentration of analyte in the test smaple required for a change in signal intensity at the first test region. In some embodiments, the number of first binding site is the same as or less than the number of second binding sites. In these embodiments, the ratio of first binding sites to second binding sites is from 1:100 to 1:1, 1:1 to 1:50, 1:1 to 1:20, 1:1 to 1:10, 1:1 to 1:5, 1:1 to 1:4, 1:1 to 1:3 or 1:1 to 1:2, or is 1:1.

Any of the above ratios of conjugated analyte to second binding sites can be combined with any of the above ratios of first binding sites to second binding sites. In some embodiments, the ratio of conjugated analyte to second binding sites is 1:1 and the ratio of first binding sites to second binding sites is 1:1 (i.e. a ratio of conjugated analyte to first binding sites to second binding sites of 1:1:1). In particular, a ratio of conjugated analyte:first binding sites:second binding sites of 1:1:1 provides the advantage that there is a more obvious change in the signal intensity of the second test region in the presence of analyte molecules in the test sample (i.e. a clean transition in the signal intensities of the first and second test regions). In particular, in the absence of analyte molecules in the test sample, the signal intensity of the first test region will be at its maximum (i.e. 100% of possible signal intensity) and the signal intensity of the second test region will be at its minimum (i.e. 0% of possible signal intensity). Thus, the presence of analyte molecules in the test sample will be indicated by a decrease of signal intensity of the first test region from 100% of its maximum and a corresponding increase in signal intensity of the second test region from 0% of its maximum.

In some embodiments, the conjugate pad comprises a mobilisable conjugated analyte, the first test region comprises an immobilised analyte-binding molecule and the second test region comprises an immobilised conjugated-analyte-binding molecule, and the ratio of conjugated analyte to analyte-binding molecule to conjugated analyte-binding molecule is from 1:1:1 to 1:100:100. Preferably, the ratio of conjugated analyte to analyte-binding molecule to conjugated analyte-binding molecule is 1:1:1.

In embodiments where the conjugate pad comprises an mobilisable analyte-binding molecule, as well as a mobilisable conjugated analyte, and the first test region comprises a capture molecule, the ratio of conjugated analyte to second binding sites, the ratio of first binding sites to second binding sites, and the ratio of conjugated analyte to first binding sites to second binding sites, may independently be any of the relevant ratios disclosed above. The ratio of the conjugated analyte to the mobilisable analyte-binding molecule to the first binding sites may be any suitable ratio for modulating the effect of different flow speeds of the target analyte molecule and the conjugated analyte from the conjugate pad to the first and second test regions. In some embodiments, the ratio of conjugated analyte to mobilisable analyte-binding molecules may be from 1:1 to 1:100, 1:1 to 1:50, 1:1 to 1:20, 1:1 to 1:10, 1:1 to 1:5, 1:1 to 1:4, 1:1 to 1:3 or 1:1 to 1:2, or is 1:1. In some embodiments, the ratio of conjugated analyte to mobilisable analyte-binding molecules to first binding sites may be from 1:1:1 to 1:100:100.

In embodiments where the conjugate pad comprises a conjugated analyte and a mobilisable analyte-binding molecule, and the first test region comprises an immobilised analyte-binding molecule, the ratio of conjugated analyte to second binding sites, the ratio of first binding sites to second binding sites, and the ratio of conjugated analyte to first binding sites to second binding sites, may independently be any of the relevant ratios disclosed above. The ratio of

conjugated analyte to mobilisable analyte-binding molecule may be any ratio suitable for acting as a concentration buffer, to set a minimum concentration threshold before a positive result is shown on the LFT.

*Test sample*

The test sample added to the device during methods of the invention may be any sample of interest. The test sample may be a liquid comprising or consisting of a body fluid, a liquid sample derived from a swab or other test (for example, cellular and non-cellular material suspended in a liquid), or a liquid sample derived from a solid sample (for example, a solution of the solid sample or some component thereof). The test sample may have been obtained from a biological organism, such as a human, animal (i.e. non-human animal) or plant, or the test sample may be an environmental sample obtained from a natural or man-made environment, or the test sample may be a food or drink sample (i.e. obtained from a food or a drink). In some embodiments, the test sample comprises or consists of a body fluid, for example, urine, blood, plasma, serum, saliva or mucus.

*Specific LFTs - NHPA, caffeine, creatinine*

In some embodiments, the devices and methods of the present invention are for the detection and/or quantification of caffeine (i.e. the analyte molecule is caffeine), for example, in blood. In particular, it is useful to test a patient's blood for caffeine before a cardiac MRI scan, as caffeine inhibits the vasodilatory action of adenosine administered during cardiac MRI scans (J. Majd-Ardekani, P. Clowes, V. Menash-Bonsu and T. O. Nunan, Nucl. Med. Commun., 2000, 21, 361-364), thereby leading to abnormal and/or false negative results. This can delay the diagnosis of heart problems in patients (E. Reyes, C. Y. Loong, M. Harbinson, J. Donovan, C. Anagnostopoulos and S. R. Underwood, J. Am. Coll. Cardiol., 2008, 52, 2008-2016). In these embodiments, the conjugated analyte comprises one of more caffeine molecules conjugated to a detectable label. The detectable label may be a gold nanoparticle. The analyte-binding molecule is capable of binding caffeine and, preferably, is an anti-caffeine antibody. The conjugated-analyte-binding molecule may be avidin, streptavidin or polystreptavidin. The conjugated analyte preferably comprises a linker, which may be BSA-biotin. In some embodiments, the conjugated analyte comprises one or more caffeine molecules conjugated to a gold nanoparticle by a BSA-biotin linker, the analyte-binding molecule is an anti-caffeine antibody and the conjugated-analyte-binding molecule is avidin, streptavidin or polystreptavidin. In methods of detecting and/or quantifying caffeine, the sample may be a blood, plasma or serum sample.

In some embodiments, the devices and methods of the present invention are for the detection and/or quantification of NHPA (i.e. the analyte molecule is NHPA). In these embodiments, the conjugated analyte comprises one or more NHPA molecules conjugated to a detectable label. The detectable label may be a gold nanoparticle. Preferably, each NHPA molecule is conjugated to the detectable label via a linker, which is preferably BSA-biotin. The analyte-binding molecule is capable of binding NHPA and, preferably, is an anti-NHPA antibody. In some embodiments, the analyte-binding molecule is an anti-3-Nitrotyrosine (3-NTyr) antibody, which has been shown to be cross-reactive with a BSA conjugate of NHPA (Wisastra et al., "Antibody-free detection of protein tyrosine nitration in tissue sections", ChemBioChem, 2011, 12, 2016-2020). The conjugated-analyte-binding molecule may be avidin, streptavidin or polystreptavidin. In some embodiments, the conjugated analyte comprises one or more NHPA molecules conjugated to a gold nanoparticle via a BSA-biotin linker, the analyte-binding molecule is an anti-3NTyr antibody, and the conjugated analyte-binding molecule is avidin, streptavidin or polystreptavidin. In methods of detecting and/or quantifying NHPA, the sample may be a urine sample.

In some embodiments, the devices and methods of the present invention are for the detection and/or quantification of creatinine (i.e. the analyte molecule is creatinine). In these embodiments, the conjugated analyte comprises one or more creatinine molecules conjugated to a detectable label. The detectable label may be a gold nanoparticle. Preferably, each creatinine molecule is conjugated to the detectable label via a linker, which is preferably BSA-biotin. The analyte-binding molecule is capable of binding creatinine and, preferably, is an anti-creatinine antibody. The conjugated-analyte-binding molecule may be avidin, streptavidin or polystreptavidin. In some embodiments, the conjugated analyte comprises one or more creatinine molecules conjugated to a gold nanoparticle via a BSA-biotin linker, the analyte-binding molecule is an anti-creatinine antibody, and the conjugated analyte-binding molecule is avidin, streptavidin or polystreptavidin. In methods of detecting and/or quantifying creatinine, the sample may be a blood, plasma or serum sample.

*Uses of the Device and Method*

The device of the invention is for detecting the presence, and optionally the quantity, of an analyte molecule in a test sample, and the present invention includes methods of detecting the presence, and optionally the quantity, an analyte molecule in a test sample. The methods and devices of the invention are useful for detecting the presence, and optionally the quantity, of an analyte molecule in test samples obtained from a wide variety of sources, and so are useful for a wide

range of purposes.

For example, the devices and methods of the invention are useful for diagnosing a disease or condition using a test sample obtained from a human, animal or plant. In particular, methods of detecting the presence, and optionally the quantity, of an analyte molecule in a test sample according to the invention can be used to inform (i.e. can contribute to) a diagnosis. The diagnosis may rely upon the result of the method of the invention alone, or the result of the method of the invention may be one of several factors leading to a diagnosis. Thus, the present invention includes a method of diagnosing a disease or condition comprising carrying out a method of detecting the presence, and optionally the quantity, of an analyte molecule in a test sample obtained from a human, animal or plant.

In another example, the devices and methods of the invention are useful for detecting the presence, and optionally the quantity, of an analyte molecule in an environmental sample, or in a food or drink sample.

The devices and methods of the invention are also useful for detecting the presence, and optionally the quantity, of markers of good health, as well as markers of bad health, (i.e. the analyte molecule is a marker indicative of good health or bad health) without necessarily leading to a specific diagnosis of a disease or condition.

In addition, methods of detecting the presence, and optionally the quantity, of an analyte molecule are also useful for monitoring the presence or amount of an analyte molecule in a subject (e.g. a human, animal or plant) or in an environment. This can be for the purpose of, for example, monitoring the health or condition, or the propensity for a disease or condition, of the subject, monitoring disease progression in the subject, monitoring the effect of a treatment on the subject, or monitoring the rate of change of the quantity of the analyte molecule in the subject or environment. When the purpose is for monitoring the rate of change of the quantity of the analyte molecule, this can be used, for example, to monitor and/or calculate the rate of clearance of a drug. Thus, the method of detecting the presence, and optionally the quantity, of an analyte molecule of the invention may be a method of monitoring the presence and/or quantity of the analyte molecule in a subject or in an environment. In some embodiments, the presence of the analyte molecule is monitored by obtaining test samples at two or more different time points and detecting the presence of the analyte molecule in each test sample. In some embodiments, the step of detecting the presence of the analyte molecule in each test sample includes quantifying the concentration of the analyte molecule in each test sample. The quantity of the analyte molecule in each test sample may be compared to a baseline quantity and/or to the quantity in a preceding test sample. The baseline quantity may be a quantity of the analyte molecule measured in a baseline test sample obtained at the start of, or before, the monitoring method, or the baseline quantity may be a reference quantity of the analyte molecule calculated as an average quantity measured in one or more control samples. Alternatively, the baseline amount may be a threshold amount or range of amounts of the analyte molecule known in the art. In some embodiments, the presence or absence, and/or the quantity, of the analyte molecule in each test sample is useful for monitoring the health or condition of the subject. For example, the presence or absence of the analyte molecule may be indicative of the quality of health (e.g. good or bad health, or propensity for a disease/condition) of the subject, or may be indicative of disease progression or the effect of a treatment on the subject. A change in the presence and/or quantity of the analyte molecule between the two or more test samples may be indicative of a change of the quality of health of the subject, or may be indicative of disease progression, the effect of a treatment on, or clearance of a substance (e.g. the analyte molecule) by, the subject.

*Measurement System*

Figure 26 shows a schematic representation of a computing system 100 for quantifying the concentration of analyte molecule in a test sample based on signal intensities of first and second test regions of an LFT according to an embodiment.

The computing system 100 comprises a processor 110, memory 120, non-volatile memory 130 and an input/output (I/O) interface 140. Optionally, the computing system 100 may comprise an optical sensor 150 (e.g. a digital camera). The computing system 100 may be any form of computing system, such as a personal computer, a server, a smartphone, a tablet, etc.

The computing system 100 is controlled by the processor 110. The processor 110 is configured to quantify the concentration of analyte molecule in a test sample based on executable code stored in the non-volatile memory 130 and loaded for execution into memory 120 (e.g. Random Access Memory, RAM).

The quantified concentration is determined based on one or more measurements of signal intensity at the first test region and one or more measurements of signal intensity at the second test region. These measurements may be obtained from one or more images (e.g. one or more photographs) of the LFT showing the first and second test regions. This image may be obtained by an optical sensor, such as a digital camera. The optical sensor may be integrated within the computing system (e.g. optical sensor 150) or may be within an external system. That is, the computing system 100 may be configured to obtain one or images of the LFT through an integrated optical sensor, or may be configured to obtain one or more images of the LFT from an external system (e.g. over a network, such as the internet).

For instance, a second system (e.g. a second computing system) may be configured to obtain one or more images showing the first and second test regions, or one or more light intensity measurements of the first and second test

regions. The computing system 100 may be configured analyse signal intensity measurements obtained from the one or more images or the one or more light measurements to quantify the concentration of the analyte molecule.

The second system may determine the one or more signal intensity measurements from the one or more images or the one or more light intensity measurements and send the one or more signal intensity measurements to the computing system 100. Alternatively, the second system may send the one or more images or the one or more light intensity measurements to the computing system 100, which may then determine the one or more signal intensity measurements.

Alternatively, the computing system 100 may obtain the one or more images or one or more light intensity measurements and may also perform the subsequent analysis to quantify the concentration of the analyte molecule.

Accordingly, where the user makes use of a mobile device (e.g. smartphone or tablet) to obtain an image of the LFT, the mobile device may be configured, through software, to perform the steps described herein to quantify the concentration of analyte molecule in the test sample. Alternatively, the image may be transferred to another computing system (the computing system 100), e.g. in the cloud, for quantitation.

The quantified concentration determined by the processor 110 may be output via the I/O interface 140 (e.g. a screen or to an external system via a network, such as the internet), may be utilised by the processor 110 for further calculations, or may be stored into non-volatile memory 130 for later use.

*Further Components*

The LFT devices of the present invention may include further components for the optimisation of the LFT, in line with conventional practice in the art. These additional components may be any suitable components known in the art for use with LFTs or ELISA assays. For example, the LFT device may comprise a buffer for modifying the flow rate of the sample along the solid support structure, or a blocking agent, for reducing non-specific binding of the conjugated analyte to the solid support structure and reducing background signals. The buffer for modifying the flow rate may be any suitable buffer such as sucrose or glycerol. In some embodiments, the LFT device comprises a buffer for modifying the flow rate, which is 5% glycerol. The blocking agent may be any suitable blocking agent known in the art, such as a blocking agent comprising BSA, PBS and Tween-20.

*Kit*

The present invention also provides kits which comprise one or more LFT devices described above. The kit may also comprise instructions for using the LFT device, and/or further reagents and/or components for carrying out the method of the present invention. The further reagents and/or components may be, for example, swabs for obtaining a sample (e.g. a mucus or saliva sample), mixing tubes or liquids for preparing a liquid sample (for example, a mucus or saliva sample may be mixed with a solution in a mixing tube before being applied to the LFT device). The further reagents and/or components may be packaged separately from the LFT device(s) within the kit, such as in individual vials, tubes or wrappers. In addition, an LFT reader as described above for measuring the signal intensity at each test region may be included with the kit. Alternatively, or in addition, the kit may comprise instructions for downloading and/or using software on a user device (e.g. a smartphone) for measuring the signal intensity at each test region.

Examples

Example 1 - Detection of caffeine

In this Example, the colorimetric detection of caffeine using a modified competitive LFT format (Figure 3) is demonstrated. Instead of having only one test line, two test lines are used in the LFT and the signal intensities at both of the test lines correlate to the concentration of caffeine present in the sample. The assay was based on the use of a AuNP-BSA-caffeine-biotin conjugate as the detection complex, and immobilised anti-caffeine antibody and avidin as two respective test lines. The free caffeine in the sample competes with a gold-labelled conjugate for binding to the anti-caffeine antibodies on the first test line, resulting in the signal intensity of the first test line being inversely proportional to the caffeine concentration present. The displaced conjugate is then captured by avidin at the second test line, hence the signal intensity of the second test line is positively correlated to the caffeine concentration present. This increases the maximum sensitivity of the assay for the detection of lower caffeine concentrations.

**Methods**

*General Materials and Methods*

NHS-LC-biotin, 1-ethyl-3-(3-dimethylaminopropyl) carbodiimide hydrochloride (EDC), avidin, HRP-labelled

anti-mouse secondary antibody and HRP-labelled avidin were purchased from Thermo Fisher Scientific. Monoclonal anti-caffeine antibody raised in mouse was purchased from Stratech Scientific. Gold(III) chloride hydrate, bovine serum albumin (BSA), theophylline-7-acetic acid, caffeine, 2-(4-hydroxyphenylazo)benzoic acid (HABA), 2,4,6-trinitrobenzenesulfonic acid (TNBS) solution and phosphate buffered saline (PBS) tablets were purchased from Sigma Aldrich. Buffers used were made in Milli-Q water. PD-10 columns prepacked with Sephadex G-25 medium were purchased from GE Healthcare Life Sciences. Costar 3370 high binding 96-well plates used for the enzyme-linked immunoassay (ELISA) were purchased from Corning. Hi-Flow Plus membrane HFB07502 purchased from Merck Millipore was used for the lateral flow assay. Test lines on the nitrocellulose membrane were made by dispensing the respective antibody or avidin solution using a

Scienion SciFlexArrayer with a Delivery Piezo Dispense Capillary (PDC).

*General Techniques*

i) UV-Vis Spectroscopy

UV-Vis spectroscopy was carried out using a Spectramax M3 microplate reader, from 350 to 750 nm, with a resolution of 1 nm. Blanks according to the buffer used in each sample were subtracted from the UV-Vis spectra as background correction.

ii) Nanoparticle Tracking Analysis (NTA)

Nanoparticle tracking analysis (NTA) was done on a Nanosight Halo LM10 detection instrument equipped with a 635 nm Laser. The samples were filtered through a 0.2 μm PTFE membrane and injected into the sample chamber using a 1 mL syringe. The captured videos (60 s) were processed using Halo 2.3 analytical software.

iii) Dynamic Light Scattering (DLS)

Dynamic light scattering (DLS) measurements were performed using DTS1061 disposable folded capillary cells on a Malvern Zetasizer Nano S instrument equipped with a 633 nm laser. Two measurements were conducted per sample with 20 runs lasting 10 s each. The z-average and zeta potential of the samples were analysed using Malvern Instruments dispersion technology 7.11 software.

iv) Transmission Electron microscopy (TEM)

Nanoparticles were characterised using TEM on a JEOL JEM-2100F machine operating at 200kV. Samples (10 μL) were dispensed onto holey carbon films on 300 mesh copper grids, purchased from Agar Scientific. The excess was removed by placing the edge of the copper grid close to a filter paper. For samples requiring negative staining, 5% (w/v) ammonium molybdate solution (10 μL) was added after the sample and the excess was removed using a filter paper. TEM experiments of the prepared samples were run by the Department of Materials at Imperial College. Size measurements were done using ImageJ software, and the average of at least 50 particles was calculated for each sample.

*Synthesis of Gold Nanoparticles*

A modified protocol of the Frens method was used. Aqua regia was prepared by mixing concentrated nitric acid and hydrochloric acid in a 1:3 volume ratio. All glassware used in the synthesis was cleaned thoroughly with aqua regia, rinsed with Milli-Q water and dried in an oven overnight. Gold(III) chloride hydrate (30 mg, 0.0762 mmol) was dissolved in Milli-Q water (250 mL) to give a pale yellow solution. The solution was heated under reflux for 30 min. Sodium citrate (500 mg, 1.94 mmol) in Milli-Q water (5 mL) was added rapidly to the boiling solution. The solution developed a red-wine colour and was heated for another 10 min. It was left to cool to room temperature and filtered through a 0.2 μm PTFE membrane. It was then characterized with UV-Vis spectroscopy, NTA, DLS and TEM.

*Preparation and Testing of BSA-biotin-caffeine Conjugates*

i) Biotinylation

Standard biotinylation protocol was followed. BSA (12 mg, 0.180 μmol) was dissolved in 0.1 M sodium phosphate, 0.15 M NaCl, pH 7.2 (1.2 mL) and added to NHS-LC-biotin (1 mg, 2.20 μmol) in N,N-dimethylformamide (DMF) (25 μL). The reaction was left at room temperature for 3 hours and kept at 4°C overnight. The sample was purified by

gel filtration with 0.1 M sodium phosphate, 0.15 M NaCl, pH 7.2 (3 mL) as the eluting buffer. Fractions containing the biotinylated BSA were identified by recording the absorbance at 286 nm. The fractions were combined and the concentration of BSA present was determined using a standard curve.

ii) HABA Assay

4'-Hydroxyazobenzene-2-carboxylic acid (HABA) (24.2 mg, 0.100 mmol) was dissolved in Milli-Q water (9.9 mL). 1 M NaOH (100 μL) was added and the solution was filtered through a 0.22 μm Millipore filter. Avidin (10 mg) and HABA solution (600 μL) were added to phosphate buffered saline (PBS) (19.4 mL) to form an orange solution. Each biotinylated sample (20 μL) was added to the HABA/avidin solution (180 μL). The absorbance values at 500 nm were recorded and used to determine the level of biotinylation according to the following equation:

$$Ratio\ of\ biotin\ to\ BSA = \frac{\Delta A_{500} \times 10}{\varepsilon_{HABA-avidin} \times l \times [BSA]}$$

where $\Delta A_{500}$ is the change in absorbance at 500 nm, $\varepsilon_{HABA\text{-}avidin}$ is the extinction coefficient of the HABA-avidin complex, l is the cell path length and [BSA] is the concentration of BSA.

iii) Hapten Conjugation

Theophylline-7-acetic acid solution (1.49 μmol, 89 μL, 4 mg/mL in DMSO) was added to 1-ethyl-3-(3-dimethylaminopropyl)carbodiimide hydrochloride (EDC) (20 mg). Biotinylated BSA solution (0.090 μmol, 600 μL, 10 mg/mL in 0.1 M sodium phosphate, 0.15 M NaCl, pH 7.2) was added. The reaction mixture was left to react at room temperature for 2 hours, after which it was kept at 4°C before gel filtration. The sample was purified by gel filtration with 0.1 M sodium phosphate, 0.15 M NaCl, pH 7.2 (3 mL) as the eluting buffer. Fractions containing the maximum protein concentration were identified by recording the absorbance at 286 nm and combined.

iv) TNBS Assay

An assay protocol reported by Sashidhar was used (R. B. Sashidhar, A. K. Capoor and D. Ramana, J. Immunol. Methods, 1994, 167, 121-127). The BSA conjugates were diluted with 0.1 M $NaHCO_3$, pH 8.0 to obtain a final concentration of 200 μg/mL (1 mL). 0.01% (w/v) 2,4,6-trinitrobenzene sulfonic acid (TNBS) (500 μL, in 0.1 M $NaHCO_3$) was added to each sample. The samples were incubated at 37.0°C for 2 hours. 10% (w/v) SDS (500 μL) and 1 M HCl (250 μL) was added to each sample. The absorbance was recorded at 335 and 420 nm. The same procedure was used with L-lysine and L-glutamic acid to obtain a standard curve for comparison. The absorbance at 335 nm was used to calculate the amino group concentration in the sample. The degree of conjugation was determined by the difference in the number of amino groups per BSA before and after conjugation.

v) Capture Enzyme-Linked Immunosorbent Assay (ELISA)

A 96-well ELISA plate was coated with anti-caffeine antibody (50 μL, 6 μg/mL, in PBS buffer, pH 7.4) by incubating the plate for 1 hour at RT. The plate was blocked with 2% (w/v) BSA in PBS with 0.05% (v/v) Tween-20, pH 7.4, for 2 hours at RT. BSA-biotin-caffeine conjugate (50 μL) at different concentrations were added to the plate and incubated for 1 hour at RT. HRP-labelled avidin (50 μL, 1:5000 dilution in blocking buffer) was added and the plate was incubated for 1 hour in the dark at RT. After thorough washing of the plate, the colour was developed by adding 3,3',5,5'-tetramethylbenzidine (TMB) liquid substrate system (50 μL). The reaction was stopped by the addition of 1 M $H_2SO_4$ (50 μL) and the absorbance at 450 nm was recorded.

vi) Competitive ELISA

Same general protocol as direct ELISA. Plate was coated with anti-caffeine antibody (50 μL, 6 μg/mL, in PBS buffer, pH 7.4) and blocked with 2% (w/v) BSA in PBS with 0.05% (v/v) Tween-20, pH 7.4. BSA-biotin-caffeine conjugate (25 μL, 40 mg/mL) was mixed with caffeine (25 μL) at different concentrations of the latter, added to the plate and incubated for 1 hour at RT. HRP-labelled avidin (50 μL, 1:5000 dilution) was added and the colour was developed by adding TMB liquid substrate system (50 μL). The reaction was stopped by the addition of 1 M $H_2SO_4$ (50 μL) and the absorbance at 450 nm was recorded.

*Preparation of AuNP-BSA Conjugates*

i) Salt-Induced Aggregation Method

A salt-induced aggregation method was used to determine the minimum amount of BSA required to stabilise the colloidal gold. Serial dilution was performed on BSA (15 mg/mL in 0.05 M sodium phosphate buffer, pH 7.0) to obtain BSA solution (25 μL) of concentrations from 0.15 mg/mL to 5 mg/mL. Colloidal gold (250 μL) was added to each sample and the solutions were left to react for 5 min. 1.7 M NaCl solution (250 μL) was added and the UV Vis spectrum of each sample was recorded after 5 min.

ii) Adsorption Method

Colloidal gold (5 mL) was added to BSA (3 mg) in 0.05 M sodium phosphate (200 μL). 1% (w/v) polyethylene glycol (PEG, MW 20000) (125 μL) was added for the stabilisation and the reaction mixture was left to react at room temperature for 2 h. The mixture was centrifuged at 12000 rpm for 40 min. The supernatant was removed and the pellet was reconstituted in 0.05 M sodium phosphate (5 mL) and 1% PEG (125 μL). The AuNP-BSA conjugate was characterised by UV-Vis spectroscopy.

iii) Reduction Method

2-Mercaptoethanol (0.50 μL, 25 mM) was added to BSA-biotin-caffeine conjugate (300 μL, 0.697 mg, 2.32 mg/mL). The reaction mixture was well mixed and left to react at 4°C overnight. The sample was purified by gel filtration with a PD10 column and with 0.1 M sodium phosphate, 0.15 M NaCl, pH 7.2 (3 mL) as the eluting buffer. Fractions observed to contain the protein conjugate by UV-Vis spectroscopy at 286 nm were pooled (600 μL) and used in the next step. The reduced conjugate was added slowly to colloidal gold (500 μL). The reaction was left to react over 2 days at 4°C. The mixture was then centrifuged at 13400 rcf for 30 min for 3 times, washing with 10 mM Tris containing 3% (w/v) BSA each time. The pellet was reconstituted in 10 mM Tris containing 3% (w/v) BSA and filtered through a 0.2 μm PTFE syringe filter. The conjugate was characterised with TEM, with 5% (w/v) ammonium molybdate and 0.1% (w/v) trehalose as a negative stain. The conjugate was also characterised with UV-Vis spectroscopy, NTA, DLS and TEM.

iv) Ellman's Assay

An assay reported by Aitken and Learmonth (A. Aitken and M. Learmonth, The Protein Protocols Handbook, Humana Press, Totowa, NJ, 3rd edn., 2009) was used. Ellman's reagent solution was prepared by dissolving 5',5'-dithiobis(2-nitrobenzoic acid) (DTNB) (0.40 mg, 1.08 μmol) in 0.1 M sodium phosphate, 1 mM EDTA, pH 8.0 (5 mL). Different concentrations of cysteine hydrochloride (0.25 mM to 1.5 mM) were prepared by serial dilution with 0.1 M sodium phosphate, 1 mM EDTA, pH 8.0 for use as standard solutions. Ellman's reagent solution (200 μL) was added to each sample (20 μL) and the mixture was left to react for 15 min. The absorbance at 412 nm was recorded and a standard curve generated using the cysteine standard solutions was used to determine the sulfhydryl content of each sample.

v) HABA Assay with Proteinase Digestion

AuNP-BSA-biotin-caffeine conjugate (25 μL) was heated at 56°C for 10 min. 1% (w/v) Proteinase K solution (2.5 μL) was added to the sample and the mixture was left at room temperature overnight. The sample was centrifuged at 13400 rcf for 5 min. The supernatant (20 μL) was added to HABA-avidin solution (180 μL) and the absorbance at 500 nm was recorded.

*Preparation of Lateral Flow Assay (LFA)*

i) Assembly of LFA

The LFA was assembled using a nitrocellulose membrane attached to a plastic backing card. Anti-caffeine antibody and avidin were immobilised at test lines 1 and 2, respectively, in the detection zone. The membrane was dried at RT for 1 hour and then soaked in 2% (w/v) BSA in PBS with 0.05% (v/v) Tween-20, pH 7.4 for 5 min to block any excess binding sites. The membrane was dried at RT for 2 hours. The wick was attached to the membrane (2 mm overlap) and the plastic card was cut into strips (5 mm x 60 mm). Glass fibre conjugate pads were soaked in 30% (w/v) sucrose in Milli-Q water and dried in an oven at 37°C. They were then soaked in AuNP-BSA-biotin-caffeine conjugate and dried at RT for 2 hours. The conjugate pad was attached to the membrane and the prepared strips were kept in a desiccator until use.

ii) Testing of LFA strips

Different concentrations of caffeine in 2% (w/v) BSA were prepared in respective wells of a low-binding 96-well microplate. The test strips were placed into each well vertically for 3 min, after which they were removed and left to dry. Images of the test strips were taken and analysed by ImageJ software. The grey level of the pixels in each test line was measured and subtracted from the background.

**Results & Discussion**

*Synthesis and Characterisation of BSA-biotin-caffeine Conjugates*

i) Synthetic Strategy

A carrier protein is required as a scaffold for the conjugation of biotin and a caffeine derivative. The carrier protein has to be large, with a molecular weight of over 20,000 Da and sites for the conjugation of small molecules. It is also important that the carrier protein selected is not that same as the carrier protein used in the generation of the anti-caffeine antibody. As the anti-caffeine antibody used in the lateral flow assay was raised in mouse using keyhole limpet hemocyanin (KLH)-3-caffeine, BSA was chosen as the carrier protein for the conjugation of biotin and the caffeine derivative. BSA (MW 67,000 Da) is water soluble and stable, and has around 30 to 35 accessible lysine residues that make it suitable for conjugation. It is commercially available in pure form, relatively cheap, and its use as a carrier protein has been well documented (B. Law and W. N. Jenner, in Immunoassay: A Practical Guide, UK Taylor & Francis, London, 2005, pp. 16-21).

The synthetic strategy for the preparation of the BSA-biotin-caffeine conjugate is shown in Figure 4. Biotin and caffeine are attached to BSA via its primary amino groups on the lysine residues.

ii) Biotinylation

BSA was biotinylated using NHS-LC-biotin and purified by gel filtration. NHS-LC-biotin contains a biotin moiety attached to an active ester group, which reacts with the ε-amino group of lysine residues in BSA to form an amide bond. The presence of the 6-aminocaproníc acid spacer arm reduces steric hindrance and improves the accessibility of the biotin moiety for binding to avidin (D. Kim and A. E. Herr, Biomicrofluidics, 2013, 7, 41501).

Excess NHS-LC-biotin and the N-hydroxysuccinimide (NHS) by-product were removed by filtering the reaction mixture through a desalting resin. The fractions containing the BSA biotin conjugates were identified by the absorbance at 286 nm in the gel elution profile.

The level of biotinylation was determined by 4'-hydroxyazobenzene-2-carboxylic acid (HABA) assay. HABA interacts with avidin at its biotin-binding sites to form an orange complex with an absorption peak at 500 nm. As the binding affinity of biotin for avidin ($1.3 \times 10^{15}$ $M^{-1}$) is much higher than that of HABA for avidin ($6.0 \times 10^{6}$ $M^{-1}$), addition of biotin to the HABA-avidin complex readily displaces HABA from the biotin-binding site (G. T. Hermanson, Bioconjugate Techniques, Elsevier, 3rd edn., 2013). Addition of the intact BSA-biotin conjugate to the HABA-avidin complex resulted a decrease in absorbance observed at 500 nm compared to that in the HABA-avidin complex, indicating successful biotinylation. The change in absorbance at 500 nm is used to calculate the concentration of biotin in the sample and the extent of biotinylation.

Different concentrations of biotinylating reagent were used but aggregation of BSA was observed with larger molar excess of NHS-LC-biotin. The optimal molar excess of biotinylating reagent over BSA was determined to be 12, which gave 1.8 biotin molecules per BSA molecule without any significant aggregation.

iii) Hapten Conjugation

Since there was no functional group in caffeine that was suitable for covalent attachment to BSA, a caffeine derivative had to be used for the conjugation. Theophylline-7-acetic acid was chosen as the caffeine derivative to be conjugated to BSA-biotin via a free carboxylic acid group. Water soluble 1-ethyl-3-(3-dimethylaminopropyl)carbodiimide hydrochloric (EDC) was used as the coupling reagent. EDC reacts with the carboxylic acid group on theophylline-7-acetic acid to form a highly reactive O-acyl isourea intermediate. The ε amino group of lysine residues in BSA-biotin then reacts with the activated group to form an amide bond. While the ideal pH for carboxylate activation by EDC was reported to be at pH 3.5 to 4.5 (G. T. Hermanson, Bioconjugate Techniques, Elsevier, 3rd edn., 2013), precipitation of BSA-biotin was observed in MES buffer at pH 4.0. Li et al. (R. Li, Z. Wu, Y. Wangb, L. Ding and Y. Wang, Biotechnol. Reports, 2016, 9, 46-52) reported that at low pH, the hydrophobicity of BSA increases as unfolding of the protein results in increased accessibility of hydrophobic groups. In addition, the biotinylation of BSA resulted in hydrophobic aliphatic biotin

modifications that increased the tendency of the protein to aggregate in solution. While no aggregation was observed when the conjugation was carried out using BSA in MES buffer at pH 4.0, the presence of aggregates formed when the same conjugation was done using BSA-biotin suggested the need to increase the pH of the reaction buffer. A sodium phosphate buffer with a pH of 7.2 was used instead as the reaction buffer and the amide formation was observed to proceed without any protein aggregation occurring.

The conjugate was purified by gel filtration to remove the excess hapten, EDC and the isourea by-product. The fractions containing the BSA-biotin-caffeine conjugate were identified by the absorbance at 286 nm in the gel elution profile.

The hapten density is affected by the stoichiometric ratio of reagents, conditions of coupling and carrier protein used (G. T. Hermanson, Bioconjugate Techniques, Elsevier, 3rd edn., 2013). Determination of hapten density is important for optimisation of the performance of the BSA conjugate for use in an immunoassay. Direct methods such as measuring the change in absorbance, fluorescence or mass of the protein are commonly employed to assess hapten density (R. Lemus and M. H. Karol, in Allergy Methods and Protocols, eds. M. G. Jones and P. Lympany, Humana Press, Totowa, NJ, 2008, pp. 167-182. While theophylline-7-acetic acid is a strong chromophore, it has a maximum absorbance at 275 nm (J. A. Owen and K. Nakatsu, Clin. Chem., 1978, 24, 367-368), which overlaps significantly with the absorbance of BSA. Hence, the absorbance in the UV region cannot be used to quantify the extent of conjugation.

The amount of hapten conjugated was instead determined by an indirect spectroscopic method using 2,4,6-trinitrobenzenesulfonic acid (TNBS). TNBS reacts with primary amines on the surface of the protein to give an orange derivative.

Successful hapten conjugation via the lysine residues on BSA would decrease the number of primary amines available in BSA. The absorbance at 335 nm was used to determine the concentration of primary amines present in the sample by comparing with a standard curve generated using L-lysine and L-glutamic acid. The difference in the number of free amines on BSA before and after conjugation was used to determine the extent of conjugation.

The number of primary amines available in each BSA molecule before conjugation was determined to be 40.8 according to the TNBS assay. BSA has 59 lysine residues in total, while 30 to 35 of the lysine residues are considered available for conjugation due to their positions near the surface. As the TNBS assay required heating to 37°C, unfolding of the BSA protein structure likely resulted in more lysine residues being available, hence the value obtained is higher than expected for surface lysine residues. The same thermal treatment was applied to all samples, hence the degree of unfolding should be fairly consistent among the samples provided that the conjugation did not affect the stability of the protein. A comparison between the values calculated for the three samples was used to determine the extent of conjugation. The difference between the number of primary amines available on BSA and BSA-biotin was found to be 2.4, which is consistent with the value of 1.8 obtained using the HABA assay. By comparing the absorbance of the BSA-biotin and BSA-biotin caffeine conjugates, the conjugation reaction was found to give an average of 3.1 caffeine molecules per BSA molecule.

*Synthesis and Characterisation of AuNP-BSA Conjugates*

i) Synthesis of AuNPs

AuNPs were synthesised using the citrate reduction method reported by Frens (G. Frens, Nat. Phys. Sci., 1973, 241, 20-22). The citrate ions act as a reducing agent to reduce Au(III) to Au(0) for the growth of the gold colloid. The negatively charged citrate ions also stabilise the surface of the AuNPs as they are formed (J. Kimling, M. Maier, B. Okenve, V. Kotaidis, H. Ballot and A. Plech, J. Phys. Chem. B, 2006, 110, 15700-15707). The size of the gold colloid formed is dependent on the concentration of stabilising ligands in the solution. A higher concentration of the stabilising ligands results in smaller AuNPs being formed as the ligands stabilise the surface of the gold colloid and prevent further growth in size.

The AuNPs were observed to be red in colour and the absorption peak was found to be at 519 nm using UV-Vis spectroscopy. The average core diameter of the AuNPs, assuming that the citrate coating was not visible, was determined to be 16 ($\pm$2) nm by transmission electron microscopy (TEM). The results are consistent with those reported in the literature (N. Sosibo, F. Keter, A. Skepu, R. Tshikhudo and N. Revaprasadu, Nanomaterials, 2015, 5, 1211-1222).

ii) Synthesis of AuNP-BSA Conjugates

Proteins can bind to AuNPs mainly through three types of interactions: hydrophobic interactions via the tryptophan residues, electrostatic interactions via the lysine residues and gold-thiol bonding via the cysteine residues (M. Valcárcel and Á. López-Lorente, Gold Nanoparticles in Analytical Chemistry, Elsevier, 1st edn., 2014). Of the three, Brewer et al. (S. H. Brewer, W. R. Glomm, M. C. Johnson, M. K. Knag and S. Franzen, Langmuir, 2005, 21, 9303-9307) reported that the passive adsorption of BSA to AuNPs occurs mainly via electrostatic interactions between the positively

charged lysine residues on BSA and the negatively charged citrate coating. It is critical to ensure that sufficient protein is conjugated to the surface of the colloidal gold. The stability of the AuNPs is determined by a balance of electrostatic repulsive forces and van der Waals attractions between the AuNPs. Insufficient protein coating on the surface would result in the instability of the AuNPs in solutions with high salt content.

A salt-induced aggregation method (C. Fang, Z. Chen, L. Li and J. Xia, J. Pharm. Biomed. Anal., 2011, 56, 1035-1040) was used to determine the minimum amount of BSA required to stabilise the colloidal gold. Colloidal gold was added to different concentrations of BSA, followed by the addition of 1.7 M NaCl solution. As the kinetics of BSA adsorption and hence the maximum amount of BSA bound to the surface of the AuNPs are dependent on the BSA concentration, AuNPs with low concentrations of BSA will be unstable upon the addition of NaCl. The salt solution causes flocculation to occur between unstabilised AuNPs by shielding the negative charges of the AuNPs and causing them to aggregate. The flocculation can be visualised as a change in the colour of the solution from red to purple. With decreasing concentration of BSA used, a red shift in the absorbance peak is observed from 523 nm to 620 nm upon addition of 1.7 M NaCl.

The absorbance of each sample at 523 nm and 620 nm was recorded and plotted against the BSA concentration. The minimum concentration of BSA required to stabilise the AuNPs was determined to be 2 mg/mL, at which no significant shift in the absorption peak from 523 nm to 620 nm was observed.

BSA-biotin-caffeine conjugate was added to the AuNPs and incubated overnight for the passive adsorption to take place. However, the complex formed was observed to be unstable in solution. Black insoluble solid was observed at the bottom of the sample tubes after complexation of the BSA-biotin-caffeine conjugate to the AuNPs, even when a large excess of the conjugate was used. The presence of a black solid indicates aggregation due to the poor stabilisation of the AuNPs. As the conjugation of biotin and the caffeine derivative to BSA occurs via the lysine residues, the surface of BSA becomes less positively charged. The electrostatic interactions are hence not as strong, and the AuNPs are only weakly stabilised. The adsorption method was deemed unsuitable for conjugation of the BSA biotin-caffeine conjugate onto the surface of the AuNPs.

Kaur et al. (J. Kaur, K. V. Singh, R. Boro, K. R. Thampi, M. Raje, G. C. Varshney and C. R. Suri, Environ. Sci. Technol., 2007, 41, 5028-5036) reported that the AuNP-protein conjugates formed via the gold-thiol bond are more stable compared those formed via electrostatic interactions between the protein and the surface of AuNPs. BSA has 1 free cysteine residue and 17 disulfide linkages which can be cleaved to provide free thiol groups for conjugation with the AuNPs (I. Rombouts, B. Lagrain, K. A. Scherf, M. A. Lambrecht, P. Koehler and J. A. Delcour, Sci. Rep., 2015, 5, 12210). 2-Mercaptoethanol was used to reduce the intrachain disulfide bonds in BSA to create free sulfhydryl groups that can be bonded to the AuNPs. The reduced BSA-biotin-caffeine conjugates were then purified by gel filtration and the concentration of sulfhydryl groups present was determined by a spectroscopic method using Ellman's reagent, 5,5'-dithiobis(2-nitrobenzoic acid) (DTNB). DTNB reacts with free sulfhydryl groups in the sample to produce a mixed disulphide and a yellow product, 2-nitro-5-thiobenzoic acid (TNB). The concentration of sulfhydryl groups was calculated by comparing the absorbance of the solution at 412 nm to a standard curve generated using L-cysteine.

The difference in the number of sulfhydryl groups after reduction of BSA was determined. The number of free sulfhydryl groups per BSA molecule increased from 0.9 to 2.7 upon treatment with 2-mercaptoethanol.

The free sulfhydryl groups on the BSA displace the citrate ions stabilising the AuNPs and interact directly with the surface of the AuNPs. No black solid was observed upon complexation of the reduced BSA conjugate to the AuNPs, indicating sufficient stabilisation of the AuNPs. The AuNP-BSA-biotin-caffeine conjugate formed was stable even after two months when kept at 4°C.

iii) Characterisation of AuNPs and AuNP-BSA Conjugates

a) UV-Vis Spectroscopy

AuNPs exhibit localised surface plasmon resonance (LSPR) due to the oscillations of the surface conduction electrons upon absorption of light (C. Larosa, E. Stura, R. Eggenhöffner and C. Nicolini, Materials (Basel), 2009, 2, 1193-1204). The LSPR signal can be detected as a strong absorption peak in the visible region of a UV-Vis spectrum. The intensity and wavelength of the LSPR peak observed are dependent on various factors including the size and shape of the AuNPs and the dielectric constant of the solution (X. Huang and M. A. El-Sayed, J. Adv. Res., 2010, 1, 13-28).

The absorption maximum of the citrate-stabilised AuNPs was observed at 519 nm. A red shift from 519 nm to 523 nm upon reaction with BSA indicated successful adsorption of the protein to the AuNPs. The red shift of the LSPR peak is consistent with the experimental results reported by Balog et al (S. Balog, L. Rodriguez-Lorenzo, C. A. Monnier, M. Obiols-Rabasa, B. Rothen-Rutishauser, P. Schurtenberger and A. Petri-Fink, Nanoscale, 2015, 7, 5991-5997. Mie theory predicted that an increase in refractive index would lead to a red shift in absorption maximum peak (S. Dominguez-Medina, S. McDonough, P. Swanglap, C. F. Landes and S. Link, Langmuir, 2012, 28, 9131-9139). The red shift in the peak wavelength is possibly attributed to an increase in the refractive index of the AuNPs upon protein adsorption to the surface.

b) Size characterisation

Due to the high electron density of the AuNPs, they can be visualised using TEM for size characterisation. The AuNPs were spherical, monodisperse and 16 (±2) nm in size. The size and quality of the AuNPs are important for determining the sensitivity of a lateral flow assay (LFA) (C. Fang, Z. Chen, L. Li and J. Xia, J. Pharm. Biomed. Anal., 2011, 56, 1035-1040). Small AuNPs less than 10 nm in diameter migrate quickly but provide weakly visible signal at the test lines and limit the sensitivity of the assay. While AuNPs larger than 40 nm provide intense red signal at the test lines, they suffer from slow migration through the pores of the membrane, cause steric hindrance for ligand-receptor binding and are less stable towards flocculation (S. Lou, J. Ye, K. Li and A. Wu, Analyst, 2012, 137, 1174). The 16-nm AuNPs obtained were stable and provided a clearly visible signal in the LFA. The AuNPs synthesised were deemed to be of good quality due to narrow size distribution and uniform shape.

Due to the poor electron scattering ability of proteins, the use of a heavy metal as a negative stain was necessary to visualise the BSA-biotin-caffeine conjugate bound to the AuNPs. 5% (w/v) Ammonium molybdate was used as a negative stain. A white halo indicating the BSA conjugate around each AuNP was observed. The conjugation of BSA on the surface of the AuNPs resulted in the formation of a protein shell around each AuNP, and the AuNP-BSA-biotin-caffeine conjugate formed was spherical. The size of the AuNP-BSA-biotin-caffeine conjugate was found to be 19 (±2) nm. The thickness of the shell was approximately 3 nm, which corresponds to the diameter of BSA reported in literature (M. Su, C. Wang and C. Bai, Chinese Sci. Bull., 1998, 43, 1882-1886). The TEM measurements are indicative of the formation of a monolayer of BSA around each AuNP. The AuNP-BSA-biotin-caffeine conjugates were well stabilised as no aggregation of the particles was observed by TEM.

Nanoparticle tracking analysis (NTA) and dynamic light scattering (DLS) were also employed as alterative techniques to characterise the size of the AuNPs and the AuNP-BSA-biotin caffeine conjugate synthesised. NTA relies on tracking the Brownian motion of individual nanoparticles to calculate the nanoparticle size based on the Stokes-Einstein equation while DLS relies on the fluctuations in scattered light intensity due to Brownian motion of the nanoparticles to determine the nanoparticle size (B. J. Frisken, Appl. Opt., 2001, 40, 4087). The average diameter for the AuNPs was measured to be 17 nm and 24 nm using NTA and DLS respectively. The particle size reported using NTA was more consistent with the particle size determined by TEM. As the light scattering intensity is proportional to the sixth power of the nanoparticle diameter, DLS is more sensitive for larger particles, leading to an overestimation of the average nanoparticle size.

An increase in the hydrodynamic diameter was observed upon the binding of the BSA biotin-caffeine conjugates to the AuNPs. The change in hydrodynamic diameter was measured to be 5 nm using NTA and 7 nm using DLS. As the DLS measurements by Li et al. (Y. Li, G. Yang and Z. Mei, Acta Pharm. Sin. B, 2012, 2, 53-59) found the hydrodynamic diameter of BSA to be 7.2 nm, the NTA and DLS measurements of the AuNP-BSA-biotin-caffeine conjugate support the formation of a BSA monolayer around each AuNP core.

The average surface area of the 16-nm AuNPs (804.25 $nm^2$) and the diameter of the BSA molecules (3 nm) were used to calculate a theoretical maximum of 119 BSA molecules bound to each AuNP for monolayer coverage. The number of BSA-biotin-caffeine molecules conjugated around each AuNP was determined experimentally using the HABA assay with Proteinase K digestion. The HABA assay cannot be used directly with the AuNP-BSA-biotin caffeine conjugate as the region of absorbance of the AuNP overlaps with that of HABA avidin complex. The concentration of the AuNPs was determined by UV-Vis spectroscopy based on the correlation between the size of the AuNPs and its extinction coefficient at 450 nm ($\varepsilon_{450}$) (W. Haiss, N. T. K. Thanh, J. Aveyard and D. G. Fernig, Anal. Chem., 2007, 79, 4215-4221. A $\varepsilon_{450}$ value of $2.67 \times 10^8$ was used to calculate the concentration of the AuNPs from its absorbance at 450 nm. The AuNP-BSA-biotin-caffeine conjugates were subjected to Proteinase K digestion, which allowed the biotin moieties to be cleaved from the AuNP conjugates. The AuNPs were removed by centrifugation and the supernatant was analysed by HABA assay to determine the biotin concentration. The ratio of biotin moieties to the AuNPs was used to calculate the ratio of BSA molecules to AuNPs. An average of 56 BSA molecules were bound to each AuNP, and this value is below the theoretical maximum calculated based on the size of the AuNPs. This corresponds to a surface coverage of $6.96 \times 10^{12}$ BSA molecules/cm2, which is above the minimum surface coverage of $3.8 \times 10^{12}$ BSA molecules/$cm^2$ required to avoid the aggregation of the AuNPs as reported by Shi et al. (X. Shi, D. Li, J. Xie, S. Wang, Z. Wu and H. Chen, Chinese Sci. Bull., 2012, 57, 1109-1115). This indicates that the AuNPs were well stabilised by the BSA-biotin-caffeine conjugates on the surface.

c) Zeta Potential Characterisation

The zeta (ζ) potential of the citrate-stabilised AuNPs and AuNP-BSA-biotin-caffeine conjugates were characterised by DLS. Zeta potential measurements provide an indication of stability for charge-stabilised nanoparticles. Sufficient electronic repulsion is present among particles with zeta potential above 25 mV or below -25 mV to prevent aggregation (S. H. Brewer, W. R. Glomm, M. C. Johnson, M. K. Knag and S. Franzen, Langmuir, 2005, 21, 9303-9307. The

zeta potential of the AuNPs synthesised was -37.2 mV. The highly negative surface charge indicated that the AuNPs were well stabilised by the citrate coating. An increase in zeta potential from - 37.2 mV to -31.5 mV was observed upon conjugation of the BSA-biotin caffeine conjugate to the AuNPs. This indicated some displacement of the negatively charged citrate molecules at the AuNPs upon BSA binding via thiol-gold bonds, supporting successful conjugation. The overall surface charge is still negative as BSA is negatively charged at pH above its isoelectric point of 4.7.

d) Aggregation Studies with Avidin

The binding ability of the AuNP-BSA-biotin-caffeine conjugate to avidin was examined by incubating the conjugate with avidin overnight. Avidin is a tetrameric protein with four biotinbinding sites. Kim et al. (W.J. Kim, S.-H. Choi, Y.-S. Rho and D.-J. Yoo, Bull. Korean Chem. Soc., 2011, 32, 4171-4175) reported that the specific biotin-avidin interaction would cause biotinylated AuNPs to aggregate together. This was observed as a slow colour change from red to purple after the addition of avidin. The aggregation was dependent on the concentration of avidin present. Aggregation would be observed when the avidin bound to biotin moieties on one AuNP is able to cross-link with adjacent AuNPs with unsaturated biotin sites. At a low concentration of avidin (0.1 μg/mL), no aggregation was observed, as there was insufficient avidin for binding to the biotin moieties on the AuNPs. At a higher concentration of avidin (1 μg/mL), aggregation of the AuNPs was observed as each avidin binds to at least two biotin moieties on two different AuNPs. A significant broadening and red shift of its absorption peak from 523 nm to 548 nm was observed after overnight incubation. The red shift of the absorption peak is attributed to LSPR coupling interactions arising from the polarisation of the conduction electron oscillations (Y. Yang, S. Matsubara, M. Nogami and J. Shi, Mater. Sci. Eng. B, 2007, 140, 172-176) between adjacent AuNPs linked by avidin molecules. The peak broadening is due to variations in the coupling interactions as they are highly dependent on the size of the aggregates and the orientation of the AuNPs within the aggregate (K. Aslan, C. C. Luhrs and V. H. Pérez-Luna, J. Phys. Chem. B, 2004, 108, 15631-15639).

The aggregation induced by the avidin molecules binding to the biotin on the AuNPs was also visualised using TEM. This showed that the binding site of biotin to avidin was not hindered by the binding of the BSA-biotin-caffeine conjugate to the AuNP. This also confirms that the treatment of BSA-biotin-caffeine with 2-mercaptoethanol did not affect the binding of biotin to the protein.

*Enzyme-Linked Immunosorbent Assay (ELISA)*

i) Capture ELISA

A capture enzyme-linked immunosorbent assay (ELISA) was carried out to determine the binding ability of the BSA-biotin-caffeine conjugate to both the anti-caffeine antibody and avidin. The format of the capture ELISA used is shown in a schematic representation in Figure 2.

The wells were coated with anti-caffeine antibodies and excess binding sites were blocked with BSA. Other blocking agents such as Tween-20 and a commercially available synthetic blocker (ELISA SynBlock) were tested but the signal-to-noise ratio obtained was poor due to high non-specific binding. Milk-protein based blocking agents such as casein were avoided as trace amounts of biotin present may affect the specific biotin-avidin interaction in the assay. Using 2% (w/v) BSA as the blocking agent ensured that the signal obtained was due to the BSA-biotin-caffeine conjugate specifically binding to the anti-caffeine antibody and not due to the hydrophobic or electrostatic interactions of the conjugate with the well.

Different concentrations of the BSA-biotin-caffeine conjugate were added to the respective wells and HRP-labelled avidin was added. The 3,3',5,5'-tetramethylbenzidine (TMB) substrate then reacted to form a chromogenic derivative in the presence of horseradish peroxidase (HRP) enzyme. The colour developed is proportional to the amount of bound BSA-biotin-caffeine conjugate present. The absorbance of the solution at 450 nm was recorded and plotted against the concentration of the BSA-biotin-caffeine conjugate. The presence of a signal confirmed that the BSA-biotin-caffeine conjugate synthesised was able to bind successfully to both the anti-caffeine antibody and avidin at the same time. With increasing concentration of the BSA-biotin-caffeine conjugate, more HRP-labelled avidin was captured by the biotin-avidin interaction, resulting in increasing absorbance observed at 450 nm. The absorbance against concentration plot is non-linear as there is increasing saturation of the anti-caffeine antibody binding sites at high concentrations of the BSA-biotin-caffeine conjugate.

ii) Competitive ELISA

The same ELISA format was used to test the competition of the BSA-biotin-caffeine conjugate and free caffeine for binding to the anti-caffeine antibody coated on the wells. A fixed amount of BSA-biotin-caffeine conjugate (20 μg/mL) was used and the concentration of free caffeine was varied. The absorbance at 450 nm was plotted against the

concentration of caffeine in a semi-log graph as shown in Figure 5. The data was fitted with a dose response model and the $IC_{50}$ value was found to be 1 ng/mL. The detection limit, determined by the concentration of caffeine that results in 10% inhibition, was found to be 0.03 ng/mL.

The competitive ELISA was repeated using the AuNP-BSA-biotin-caffeine conjugate to determine if the conjugation with the AuNPs had any effect on the binding ability of the BSA-biotin-caffeine conjugate to the anti-caffeine antibody and avidin. A similar semi-log graph was plotted as shown in Figure 6. The data was fitted with a dose response model and the $IC_{50}$ value was found to be 7 ng/mL. The detection limit was 0.4 ng/mL. There is a slight decrease in the sensitivity of the assay when AuNP-BSA-biotin-caffeine was used, possibly attributed to the steric hindrance of the AuNP conjugate. As the AuNP-BSA-biotin caffeine conjugate is quite large, the binding of a AuNP conjugate to an antibody might block the binding of other AuNP conjugates to the adjacent antibodies. However, the assay was sufficiently sensitive for the intended application, as the range of caffeine to be detected in blood is 0.1 to 10 $\mu$g/mL.

The ELISA results demonstrated that the AuNP-BSA-biotin-caffeine conjugate retained binding ability to both the anti-caffeine antibody and avidin. The competition established between the AuNP-BSA-biotin-caffeine conjugate and free caffeine can be used to determine the caffeine concentration in a sample accurately with a low detection limit.

*Lateral Flow Assay (LFA)*

i) Optimisation of Assay Design

The competition between the AuNP-BSA-biotin-caffeine conjugate and free caffeine for binding to the anti-caffeine antibody was used to develop a LFA. The initial design of the LFA for the detection of caffeine is shown in Figure 7. The anti-caffeine antibody was incubated with excess AuNP-BSA-biotin-caffeine conjugate. The antibody-AuNP-BSA-biotin-caffeine complex that was obtained after centrifugation to remove excess antibody was deposited on the conjugate pad. Anti-mouse antibody was coated on the first test line to capture all of the anti-caffeine antibodies. Free caffeine present in the sample would displace the AuNP BSA-biotin-caffeine conjugate from the anti-caffeine antibody and the AuNP-BSA-biotin caffeine conjugate displaced will be captured at the second test line via the specific biotin avidin interaction.

Initial testing with this format was carried out; however, the signal observed at test line 1 was extremely weak. This indicated that the binding of the anti-caffeine antibody-AuNP BSA-biotin-caffeine complex to the secondary antibody was poor. The dissociation of some anti-caffeine antibodies from the complex might have competed with antibody-AuNP-BSA biotin-caffeine complex and saturated the binding sites on the anti-mouse antibodies. The binding of the AuNP-BSA-biotin-caffeine conjugate to the anti-caffeine antibody might have also posed steric hindrance to the binding of the secondary antibody. The epitope on the caffeine antibody that is recognised by the anti-mouse antibody might be blocked by the AuNP label, resulting in a significant decrease in sensitivity. The binding kinetics involved in this format were deemed too slow and unfavourable for the detection of caffeine.

A modified competitive LFA format without the use of the secondary antibody was proposed as shown in Figure 3. Instead of drying the anti-caffeine antibody with the AuNP-BSA-biotin-caffeine conjugate at the conjugate pad, the anti-caffeine antibody was deposited as the first test line. The competition for the caffeine antibody between the AuNP-BSA-biotin-caffeine conjugate and the free caffeine in the sample hence occurs at the test line instead of along the membrane.

ii) Assay Working Principle

The working principle and the expected test results of using the LFA design in Figure 7 are shown in a schematic representation in Figure 8. The AuNP-BSA-biotin-caffeine conjugate is deposited on the conjugate pad. Upon application of the sample, the liquid flows along the membrane and mobilises the gold conjugate. In the absence of caffeine in the sample (Figure 8a), the AuNP-BSA-biotin-caffeine conjugate is captured by the anti-caffeine antibody at the first test line. In the presence of caffeine in the sample (Figure 8b), the free caffeine competes with the AuNP-BSA-biotin-caffeine conjugate for binding to the anti-caffeine antibody. The AuNP-BSA-biotin-caffeine conjugate that is displaced from the first test line binds to the avidin at the second test line. The signal intensity at the second test line is proportional to the concentration of caffeine present in the sample.

iii) Optimisation of LFA Conditions

Various conditions such as the amount of conjugate, the concentration of antibody and avidin at the test lines and the blocking of non-specific binding were optimised for assay performance.

a) Blocking of Membrane

High background signal was observed when no blocking agents were used in the sample buffer. Non-specific binding of the AuNP-BSA-biotin-caffeine conjugate to the negatively charged nitrocellulose membrane occurred via hydrophobic and electrostatic interactions, leading to a pale pink background on the rest of the nitrocellulose membrane after each run. Washing the membrane with PBS buffer after the run did not remove the background signal, hence blocking agents were required to reduce the non-specific binding. The same blocking buffer (2% (w/v) BSA in PBS with 0.05% (v/v) Tween-20) used for ELISA was tested in the LFA and found to significantly reduce the background signal.

b) Lateral Flow Rate

The capillary flow rate decreases exponentially through the membrane (G. A. Posthuma-Trumpie, J. Korf and A. van Amerongen, Anal. Bioanal. Chem., 2008, 393, 569-582), hence test lines further from the sample pad provide more time for interaction between the ligand and receptor. As the anti-caffeine antibody test line was dispensed relatively close to where the sample was applied, the quick flow rate of the sample might not have allowed enough time for the AuNP-BSA-biotin-caffeine conjugate to interact with the anti-caffeine antibody. The migrating speed of the AuNP-BSA-biotin-caffeine conjugate can be slowed by introducing agents that reduce the lateral wicking rate of the membrane. For example, sucrose has been commonly used for slowing the flow rate in paper-based assays (B. Lutz, T. Liang, E. Fu, S. Ramachandran, P. Kauffman and P. Yager, in 16th International Conference on Miniaturized Systems for Chemistry and Life Sciences, Okinawa, Japan, 2012, vol. 20, pp. 788-790) as it has excellent aqueous solubility and flow impedance properties (WO 2005069007 A1, 2005, 1-25). 30% (w/v) sucrose was dried on the conjugate pad to form a sucrose glaze, which can then be dissolved upon application of the sample to slow the flow rate along the membrane. The intensity of the second test line was observed to decrease relative to that of the first test line; however, the reduced flow rate was still insufficient to trap all of the AuNP-BSA-biotin-caffeine at the first test line. Higher concentrations of sucrose were not tested as concentrations above 50% (w/v) sucrose were found to be too viscous for handling. Upon drying on the conjugate pad, the sucrose glaze made the conjugate pad significantly stiffer and possibly interfered with the capture and release of AuNP-BSA-biotin-caffeine conjugate at the conjugate pad.

Hua et al. (F. Hua, P. Zhang, F. Zhang, Y. Zhao, C. Li, C. Sun, X. Wang, R. Yang, C. Wang, A. Yu and L. Zhou, Sci. Rep., 2015, 5, 17178) reported that the use of PEG and glycerol affected the signal intensities at the test and control lines in a LFA due to the high viscosities of the solutions. The use of glycerol in the optimisation of the LFA was hence explored. Different concentrations of glycerol were tested as shown in Figure 9. Two lines of anti-caffeine antibody were dispensed in the first test zone and one line of avidin was dispensed in the second test zone. The second line of anti-caffeine antibody was aimed at trapping any excess AuNP-BSA-biotin-caffeine conjugate that migrated past the first line of anti-caffeine antibody. However, this was still unsuccessful at capturing all of the AuNP-BSA-biotin-caffeine conjugate before it reached the avidin test line. When no glycerol was used, three lines were observed in a test strip ran with only buffer solution. While the addition of 2.5% (v/v) glycerol reduced the signal intensity at the second test zone, it was still insufficient to trap all of the conjugate at the first test zone (Figure 9).

The addition of 10% (v/v) glycerol was observed to be sufficient to trap all of the AuNP-BSA biotin-caffeine conjugate at the first test line. However, a test run with a sample containing 100 μg/mL of caffeine failed to produce a positive signal at the second test line (Figure 9). The presence of glycerol slowed down the migration of the AuNP-BSA-biotin-caffeine conjugate too much such that the competition between the free caffeine and the conjugate for the anti-caffeine antibody could not take place at the first test line. The free caffeine molecules in the sample have a much lower molecular weight compared to the AuNP-BSA-biotin caffeine conjugate, hence they migrate a lot quicker than the conjugate through the membrane even in the presence of glycerol. Kim et al. (Y. A. Kim, E. H. Lee, K. O. Kim, Y. T. Lee, B. D. Hammock and H. S. Lee, Anal. Chim. Acta, 2011, 693, 106-113) previously reported that the relative migration speed between the two competing molecules is critical for the sensitivity of a competitive LFA.

While the analyte in the sample should migrate ahead of the conjugate, the migration speed of the analyte should only be slightly higher than that of the conjugate so that sufficient overlap between the two competing molecules can be achieved on the membrane.

5% (v/v) glycerol was found to be optimal for slowing the migration speed of the AuNP-BSA biotin-caffeine conjugate without adversely affecting the competition between the conjugate and free caffeine (Figure 9). Most of the AuNP-BSA-biotin-caffeine conjugates were successfully captured by the anti-caffeine antibody at the first test zone. When a sample containing 100 μg/mL of caffeine was run, competition could be achieved between the migrating AuNP-BSA-biotin-caffeine conjugate and free caffeine, resulting in a strong signal at the second test zone.

iv) Sensitivity Testing

The effects of applying different concentrations of caffeine to the LFA were investigated to determine the working

range and sensitivity that can be achieved for caffeine detection. The LFA test strips were prepared according to the design of the assay shown in Figure 3 and run using different concentrations of caffeine ranging from 1 ng/mL to 100 μg/mL. The optimised running buffer included glycerol as an agent to regulate wicking rate, and BSA and Tween-20 as blocking agents to reduce non-specific binding. The results of the test strips are shown in Figure 10.

With increasing concentration of caffeine, there was a corresponding decrease in the intensity of test line 1 as expected for a competitive assay. No colour was developed at test line 1 for caffeine concentrations above 10 μg/mL. This is the visual detection limit for detecting the presence of caffeine in a sample if no calibration was performed. This is an inherent limitation of traditional competitive assays, as a signal will always be present at the test line except when the analyte is present at a sufficiently high concentration to saturate the test line completely. Without a standard curve to calibrate the signal intensity against the concentration of target analyte, the assay only provides a qualitative result for the presence of caffeine in concentrations above 10 μg/mL. This falls short of the working range required for the detection of caffeine in blood. The sensitivity of an assay can be improved by using silver (R.-H. Shyu, H.-F. Shyu, H.-W. Liu and S.-S. Tang, Toxicon, 2002, 40, 255-258) or gold enhancement methods (J. Kaur, K. V. Singh, R. Boro, K. R. Thampi, M. Raje, G. C. Varshney and C. R. Suri, Environ. Sci. Technol., 2007, 41, 5028-5036) to amplify the signal. However, such methods are undesirable as the convenience of a one-step LFA is lost.

This limitation was overcome by having a second test line to capture the AuNP-BSA-biotin caffeine conjugate displaced from binding to anti-caffeine antibody by the free caffeine in the sample. There was a corresponding increase in the signal intensity of test line 2 with increasing caffeine concentrations in the sample (Figure 10). With the second test line, the visual detection limit is improved to 10 ng/mL. This provides a 1000-fold increase in sensitivity of the assay for caffeine detection. The positive signal of test line 2 in the presence of caffeine concentrations above 10 ng/mL makes qualitative analysis of caffeine content in a sample more straightforward, as compared to the negative readout associated with traditional competitive assays. The improved sensitivity makes this assay suitable for the testing of blood samples with caffeine concentration between 0.1 μg/mL and 10 μg/mL.

As well as visual inspection, the signal intensity of the test lines was analysed using ImageJ software. The signal intensities of test lines 1 and 2 at different caffeine concentrations were plotted in a semi-log graph as shown in Figure 11 (results were measured in triplicates). The $IC_{50}$ values of test lines 1 and 2 are 5 ng/mL and 8 ng/mL respectively. The $IC_{50}$ values are comparable to that obtained in the competitive ELISA. A comparison of the ratio of the signal intensities of the two test lines can be used for the semi-quantitative analysis of caffeine content in samples as shown in Table 1.

Table 1 - Semi-quantitative analysis of caffeine concentration based on the ratio between the signal intensities of test lines 1 and 2.

| Caffeine concentration | Test Line 1 | Test Line 2 | Signal Intensity Ratio (Test Line 2/Test Line 1) |
|---|---|---|---|
| **None** | Intense signal | No signal | 0 |
| **Low** | Stronger signal | Weaker signal | <1 |
| **Moderate** | Equal signal | Equal signal | 1 |
| **High** | Weaker Signal | Stringer signal | >1 |
| **Very High** | No signal | Intense signal | ∞ |

v) Stability of Test Strips

The shelf life stability of LFA test strips are important as the strips are typically produced in batch and kept for some period of time before use. The stability of the reagents printed on the nitrocellulose membrane and the AuNP-BSA-biotin-caffeine conjugate deposited on the conjugate pad was examined. The test strips were prepared and kept in a desiccator for seven days at room temperature. The test was run with different concentrations of caffeine, and the results are shown in Figure 12. The visual detection limit for caffeine concentration remained at 10 ng/mL for test line 2.

The signal intensities of test lines 1 and 2 at different caffeine concentrations were plotted in a semi-log graph, and the $IC_{50}$ values of test lines 1 and 2 were 3ng/ml and 11ng/ml, respectively. There was no significant change in the sensitivity of the assay after storage for one week, indicating that the reagents maintained good stability and reactivity after being dried on the membrane. Traditional competitive and sandwich LFAs incorporate a positive control line that confirms the validity of the test result by ensuring the reactivity of the reagents present in the assays. While no control line was incorporated in this format, the two test lines serve as a crosscheck and the presence of at least one of the lines is indicative of the assay being valid. In the case of degradation of the reagents on the strips, the reactivity of both the anti-caffeine antibody and avidin should be diminished and no colour should develop at either line.

*Conclusion*

A competitive LFA was successfully developed for the colorimetric detection of caffeine, with the signal intensity being proportional to the caffeine concentration present in the sample. The binding ability of the AuNP-BSA-biotin-caffeine conjugate to avidin and the anti-caffeine antibody was demonstrated using ELISA. A LFA suitable for the detection of caffeine concentration was developed by optimising the design, blocking and flow rate of the modified competitive LFA format.

The main advantages offered by this LFA format are its high sensitivity and positive readout. The incorporation of a second test line increases the sensitivity by 1000-fold, resulting in a visual detection limit of 10 ng/mL for caffeine. This makes the working range of the assay suitable for detecting caffeine concentrations in blood samples, compared to a commercially available LFA for caffeine detection74 which lacks the required sensitivity. The test is also quick, producing qualitative results upon visual inspection of the test strip after 5 min of applying the sample. No specialised equipment or additional washing steps are needed, and only a small amount of sample is required to run the test. This makes it easy to administer as a finger prick blood test.

This Example also serves as a proof-of-concept of a competitive format that allows for the detection of small target analytes with a positive readout. This overcomes the limitations of traditional competitive assays, which often have poor sensitivity and narrow analyte detection range. The format of this competitive assay can be applied to the detection of other small molecules by modifying the hapten conjugated to BSA and the antibody immobilised at the first test line. The use of two test lines allows for some cross-referencing of the result, and the ratio of the signal intensities of the two test lines can be used to provide semi-quantitative data. The one-step assay does not require any washing, enhancement or calibration steps, and is relatively low cost and convenient to use as a point-of-care test.

Example 2 - Detection of NHPA

The objective of this study was to develop an *in vitro* diagnostic test to measure NHPA in urine in the form of a modified competitive lateral flow immunoassay and to demonstrate its performance (sensitivity, limit of detection, linearity) using laboratory (i.e. non-clinical) samples.

This work was focused on using 3-Nitrotyrosine (3-NTyr) antibody and lateral flow assay (LFA) to deliver a point-of-care (POC) test for detection of NHPA in solution.

There are no reports of an antibody specific for NHPA; however, a BSA conjugate of NHPA is reported to bind to a monoclonal antibody against 3-Nitrotyrosine (3-NTyr) and this antibody would therefore be expected to bind to NHPA as well as 3-NTyr. Although 3-NTyr is also found in urine, its concentrations are several hundred-fold lower than NHPA and therefore is not expected to interfere with the assay. The following work was conducted to assess feasibility of the 3-NTyr antibody to detect NHPA in urine in the modified competitive assay format.

*A - Conjugate Synthesis*

i) Coupling of biotin to BSA

1% BSA (20 mg, 0.3 μmol) was dissolved in 0.1 M sodium phosphate, 0.15 M NaCl, pH 7.2 (2 mL) and added to NHS-LC-biotin (1.12 mg, 2.46 μmol) in dimethyl sulfoxide (DMSO) (40 μL). The reaction was left on ice for 2 hours. The sample was then purified by gel filtration with 20mM Borax buffer pH 8.0 as the eluting buffer. Fractions containing the biotinylated BSA were identified by recording the absorbance at 280 nm and combined.

To quantitate biotinylation, a solution containing the biotinylated protein is added to a mixture of 4'-Hydroxyazobenzene-2-carboxylic acid (HABA) and avidin. Because of its higher affinity for avidin, biotin displaces the HABA and the absorbance at 500nm decreases proportionately.

The amount of biotin present in the solution was quantitated by measuring the absorbance of the HABA-avidin solution before and after addition of the biotin-containing sample, using the same equation as set out in Example 1, above.

The biotinylation reaction was successful and the calculation showed that the synthesised product has on average 3.3 molecules of biotin per 1 molecule of BSA.

ii) Coupling of NHPA to biotinylated BSA

3-nitro-4-hydroxyphenylacetic acid (NHPA) solution (1.43 μmol, 5.64 μL, 50 mg/mL in ethanol) was added to 1ethyl-3-(3-dimethylaminopropyl)carbodiimide hydrochloride (EDC) (6.85 mg) in DI water (3000 μL, pH 4.5). Biotinylated BSA solution from step 1 (0.24 μmol, 3000 μL, 5.28 mg/mL in 20 mM Borax buffer, pH 8.0) was added. The reaction mixture was left to react on the rotator at room temperature for 2 hours, and then overnight at 4 °C. The sample was purified by gel filtration with 2 mM Borax buffer (pH 8.0) as the eluting buffer. Fractions containing the maximum protein concentration

were identified by recording the absorbance at 280 nm and combined.

Spectral analysis, using a calibration curve, was performed to quantitatively assess the success of the reaction. Based on the calculation, the synthesised product has on average 3.6 molecules of NHPA per 1 molecule of BSA.

iii) Conjugation of NHPA-biotin-BSA to gold nanoparticles

The spectral analysis of gold nanoparticles (PBS stabilised) showed a distinct peak at 523 nm, which is as expected for 20 nm nanoparticles.

Gold nanoparticles (GNP) (1 mL, 1 OD) were first centrifuged at 14000 rpm for 5 min. The solution appeared clear and nanoparticles pellet formed on the bottom of the Eppendorf tube. The supernatant was carefully removed without disturbing the pellet. BSA-Biotin-NHPA coupling in 2 mM Borax buffer pH 8.0 (400 μg/ml, 100 μL) was added to GNP pellet. The mix was rotated at RT for 4 h.

The conjugate was then centrifuged for 15 min at 8500 rpm, the supernatant removed, and finally the GNP were resuspended in 1 mL of buffer containing 20 mM tris, 2%BSA, 4% sucrose, 5% glycerol, 0.05% tween 20, pH 7.2.

*B - Trial of Strips in Urine*

In this part, the performance of conventional LFAs for NHPA detection under real background conditions was assessed.

i) Lateral Flow Assay Assembly

The LFA was assembled using a nitrocellulose membrane attached to a plastic backing card (Figure 13). An anti-3-Nitrotyrosine antibody line was printed at 1mg/mL concentration, the line consisted of 20 lines, each with 25 μm width, using 0.1 μL antibody solution/cm/line. A streptavidin line was printed at 1mg/mL concentration and consisted of 10 lines, each with 50 μm width, using 0.1 μL streptavidin solution/cm/line. The membrane was sealed in a foil bag with desiccant silica gel sachets and stored in the fridge until used.

On the test day the wicking pad (5 x 17 mm) was attached to the membrane (2 mm overlap) and the plastic card was cut into strips (5 mm width). Glass fibre conjugate pads (5 x 7 mm) were soaked in AuNP-BSA-biotin-NHPA conjugate (0.5 OD) and dried in a desiccator for 2 hours. The conjugate pad was then attached to the membrane (2mm overlap). The sample pad (5 x 17mm) was soaked in the running buffer solution (20 mM tris, 2% (w/v) BSA, 4% (w/v) sucrose, 5% (v/v) glycerol, 0.05% (v/v) tween 20, pH 7.2), dried at RT overnight and then attached to the membrane to overlap the conjugate pad by 2mm.

ii) Testing of LFA strips

Different concentrations of NHPA in synthetic urine (200 μL) were prepared in respective wells of a low-binding 96-well microplate. The test strips were placed into each well vertically and left until the flow front reached the end of the wicking pad, after which they were removed and imaged. The images of the test strips (Figure 14) were analysed by ImageJ software. The ImageJ analysis showed two peaks for the streptavidin line and the antibody line (Figure 15a). The grey level of the pixels in each test line was measured and subtracted from the background to calculate each line intensity and its dependence on the NHPA concentration (Figure 15b).

With increasing NHPA concentration, less AuNP-BSA-biotin-NHPA conjugate was captured by the anti-3-nitrotyrosine antibody, leading to a linear reduction in the signal intensity at the antibody test line (Figure 15b). At 250 ng/ml of NHPA in urine samples, the antibody line's intensity is shown to have decreased by over 90%.

The reproducibility of the assay was assessed using three strips for each concentration of NHPA (Figure 16). It was then compared to the strips prepared from two other batches of AuNPs. The results are summarised in Figure 17, which is an Image J analysis of the antibody lines, where each data point is the average of five strips. The analysis of test lines for all strips showed good reproducibility (Figure 17).

Figure 17 shows the linear response between 0 and 150 ng/ml of NHPA concentration, after which the response plateaus, meaning that all the antibody sites have been occupied by the NHPA in solution.

Conclusion: The results presented here clearly demonstrate a working assay for NHPA detection in urine, with a threshold of 170-330 ng/ml (250 ± 80 ng/ml). The proposed protocol is suitable to distinguish between the samples above and below the threshold, as positive and negative samples, respectively. Figure 18 shows that positive samples resulted in a visible line only at the streptavidin test line, as expected, and shows that the assays had good reproducibility.

Example 3 - Detection of creatinine

Using a similar method to that set out in Example 1, the LFT design of Figure 3 was used for the detection of creatinine.

**Methods**

*Gold nanoparticle synthesis*

Gold(lll)chloride hydrate (30 mg, 0.0762 mmol) was added to Milli Q water (250 ml) and heated under reflux for 30 mins. Sodium citrate (500 mg, 1.94 mmol) solution was added to it. After heating for another 30 mins, it was cooled and filtered using a 0.2 mM PTFE membrane, to give a red color solution. Characterization was done using UVvis spectroscopy and NTA. This was an altered approach of the Frens protocol.

*Creatinine derivative synthesis*

Ethyl-4-bromobutyrate (26.6 ml, 100 mmol) was added to Creatinine (9g, 80 mmol) in DMF(50ml) and heated at 85oC for 4 hours. Ethyl acetate (500ml) was then added after cooling the solution to form a precipitate (12.71g, 52% yield) that was then filtered off. The precipitate (3.1g, 10 mmol) was added to KOH (10 mmol) in methanol (25 ml). Methanol was evaporated off and ethanol was added. Precipitated salt was removed by vacuum filtration and the filtrate was heated to evaporate the ethanol. The solution was then added to NaOH (0.5 M, 10ml) and heated under reflux for 6 hours. Ethanol was heated off and concentrated HCl was added until pH was 3. The water was evaporated off to give a yellow oil and NaCl that was removed by centrifugation. The product was characterized by NMR, Mass spectroscopy and IR.

*Biotin conjugation to BSA*

NHS-LC-Biotin (2mg, 4.40 μmol) was dissolved in DMF (50ml) and added to BSA (24 mg, 0.360 μmol) dissolved in 1.2 ml buffer (0.1M sodium phosphate, 0.15 M NaCl, pH 7.2). The mixture was kept at room temperature for 5 hours and then gel filtrated, using the same buffer. UV vis spectroscopy at 286 nm was done of the different fractions and the fractions containing the maximum absorbance were combined.

*HABA Assay*

4'Hydroxyazobenzene-2-carboxylic acid (HABA) (25 mg, 0.103 mmol) was added to Milli Q water containing 1 M NaOH (100 mM). Avidin (12mg) was dissolved in PBS (19.4 ml, 0.02 mM, pH-7.4) and filtered HABA solution (600 ml) was added to it. The mixture was added to the biotinylated samples and the absorbance at 500 nm recorded.

*Creatinine conjugation to BSA*

Creatinine derivative (20 μL) was added to EDC (20mg) in phosphate buffer solution (80μmol, pH7.4). It was kept for 4 hours at room temperature, and then purified by gel filtration using the same phosphate buffer solution. UV vis was used to detect the maximum protein concentration.

*TNBS assay*

BSA conjugates (200 μg/ml), were added to 2,4,6 trinitrobenzene sulfonic acid(TNBS) (500 μL, in 0.12 M NaHCO3) and incubated for 3 hours at 37 0C. The absorbance at 335 nm were recorded to calculate the amino group concentration.

*Capture ELISA*

Anti-creatinine antibody (50 μL, 8 μg/ml ) was added to a 96 well ELISA plate and 1 hour and 2% BSA in PBS(10 μL) was added to it. Different concentrations of BSA-biotin creatinine (50 μL) were added. HRP-labelled avidin (50 μL) was added and the plate was left in the dark for 1 hr. The wells were then washed four times using PBS buffer and then TMB (50 μL) was added to from blue colour. H2SO4 (50 μL, 1M) was added and the colour changed to yellow. The absorbance were then recorded 450 nm.

*Competitive ELISA*

The same method was used as above. Only in this case, the BSA conjugates were kept constant of 25 μL and different concentrations of creatinine were added of total volume 50 μL.

*Conjugation of gold to BSA conjugate*

The reduction method described in Example 1 was used. BSA conjugates were added to 2 Mercaptoehanol (0.50 μL, 25mM) and kept at room temperature for 6 hours. It was then gel filtrated using the phosphate buffer. 1000 μL of the sample were added to filtered gold particle solution (1ml) and left for 3 days at 4°C. The mixture was then centrifuged for 1 hour at 13,400 rcf and the supernatant removed. The pellet was dissolved in 3% BSA solution (1ml, 10 mM Tris buffer). This was characterized using NTA and UV VIS spectroscopy.

*Ellman's Assay*

5'5'-dithiobis 2 nitro benzoic acid (DTNB) (0.42mg, 1.22 μmol) was added to sodium phosphate buffer (0.1M, pH 8, 1mM EDTA, 5 mL) to form the Ellman's reagent. Reduced BSA samples (20 μL) were added to Ellman's reagent (180 μL) and absorbance at 412 nm recorded using UVvis spectroscopy.

*Making of LFA strips*

Sample pads were attached to the backing card. Nitrocellulose membranes were attached and an anti-creatinine antibody and avidin were immobilised on it in two straight lines (a first line for the antibody and a second line for the avidin). 1% BSA solution was added to the membrane and dried. Strips were made and wick was attached at the other end. The conjugate pads were soaked in 30% sucrose solution and AuNP-BSA-biotin-creatinine conjugates were added twice and dried for 2 hours. The pads were then attached to the strips.

*Testing LFA strips*

Different concentrations of creatinine solutions were made and added to the wells of the 96 well plates. The strips were placed in the wells, by immersing the sample pads in solution for 10 mins. After drying, images were taken by a camera and analyzed by Image J software. The values obtained were plotted in a semi log graph (Figure 20).

**Results and Discussion**

*Biotinylation*

As in Example 1, the biotin conjugated to the BSA was quantified using a HABA assay. The ratio of biotin to BSA was determined to be 2.2 biotin per BSA.

*Creatinine derivative synthesis*

Creatinine itself does not have any carboxylic acid group attached to it, so a derivative was synthesized that contains an acid group that can then form an amide linkage with the BSA. The following steps were carried out to synthesize the creatinine derivative, as also shown in Scheme 1.

Step 1: creatinine was reacted with ethyl 4 bromobutyrate to form a yellow solid intermediate, 4-(2-imino-3-methyl-5-oxoimidazolidin-1-yl) butyric acid ethyl ester hydrobromide.

Step 2: the hydrobromide was then neutralized using the base KOH.

Step 3: 4-(2-imino-3-methyl-5-oxoimidazolidin-1-yl) butyric acid ethyl ester was heated under reflux with a base, to remove the ester group.

Step 4: a strong acid was used to convert the sodium carboxylate salt to, 4-(2- imino-3-methyl-5-oxoimidazolidin-1-yl) butyric acid which is the creatinine derivative. NaCl was produced which was deposited at the bottom.

Scheme 1:

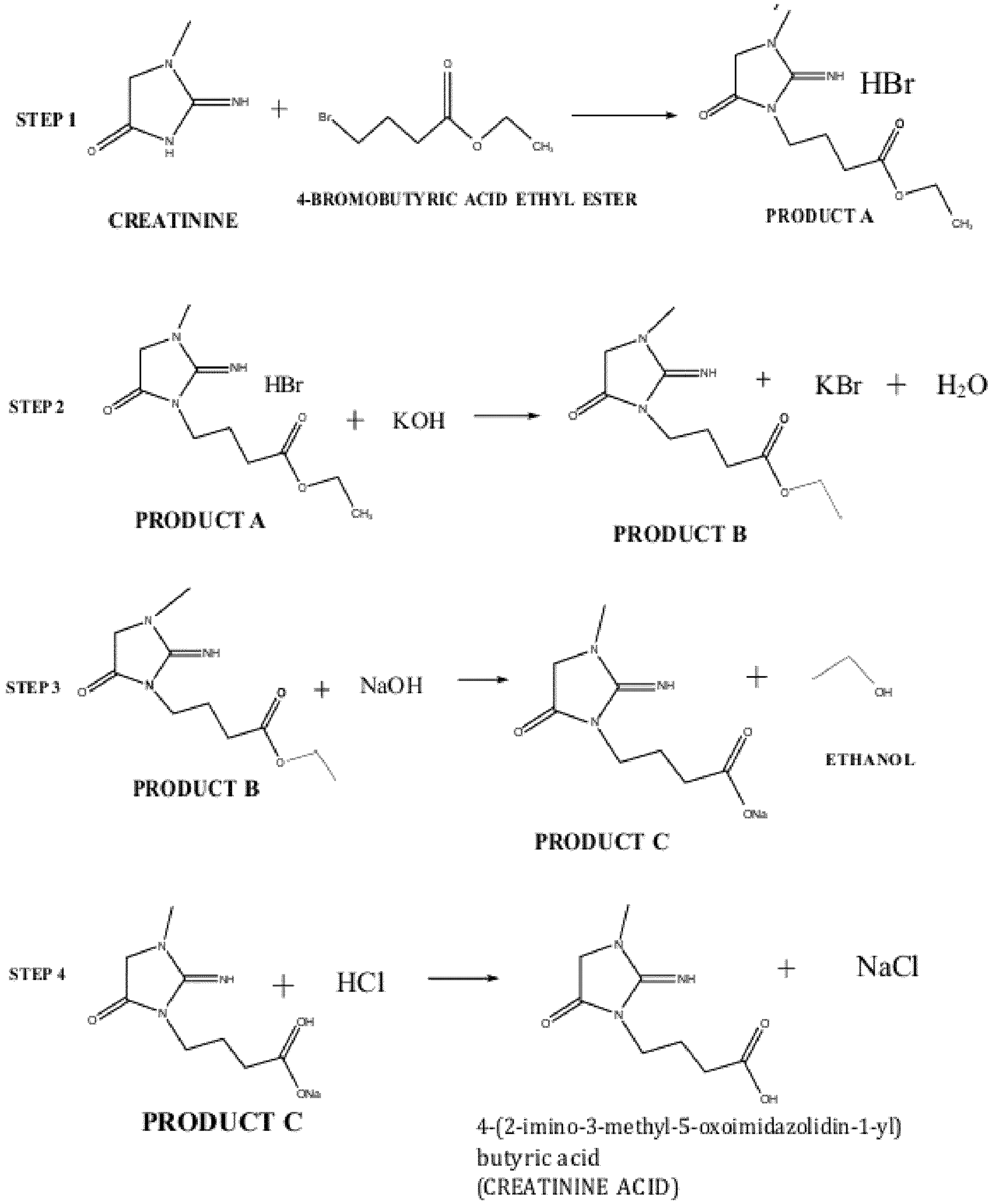


The creatinine derivative was then characterised by NMR, IR and mass spectroscopy.

i) Infrared Spectroscopy (IR)

IR was used to confirm the carboxylic acid group attached to the creatinine molecule. The broad peak to the far left at 3375 $cm^{-1}$ confirmed the OH stretch of the COOH group. The sharp peak at 1697 $cm^{-1}$ confirmed the C=O of the COOH. The values were within the range of the literature values, as shown in Table 2.

Table 2 - IR values

| Functional Group | Value observed ($cm^{-1}$) | Literature value ($cm^{-1}$) |
|---|---|---|
| O-H | 3375 | 300-350 |
| C=O | 1697 | 1680-1720 |

ii) NMR

$^1$HNMR and $^{13}$CNMR were carried out. The literature value of creatinine and 4-amino butyric acid ($H_2N-CH_2-CH_2-CH_2-COOH$) were used to compare the structure of the creatinine derivative. The creatinine part of the product was in the range of literature values of creatinine. The carboxylic acid part of the product was also in the range of literature values for 4-aminobutyric acid. One $CH_2$ moiety of the carboxylic acid was not within the literature ranges - the HNMR range should have been within 3.25 to 3.75 ppm, but the value observed was 4.36 ppm. This difference is reasonable as the reference literature value was for $CH_2$ attached to $NH_2$, whereas, in the creatinine derivative, the $CH_2$ was attached to a nitrogen which is part of a five-membered ring. Resonance within the ring causes deshielding of the electrons of atoms bonded to it. Higher deshielding leads to chemical shifts further to the left. The same was observed in the CNMR. All values were within the range except the same $CH_2$ as discussed in relation to the HNMR, which had a value of 70.61 ppm. This higher chemical shift was hence due to the deshielding of electrons caused by resonance in the ring structure.

The OH of the COOH group was also not observed in the HNMR spectra. $D_2O$ was used as the solvent in HNMR, and the H and the D in solution can exchange with each other resulting in COOD and HOD formation. Deuterium is NMR active but the signal is of different energy and is not seen in the NMR spectra. Hence, the OH signal was not observed in the HNMR spectra.

Carbonyl carbon also showed no signal in the $^{13}$CNMR spectrum. However, the IR spectroscopy already confirmed the presence of the carboxylic acid group in the creatinine derivative.

iii) Mass Spectroscopy

The electrospray ionization technique was used to characterize the creatinine derivative. The molecular weight of the creatinine derivative was 199, and a large peak at 200 was seen, confirming the presence of the product.

Therefore, a creatinine derivative was successfully synthesised that contained a carboxylic acid group to attach to the BSA via an amide bond.

*Creatinine derivative conjugation*

This creatinine acid derivative containing a carboxylic acid group could then bond to the BSA. EDC was added to activate the derivative. The carboxylic acid group of the derivative reacts with EDC to form an intermediate that can then form an amide bond with the lysine residues in BSA. The mixture was purified by gel filtration to remove the excess creatinine derivative, EDC and the side product produced.

The first 3 fractions had the maximum BSA conjugates and hence these three fractions were combined and used in the next step.

To determine the amount of creatinine conjugated, 2,4,6 trinitrobenzene sulfonic acid (TNBS) was used, and the results are shown in Table 3 below.

Table 3 - Determination of substitution of amino group by TNBS assay

| | BSA | BSA-biotin | BSA-biotin-creatinine |
|---|---|---|---|
| Absorbance at 335nm | 0.2125 | 0.2030 | 0.1869 |
| Concentration of amine/$10^{-5}$M | 4.4318 | 4.1303 | 3.6103 |
| Number of primary amines per BSA | 35.2 | 32.81 | 28.67 |

Absorbance of BSA-biotin-creatinine was lower than that of BSA-biotin, which was further lower than that of BSA. As BSA was conjugated with biotin and creatinine via amide bonds, fewer primary amines were therefore available. As a result, less TNBS could bind to BSA, resulting in a decrease in the orange product formation and hence a decrease of the colour intensity.

The values for the concentration of amine were taken from standard Lysine and glutamic acid curves.

There are 30-35 lysine residues that are available on the surface of a BSA molecule for conjugation, out of total 59

lysine residues. This was consistent with the data that determined 32.6 primary amines per BSA. Biotin conjugation resulted in a decrease to 30.35, which is a net decrease of 2.4 amines. This was consistent with the HABA assay value of 2.1 biotin per BSA.

Approximately 2 biotin molecules bonded to each BSA molecule. Further creatinine conjugation resulted in a decrease to 26.53 amines per BSA, i.e. another net decrease of 3.82 molecules. Therefore, on average, there were approximately 4 creatinine molecules per BSA molecule.

*Gold nanoparticle synthesis and conjugation with BSA*

Example 1 above showed that the adsorption method of conjugating BSA to gold nanoparticles was not suitable, with incubation, for passive adsorption to occur, resulted in aggregation. In particular, black insoluble solids were deposited. The electrostatic interaction between the gold nanoparticles and the BSA as not strong enough to cause efficient gold-BSA conjugation. Therefore, the reduction method was used to conjugate the creatinine-biotin-BSA to the gold nanoparticles.

Ellman's reagent containing DTNB was used to quantify the sulfhydryl groups present in the BSA, using absorbance at 412 nm. The absorbance was seen to increase in reduced BSA conjugate compare to non-reduced BSA conjugate. The concentration of the sulfhydryl groups was calculated from the standard L-cysteine curve. The number of sulfhydryl groups increased significantly from 0.81 to 2.51, with a net increase of 1.7, confirming the reduction of the BSA conjugates.

Gold nanoparticle (AuNP) samples were then added to the reduced BSA conjugate. The sulfhydryl groups can then form bonds with the gold nanoparticles. An UV vis was done to characterise the AuNP and the AuNP-BSA conjugates. The absorption peak for the AuNP was at 520 nm. Upon binding to the BSA-biotin-creatinine conjugate, the new maximum was at 526 nm.

Size characterization was done using NTA. NaCl-$Na_3PO_4$ buffer was used to do the measurement but it showed aggregation as a peak around 600 nm was observed. Using MilliQ water for the NTA of AuNP, and Tris buffer for the NTA of AuNP-BSA-biotin-creatinine conjugate, showed no aggregation in the samples. The aggregation was mainly due to the buffer that contained Na+ ions and Cl- ions that could bind to the AuNPs, forming larger complex molecules. Such ions were not present in the other buffers used.

NTA for AUNP showed a sharp peak at 80 nm, confirming the size of the gold nanoparticle. NTA for AUNP-BSA-creatinine conjugate showed an additional peak at 97 nm, which confirmed the size of the AUNP-BSA conjugate molecule.

*Capture ELISA*

An ELISA was carried out to ensure that the binding of creatinine-anti-creatinine antibody and biotin-avidin follows the concentration proportionality. Decreasing the concentration of the BSA resulted in a decrease in absorbance at 450 nm, as shown in Figure 27. For concentrations ranging from 25 μg/ml decreasing to 0.625 μg/ml, significant changes in colour intensity and absorbance were observed

Concentrations of BSA above 50 μg/ml were also tested. 150 μg/ml was used and diluted to 125, 100, 75 and 50 μg/ml. No significant change in absorption was seen by decreasing the concentration in this region. This was because at concentrations above 25 μg/ml, all the antibodies were saturated with the BSA. As a result, at concentrations between 50 to 150 μg/ml, the same amount of chromogenic TMB product was produced, resulting in no significant change in the absorbance graph.

*Competitive ELISA*

This was a repeat of the capture ELISA but in this case the BSA conjugate was kept constant and different concentrations of free creatinine were added. A semi log graph was plotted to produce a dose response curve (Figure 28). As the concentration of free creatinine was increased, this was bound to antibody, and fewer antibody sites were available for binding to the BSA-biotin-creatinine conjugate, such that the absorbance decreased.

*Lateral Flow Assay*

Different concentrations of creatinine were used in each liquid sample to see its effect on signal intensity of test line 1 and 2. Creatinine concentrations ranging from 0 ng/ml to 100 μg /ml were tested. The resulting LFTs strips are shown in Figure 19. Figures 19 and 20 show that the modified LFT design performed as expected for the detection of creatinine. In particular:

- 0 ng/ml and 1 mg/ml creatinine produced a strong signal at the antibody test line (test line 1), and produced no signal at

the avidin test line (test line 2). This suggests that no AuNP-BSA-biotin-creatinine conjugate was displaced from test line 1 and all conjugates were captured by the antibodies.

- 5 ng/ml and 10 ng/ml creatinine slightly reduced the signal of test line 1, and the signal of test line 2 was slightly increased, suggesting that some AuNP-BSA-biotin-creatinine conjugates were displaced and captured by test line 2.
- The signal intensity of test line 1 was significantly reduced, and the signal intensity of test line 2 was significantly increased, as the concentration of creatinine was increased to 100 ng/ml and 1 μg/ml. This suggests that competition between creatinine and the AuNP-BSA-biotin-creatinine conjugate was high, resulting in more AuNP-BSA-biotin-creatinine conjugates being displaced from test line 1 and being captured by test line 2.
- The signal intensity of test line 1 was negligible at 10 μg/ml creatinine and no signal was observed at 100 μg/ml creatinine. The signal intensity of test line 2 was high for 10μg/ml and 100μg/ml creatinine. This suggests that no AuNP-BSA-biotin-creatinine conjugate bound to test line 1, and that the conjugate instead bound to test line 2.

Figure 20 shows that the signal of test line 2 increased in intensity as the concentration of creatinine increased, showing a proportional relationship. The signal of test line 1 decreased in intensity as the concentration of creatinine was increased, showing an inverse proportional relationship.

## Claims

1. A lateral flow test device comprising a solid support structure comprising a sample-receiving region, a conjugate pad, a first test region and a second test region, wherein the solid support structure is configured to permit liquid to flow sequentially from the sample-receiving region via the conjugate pad to the first test region and then the second test region, wherein

   i) the conjugate pad comprises a mobilisable conjugated analyte comprising one or more analyte molecules conjugated to a detectable label;
   ii)

      a) the first test region comprises an immobilised analyte-binding molecule defining a first binding site,
      b) the conjugate pad comprises a mobilisable analyte-binding molecule and the first test region comprises an immobilised capture molecule for immobilising the analyte-binding molecule, wherein the immobilised capture molecule defines a first binding site, or
      c) the conjugate pad comprises a mobilisable analyte-binding molecule and the first test region comprises an immobilised analyte-binding molecule defining a first binding site, and

   iii) the second test region comprises an immobilised conjugated-analyte-binding molecule which binds the conjugated analyte and does not bind unconjugated analyte molecules, and which defines a second binding site,

   and wherein the number of molecules of the conjugated analyte is less than or equal to the number of second binding sites.

2. The lateral flow test device of claim 1, wherein the number of molecules of the conjugated analyte is less than the number of second binding sites on the second test region, and/or wherein the number of first binding sites is equal to the number of molecules of the conjugated analyte.

3. The lateral flow test device of claim 1 or 2, wherein:

   the conjugated analyte of i), the immobilised analyte-binding molecule of ii)a) and the conjugated-analyte-binding molecule of iii) are present in a ratio of 1:1:1; or
   the conjugated analyte of i), the mobilisable analyte-binding molecule of ii)b) the capture molecule of ii)b) and the conjugated-analyte-binding molecule of iii) are present in a ratio of 1:1:1:1.

4. The lateral flow test device of any one of the preceding claims, wherein each analyte molecule of the conjugated analyte is conjugated to the detectable label via a linking molecule, preferably wherein the linking molecule is biotin-BSA.

5. The lateral flow test device of claim 4, wherein the conjugated-analyte-binding molecule is specific for, and binds, the linking molecule, preferably wherein the conjugated-analyte-binding molecule is avidin, streptavidin or polystreptavidin.

**6.** A method for detecting the presence of an analyte molecule in a test sample, the method comprising:

i) providing the lateral flow test device of any one of claims 1-5,
ii) applying the test sample to the sample-receiving region of the lateral flow test device and allowing the test sample to migrate to the conjugate pad and to mix with the conjugated analyte, and optionally the mobilisable analyte-binding molecule,
iii) allowing the test sample and conjugated analyte, and optionally the mobilisable analyte-binding molecule, to migrate sequentially to the first test region and second test region and to contact the immobilised molecules in each of the first and second test regions,
iv) detecting a signal at each of the first and second test regions, wherein a change in signal intensity at both of the first and second test regions indicates the presence of the analyte molecule in the test sample.

**7.** The method of claim 6, wherein the signal is an optical signal.

**8.** The method of claim 6 or claim 7 further comprising:
v) quantifying a concentration of analyte molecule in the test sample based on a ratio of the signal intensity at the first test region to the signal intensity at the second test region.

**9.** The method of any of claims 6 to 8, wherein, in step iv), a decrease of the signal intensity at the first test region and an increase of the signal intensity at the second test region indicates the presence of the analyte molecule in the test sample.

**10.** The method of any one of claims 6-9, wherein the signal intensities are measured as a percentage of a maximum signal intensity and wherein step iv) comprises measuring the signal intensity at one of the first and second test regions and predicting the signal intensity at the other of the first and second test regions based on the sum of the signal intensity of the first and second test regions being 100% of the maximum signal intensity, preferably wherein step iv) comprises:

a) measuring the intensity of the signal at the first test region and predicting the intensity of the signal at the second test region based on the sum of the signal intensity of the first and second test regions being 100% of the maximum signal intensity, thereby calculating a first measured:predicted signal intensity ratio,
b) measuring the intensity of the signal at the second test region and predicting the intensity of the signal at the first test region based on the sum of the signal intensity of the first and second test regions being 100% of the maximum signal intensity, thereby calculating a second measured: predicted signal intensity ratio,
c) calculating an average signal intensity at each of the first and second test regions based on the first and second measured:predicted signal intensity ratios, to determine an average first:second test region signal intensity ratio,
d) calculating a ratio of the measured signal intensities of the first and second test regions,
e) comparing the average first:second test region signal intensity ratio to the ratio of the measured signal intensities, and
f) when the average first:second test region signal intensity ratio matches the ratio of the measured signal intensities, quantifying the concentration of analyte molecule in the test sample based on the average first:second test region signal intensity ratio,

wherein steps a) and b) can be carried out in either order.

**11.** The method of claim 10, wherein:

the intensity of the signal at the first test region and the intensity of the signal at the second test region are scaled using a calibration factor when measured; and
step iv) further comprises:
g) when the average first:second test region signal intensity ratio does not match the ratio of the measured signal intensities calculated in step d), adjusting the calibration factor and repeating steps a) to f) using the adjusted calibration factor, preferably wherein adjusting the calibration factor comprises:

determining whether the measured signal intensities of the first and second test regions add up to greater than the maximum signal intensity;

in response to the measured signal intensities of the first and second test regions adding up to greater than the maximum signal intensity, reducing the calibration factor; and
in response to the measured signal intensities of the first and second test regions not adding up to greater than the maximum signal intensity, increasing the calibration factor.

**12.** A method of diagnosing a disease or condition comprising carrying out the method as defined in any one of claims 6-11, wherein the test sample has been obtained from a human, animal or plant subject.

**13.** A computer-implemented method of detecting the presence of an analyte molecule in a test sample, the method comprising:

obtaining a signal intensity measurement at each of the first and second test regions of a lateral flow test device of any of claims 1-5 after the test sample has been applied to the lateral flow test device;
determining whether there has been a change in signal intensity at both of the first and second test regions;
in response to determining that there has been a change in signal intensity at both the first and second test regions, outputting an indication of the presence of the analyte molecule in the test sample.

**14.** The method of claim 13, wherein determining whether there has been a change in signal intensity at both of the first and second test regions comprises:

determining that there has been a change in one of the first and second test regions in response to the signal intensity for the one of the first and second test regions being less than a maximum signal intensity; and
determining that there has been a change in the other of the first and second test regions in response to the signal intensity for the other of the first and second test regions being greater than a minimum signal intensity.

**15.** A kit comprising a lateral flow test device as defined in any one of claims 1-5.

**Patentansprüche**

**1.** Lateral-Flow-Testvorrichtung umfassend eine feste Trägerstruktur umfassend einen probenaufnehmenden Bereich, ein Konjugat-Pad, einen ersten Testbereich und einen zweiten Testbereich, wobei die feste Trägerstruktur dazu konfiguriert ist, es Flüssigkeiten zu erlauben, nacheinander von dem probenaufnehmenden Bereich über das Konjugat-Pad zu dem ersten Testbereich und dem zweiten Testbereich zu fließen, wobei

i) das Konjugat-Pad einen mobilisierbaren konjugierten Analyten umfasst, der ein oder mehrere Analytenmoleküle umfasst, die an eine nachweisbare Markierung konjugiert sind;
ii)

a) der erste Testbereich ein immobilisiertes analytenbindendes Molekül umfasst, das eine erste Bindungsstelle definiert,
b) das Konjugat-Pad ein mobilisierbares analytenbindendes Molekül umfasst und der erste Testbereich ein immobilisiertes Fängermolekül zum Immobilisieren des analytenbindenden Moleküls umfasst, wobei das immobilisierte Fängermolekül eine erste Bindungsstelle definiert; oder
c) das Konjugat-Pad ein mobilisierbares analytenbindendes Molekül umfasst und der erste Testbereich ein immobilisiertes analytenbindendes Molekül umfasst, das eine erste Bindungsstelle definiert; und

iii) der zweite Testbereich ein immobilisiertes konjugierten-analytenbindendes Molekül umfasst, das sich an den konjugierten Analyten bindet und sich nicht an unkonjugierte Analytenmoleküle bindet, und das eine zweite Bindungsstelle definiert,

und wobei die Anzahl von Molekülen des konjugierten Analyten geringer oder gleich der Anzahl der zweiten Bindungsstellen ist.

**2.** Lateral-Flow-Testvorrichtung nach Anspruch 1, wobei die Anzahl der Moleküle des konjugierten Analyten geringer ist als die Anzahl von zweiten Bindungsstellen auf dem zweiten Testbereich, und/oder wobei die Anzahl von ersten Bindungsstellen gleich der Anzahl von Molekülen des konjugierten Analyten ist.

**3.** Lateral-Flow-Testvorrichtung nach Anspruch 1 oder 2, wobei:

der konjugierte Analyt nach i), das immobilisierte analytenbindende Molekül nach ii)a) und das konjugierten-analytenbindende Molekül nach iii) in einem Verhältnis von 1:1:1 vorliegen, oder
der konjugierte Analyt nach i), das mobilisierbare analytenbindende Molekül nach ii)b, das Fängermolekül nach ii)b) und das konjugierten-analytenbindende Molekül nach iii) in einem Verhältnis von 1:1:1:1 vorliegen.

**4.** Lateral-Flow-Testvorrichtung nach einem der vorhergehenden Ansprüche, wobei jedes Analytenmolekül des konjugierten Analyten über ein Verbindungsmolekül an die nachweisbare Markierung konjugiert ist, vorzugsweise wobei das Verbindungsmolekül Biotin-BSA ist.

**5.** Lateral-Flow-Testvorrichtung nach Anspruch 4, wobei das konjugierten-analytenbindende Molekül für das Verbindungsmolekül spezifisch ist und sich daran bindet, vorzugsweise wobei das konjugierten-analytenbindende Molekül Avidin, Streptavidin oder Polystreptavidin ist.

**6.** Verfahren zum Nachweisen des Vorliegens eines Analytenmoleküls in einer Testprobe, wobei das Verfahren Folgendes umfasst:

i) Bereitstellen der Lateral-Flow-Testvorrichtung nach einem der Ansprüche 1 bis 5,
ii) Aufbringen der Testprobe auf den probenaufnehmenden Bereich der Lateral-Flow-Testvorrichtung und der Testprobe gestatten sich zu dem Konjugat-Pad bewegen und sich mit dem konjugierten Analyten, und optional mit dem mobilisierbaren analytenbindenden Molekül, zu mischen
iii) der Testprobe und konjugiertem Analyten, und optional dem mobilisierbaren analytenbindenden Molekül, gestatten sich nacheinander zu dem ersten Testbereich und zweiten Testbereich zu bewegen und mit den immobilisierten Molekülen in sowohl dem ersten als auch dem zweiten Testbereich in Kontakt zu kommen,
iv) Nachweisen eines Signals in sowohl dem ersten als auch dem zweiten Testbereich, wobei eine Veränderung der Signalintensität in sowohl dem ersten als auch dem zweiten Testbereich das Vorliegen des Analytenmoleküls in der Testprobe anzeigt.

**7.** Verfahren nach Anspruch 6, wobei das Signal ein optisches Signal ist.

**8.** Verfahren nach Anspruch 6 oder Anspruch 7, ferner umfassend:
v) Quantifizieren einer Konzentration des Analytenmoleküls in der Testprobe basierend auf einem Verhältnis der Signalintensität in dem ersten Testbereich zu der Signalintensität in dem zweiten Testbereich.

**9.** Verfahren nach einem der Ansprüche 6 bis 8, wobei, in Schritt iv), ein Rückgang der Signalintensität in dem ersten Testbereich und ein Anstieg der Signalintensität in dem zweiten Testbereich das Vorliegen des Analytenmoleküls in der Testprobe anzeigt.

**10.** Verfahren nach einem der Ansprüche 6 bis 9, wobei die Signalintensitäten als Prozentsatz einer maximalen Signalintensität gemessen werden und wobei Schritt iv) Messen der Signalintensität in einem des ersten und zweiten Testbereichs und Vorhersagen der Signalintensität in dem anderen des ersten und zweiten Testbereichs basierend darauf, dass die Summe der Signalintensität des ersten und zweiten Testbereichs 100% der maximalen Signalintensität ist, umfasst, vorzugsweise wobei Schritt iv) Folgendes umfasst:

a) Messen der Intensität des Signals in dem ersten Testbereich und Vorhersagen der Intensität des Signals in dem zweiten Testbereich basierend darauf, dass die Summe der Signalintensität des ersten und zweiten Testbereichs 100% der maximalen Signalintensität ist, dadurch Berechnen eines ersten Intensitätsverhältnisses von gemessenem zu vorhergesagtem Signal,
b) Messen der Intensität des Signals in dem zweiten Testbereich und Vorhersagen der Intensität des Signals in dem ersten Testbereich basierend darauf, dass die Summe der Signalintensität des ersten und zweiten Testbereichs 100% der maximalen Signalintensität ist, dadurch Berechnen eines zweiten Intensitätsverhältnisses von gemessenem zu vorhergesagtem Signal,
c) Berechnen einer durchschnittlichen Signalintensität in sowohl dem ersten als auch dem zweiten Testbereich basierend auf dem ersten und zweiten Intensitätsverhältnis von gemessenem zu vorhergesagtem Signal, um ein durchschnittliches Signalintensitätsverhältnis von erstem zu zweitem Testbereich zu bestimmen,
d) Berechnen eines Verhältnisses der gemessenen Signalintensitäten des ersten und zweiten Testbereichs,
e) Vergleichen des durchschnittlichen Signalintensitätsverhältnisses von erstem zu zweitem Testbereich mit dem

Verhältnis der gemessenen Signalintensitäten, und
f) wenn das durchschnittliche Signalintensitätsverhältnis von erstem zu zweitem Testbereich dem Verhältnis der gemessenen Signalintensitäten entspricht, Quantifizieren der Konzentration des Analytenmoleküls in der Testprobe basierend auf dem durchschnittlichen Signalintensitätsverhältnis von erstem zu zweitem Testbereich, wobei Schritte a) und b) in jeglicher Reihenfolge durchgeführt werden können.

**11.** Verfahren nach Anspruch 10, wobei:

die Intensität des Signals in dem ersten Testbereich und die Intensität des Signals in dem zweiten Testbereich bei der Messung unter Verwendung eines Kalibrierungsfaktors skaliert werden; und
Schritt iv) ferner Folgendes umfasst:
g) wenn das durchschnittliche Signalintensitätsverhältnis von erstem zu zweitem Testbereich dem in Schritt d) berechneten Verhältnis der gemessenen Signalintensitäten nicht entspricht, Anpassen des Kalibrierungsfaktors und Wiederholen der Schritte a) bis f) unter Verwendung des angepassten Kalibrierungsfaktors, vorzugsweise wobei das Anpassen des Kalibrierungsfaktors Folgendes umfasst:

Bestimmen, ob die gemessenen Signalintensitäten des ersten und zweiten Testbereichs addiert mehr ergeben als die maximale Signalintensität;
in Reaktion darauf, dass die gemessenen Signalintensitäten des ersten und zweiten Testbereichs addiert mehr ergeben als die maximale Signalintensität, Reduzieren des Kalibrierungsfaktors; und
in Reaktion, darauf, dass die gemessenen Signalintensitäten des ersten und zweiten Testbereichs addiert nicht mehr ergeben als die maximale Signalintensität, Erhöhen des Kalibrierungsfaktors.

**12.** Verfahren zum Diagnostizieren einer Krankheit oder eines Leidens umfassend Durchführen des Verfahrens wie in einem der Ansprüche 6 bis 11 definiert, wobei die Testprobe von einem menschlichen, tierischen oder pflanzlichen Testobjekt stammt.

**13.** Computerimplementiertes Verfahren zum Nachweisen des Vorliegens eines Analytenmoleküls in einer Testprobe, wobei das Verfahren Folgendes umfasst:

Erhalten einer Signalintensitätsmessung in sowohl dem ersten als auch dem zweiten Testbereich einer Lateral-Flow-Testvorrichtung nach einem der Ansprüche 1 bis 5, nachdem die Testprobe auf die Lateral-Flow-Testvorrichtung aufgebracht wurde;
Bestimmen, ob es zu einer Änderung der Signalintensität in sowohl dem ersten als auch dem zweiten Testbereich gekommen ist;
in Reaktion auf das Bestimmen, dass es zu einer Änderung der Signalintensität in sowohl dem ersten als auch dem zweiten Testbereich gekommen ist, Ausgeben eines Hinweises auf das Vorliegen des Analytenmoleküls in der Testprobe.

**14.** Verfahren nach Anspruch 13, wobei das Bestimmen, ob es zu einer Änderung der Signalintensität in sowohl dem ersten als auch dem zweiten Testbereich gekommen ist, Folgendes umfasst:

Bestimmen, dass es zu einer Änderung in einem von dem ersten und zweiten Testbereich gekommen ist, in Reaktion darauf, dass die Signalintensität für den einen von dem ersten und zweiten Testbereich geringer ist als die maximale Signalintensität; und
Bestimmen, dass es zu einer Änderung in dem anderen von dem ersten und zweiten Testbereich gekommen ist, in Reaktion darauf, dass die Signalintensität für den anderen von dem ersten und zweiten Testbereich größer ist als die minimale Signalintensität.

**15.** Kit umfassend eine Lateral-Flow-Testvorrichtung wie in einem der Ansprüche 1-5 definiert.

## Revendications

**1.** Dispositif de test à flux latéral comprenant une structure de support solide comprenant une zone de réception d’échantillon, un tampon de conjugué, une première zone de test et une deuxième zone de test, ladite structure de support solide étant configurée pour permettre à des liquides de s’écouler successivement de la zone de réception d’échantillon vers la première zone de test, puis vers la deuxième zone de test en passant par le tampon de conjugué ;

dans lequel

i) le tampon de conjugué comprend un analyte conjugué mobilisable comprenant une ou plusieurs molécules d'analyte conjuguées à un marqueur détectable ;
ii)

a) la première zone de test comprend une molécule de liaison à l'analyte immobilisée définissant un premier site de liaison,
b) le tampon de conjugué comprend une molécule de liaison à l'analyte mobilisable et la première zone de test comprend une molécule de capture immobilisée destinée à immobiliser la molécule de liaison à l'analyte, ladite molécule de capture immobilisée définissant un premier site de liaison, ou
c) le tampon de conjugué comprend une molécule de liaison à l'analyte mobilisable et la première zone de test comprend une molécule de liaison à l'analyte immobilisée définissant un premier site de liaison ; et

iii) la deuxième zone de test comprend une molécule de liaison à l'analyte conjugué immobilisée qui se lie à l'analyte conjugué et ne se lie pas aux molécules d'analyte non conjugué, et qui définit un deuxième site de liaison,

et dans lequel le nombre de molécules de l'analyte conjugué est inférieur ou égal au nombre de deuxièmes sites de liaison.

**2.** Dispositif de test à flux latéral selon la revendication 1, dans lequel le nombre de molécules de l'analyte conjugué est inférieur au nombre de deuxièmes sites de liaison présents sur la deuxième zone de test, et/ou dans lequel le nombre de premiers sites de liaison est égal au nombre de molécules de l'analyte conjugué.

**3.** Dispositif de test à flux latéral selon la revendication 1 ou 2, dans lequel :

l'analyte conjugué de i), la molécule de liaison à l'analyte immobilisée de ii)a) et la molécule de liaison à l'analyte conjugué de iii) sont présentes dans un rapport de 1:1:1, ou
l'analyte conjugué de i), la molécule de liaison à l'analyte mobilisable de ii)b), la molécule de capture de ii)b) et la molécule de liaison à l'analyte conjugué de iii) sont présentes dans un rapport de 1:1:1:1.

**4.** Dispositif de test à flux latéral selon l'une quelconque des revendications précédentes, dans lequel chaque molécule d'analyte de l'analyte conjugué est conjuguée au marqueur détectable par l'intermédiaire d'une molécule de couplage, ladite molécule de couplage étant de préférence la biotine-BSA.

**5.** Dispositif de test à flux latéral selon la revendication 4, dans lequel la molécule de liaison à l'analyte conjugué est spécifique à la molécule de couplage et se lie à celle-ci, ladite molécule de liaison à l'analyte conjugué étant de préférence l'avidine, la streptavidine ou la polystreptavidine.

**6.** Procédé de détection de la présence d'une molécule d'analyte dans un échantillon d'essai, le procédé comprenant les étapes consistant à :

i) fournir le dispositif de test à flux latéral selon l'une quelconque des revendications 1 à 5,
ii) appliquer l'échantillon d'essai sur la zone de réception d'échantillon du dispositif d'essai à flux latéral et laisser l'échantillon d'essai migrer vers le tampon de conjugué et se mélanger avec l'analyte conjugué et facultativement avec la molécule de liaison à l'analyte mobilisable,
iii) laisser l'échantillon d'essai et l'analyte conjugué, et facultativement la molécule de liaison à l'analyte mobilisable, migrer successivement vers la première zone de test et la deuxième zone de test et entrer en contact avec les molécules immobilisées dans chacune des première et deuxième zones de test,
iv) détecter un signal dans chacune des première et deuxième zones de test, un changement d'intensité du signal dans les première et deuxième zones de test indiquant la présence de la molécule d'analyte dans l'échantillon d'essai.

**7.** Procédé selon la revendication 6, dans lequel le signal est un signal optique.

**8.** Procédé selon la revendication 6 ou la revendication 7, comprenant en outre l'étape consistant à :
v) quantifier une concentration en molécule d'analyte dans l'échantillon d'essai compte tenu du rapport entre

l'intensité du signal dans la première zone de test et l'intensité du signal dans la deuxième zone de test.

**9.** Procédé selon l'une quelconque des revendications 6 à 8, dans lequel, à l'étape iv), une diminution de l'intensité du signal dans la première zone de test et une augmentation de l'intensité du signal dans la deuxième zone de test indiquent la présence de la molécule d'analyte dans l'échantillon d'essai.

**10.** Procédé selon l'une quelconque des revendications 6 à 9, dans lequel les intensités de signal sont mesurées en pourcentage d'une intensité de signal maximale, et dans lequel l'étape iv) comprend la mesure de l'intensité du signal dans l'une des première et deuxième zones de test et la prédiction de l'intensité du signal dans l'autre des première et deuxième zones de test sur la base du fait que la somme de l'intensité du signal des première et deuxième zones de test est égale à 100 % de l'intensité maximale ; ladite étape iv) comprenant de préférence :

a) la mesure de l'intensité du signal dans la première zone de test et la prédiction de l'intensité du signal dans la deuxième zone de test, sur la base du fait que la somme de l'intensité du signal des première et deuxième zones de test est égale à 100 % de l'intensité maximale du signal, calculant ainsi un premier rapport d'intensité de signal mesurée:intensité de signal prédite,
b) la mesure de l'intensité du signal dans la deuxième zone de test et la prédiction de l'intensité du signal dans la première zone de test, sur la base du fait que la somme de l'intensité du signal des première et deuxième zones de test est de 100 % de l'intensité maximale du signal, calculant ainsi un deuxième rapport d'intensité de signal mesurée:intensité de signal prédite,
c) le calcul d'une intensité de signal moyenne dans chacune des première et deuxième zones de test, compte tenu des premier et deuxième rapports d'intensité de signal mesuré:intensité de signal prédite, pour déterminer un rapport d'intensité de signal moyenne de première zone de test:intensité de signal moyenne de deuxième zone de test,
d) le calcul d'un rapport des intensités de signal mesurées des première et deuxième zones de test,
e) la comparaison du rapport d'intensité de signal moyenne de première zone de test:intensité de signal moyenne de deuxième zone de test au rapport des intensités de signal mesurées, et
f) lorsque le rapport d'intensité de signal moyenne de première zone de test:intensité de signal moyenne de deuxième zone de test correspond au rapport des intensités de signal mesurées, la quantification de la concentration en molécule d'analyte dans l'échantillon d'essai compte tenu du rapport d'intensité de signal moyenne de première zone de test:intensité de signal moyenne de deuxième zone de test,

lesdites étapes a) et b) pouvant être réalisées dans un ordre différent.

**11.** Procédé selon la revendication 10, dans lequel :

l'intensité du signal dans la première zone de test et l'intensité du signal dans la deuxième zone de test sont mises à l'échelle à l'aide d'un facteur d'étalonnage lors de la mesure ; et
l'étape iv) comprend en outre :
g) lorsque le rapport d'intensité de signal moyenne de première zone de test:intensité de signal moyenne de deuxième zone de test ne correspond pas au rapport des intensités de signal mesurées calculé à l'étape d), l'ajustement du facteur d'étalonnage et la répétition des étapes a) à f) au moyen du facteur d'étalonnage ajusté, ledit ajustement du facteur d'étalonnage comprenant de préférence les étapes consistant à :

déterminer si les intensités de signal mesurées des première et deuxième zones de test additionnées forment un total dépassant l'intensité de signal maximale,
si les intensités de signal mesurées des première et deuxième zones de test additionnées forment un total dépassant l'intensité de signal maximale, réduire le facteur d'étalonnage, et
si les intensités de signal mesurées des première et deuxième zones de test additionnées ne forment pas un total dépassant l'intensité de signal maximale, augmenter le facteur d'étalonnage.

**12.** Procédé de diagnostic d'une maladie ou d'une affection, comprenant la mise en œuvre du procédé défini dans l'une quelconque des revendications 6 à 11, ledit échantillon d'essai étant issu d'un sujet humain, animal ou végétal.

**13.** Procédé mis en œuvre par ordinateur pour détecter la présence d'une molécule d'analyte dans un échantillon d'essai, le procédé comprenant les étapes consistant à :

obtenir une mesure d'intensité de signal dans chacune des première et deuxième zones de test d'un dispositif de

test à flux latéral selon l'une quelconque des revendications 1 à 5 après que l'échantillon d'essai a été appliqué au dispositif de test à flux latéral ;
déterminer s'il y a eu un changement d'intensité du signal dans les première et deuxième zones de test ;
s'il est déterminé un changement d'intensité du signal aussi bien dans la première que dans la deuxième zone de test, émettre une indication de la présence de la molécule d'analyte dans l'échantillon d'essai.

**14.** Procédé selon la revendication 13, dans lequel la détermination d'un changement d'intensité du signal dans les première et deuxième zones de test comprend :

la détermination qu'il y a eu un changement dans l'une des première et deuxième zones de test si l'intensité du signal propre à l'une des première et deuxième zones de test est inférieure à une intensité de signal maximale, et la détermination qu'il y a eu un changement dans l'autre des première et deuxième zones de test si l'intensité du signal propre à l'autre des première et deuxième zones de test est supérieure à une intensité de signal minimale.

**15.** Kit comprenant un dispositif de test à flux latéral tel que défini dans l'une quelconque des revendications 1 à 5.

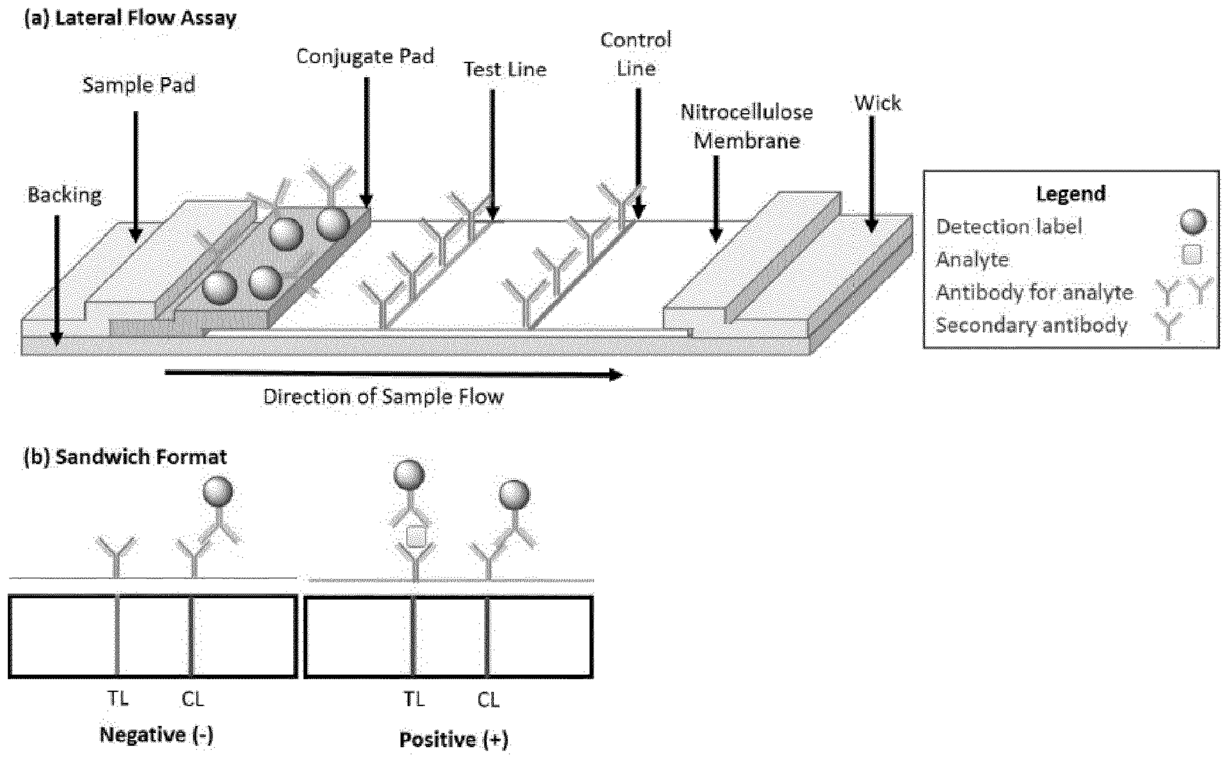
(a) Lateral Flow Assay
Conjugate Pad
Control Line
Test Line
Sample Pad
Nitrocellulose Membrane
Wick
Backing
Legend
Detection label
Analyte
Antibody for analyte
Secondary antibody
Direction of Sample Flow
(b) Sandwich Format
TL
CL
Negative (-)
TL
CL
Positive (+)

Fig. 1

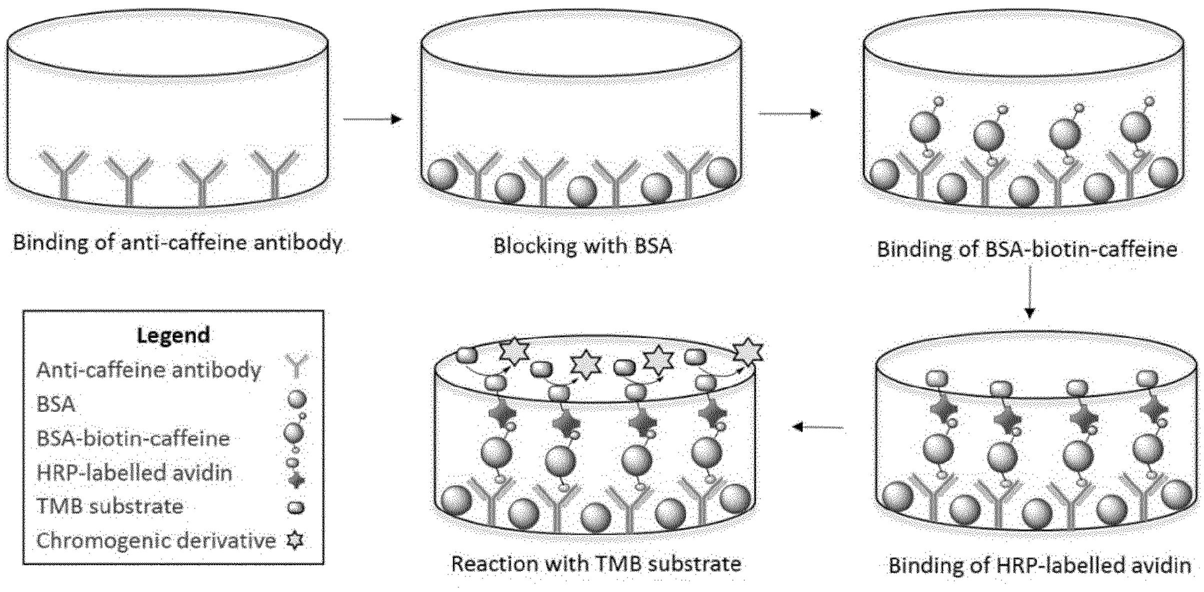
Binding of anti-caffeine antibody
Blocking with BSA
Binding of BSA-biotin-caffeine
Legend
Anti-caffeine antibody
BSA
BSA-biotin-caffeine
HRP-labelled avidin
TMB substrate
Chromogenic derivative
Reaction with TMB substrate
Binding of HRP-labelled avidin

Fig. 2

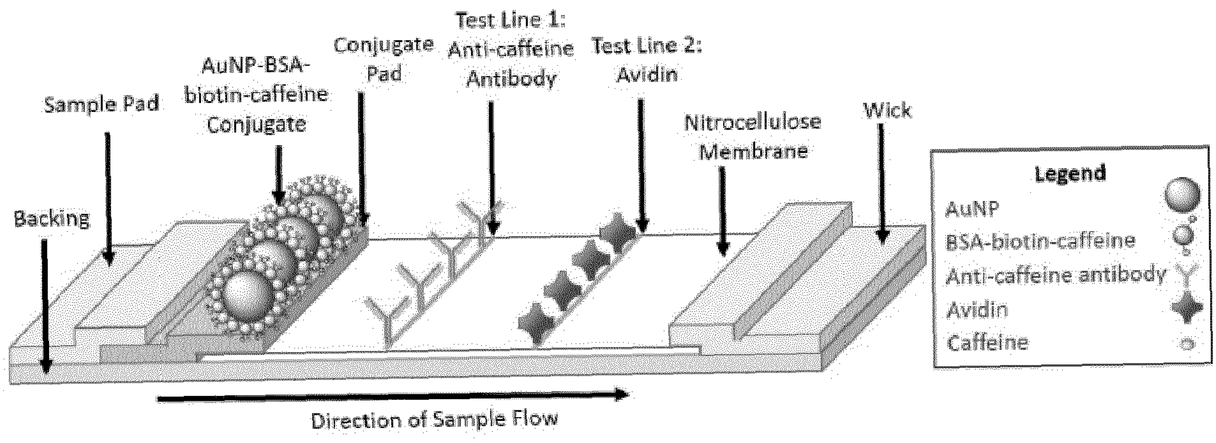
Test Line 1: Anti-caffeine Antibody
Test Line 2: Avidin
Conjugate Pad
AuNP-BSA-biotin-caffeine Conjugate
Sample Pad
Backing
Nitrocellulose Membrane
Wick
Legend
AuNP
BSA-biotin-caffeine
Anti-caffeine antibody
Avidin
Caffeine
Direction of Sample Flow

Fig. 3

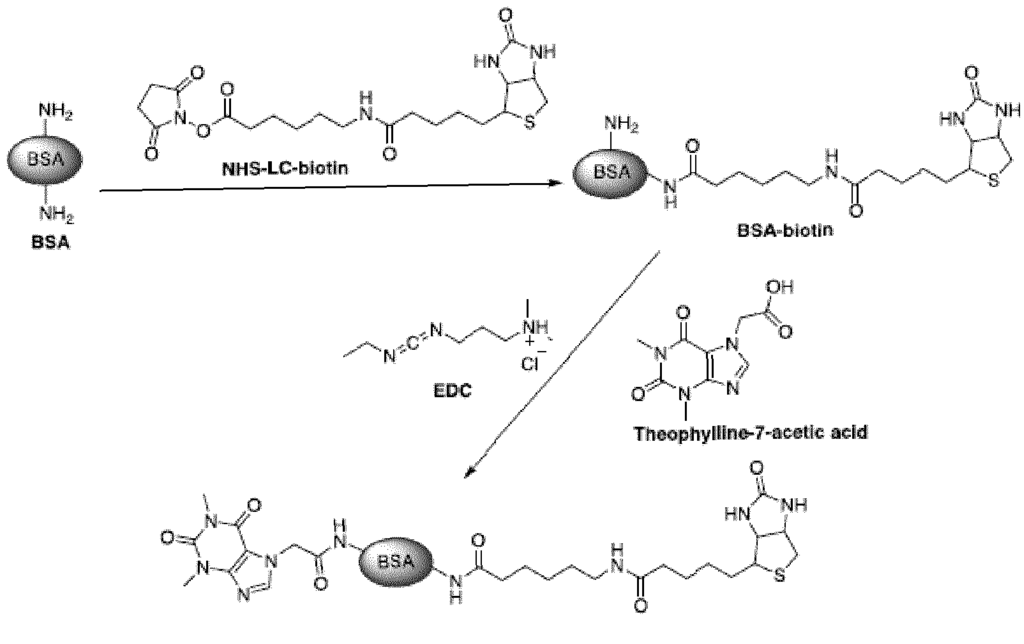
NHS-LC-biotin
BSA
BSA-biotin
EDC
Theophylline-7-acetic acid

Fig. 4

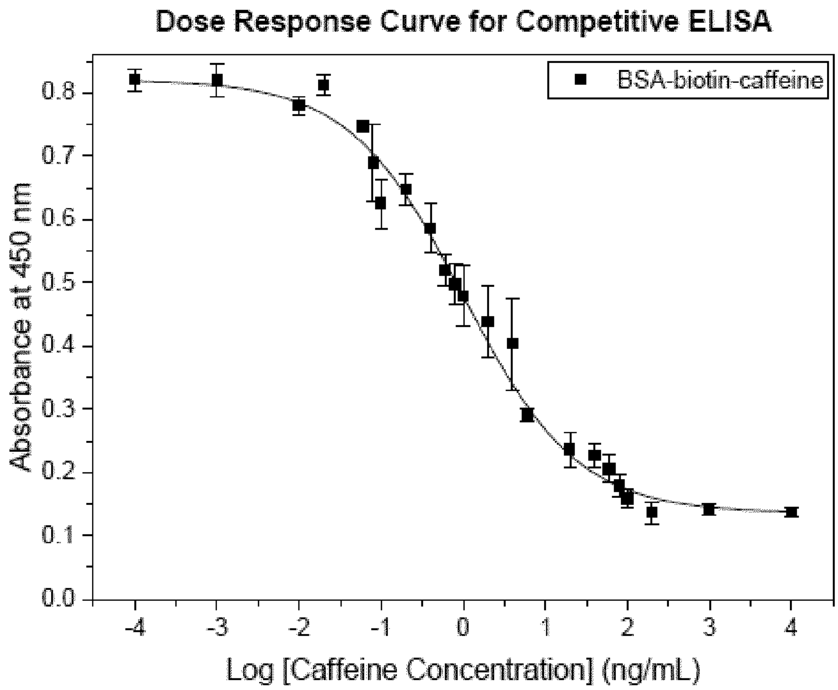
Dose Response Curve for Competitive ELISA
BSA-biotin-caffeine
Absorbance at 450 nm
0.0
0.1
0.2
0.3
0.4
0.5
0.6
0.7
0.8
-4
-3
-2
-1
0
1
2
3
4
Log [Caffeine Concentration] (ng/mL)

Fig. 5

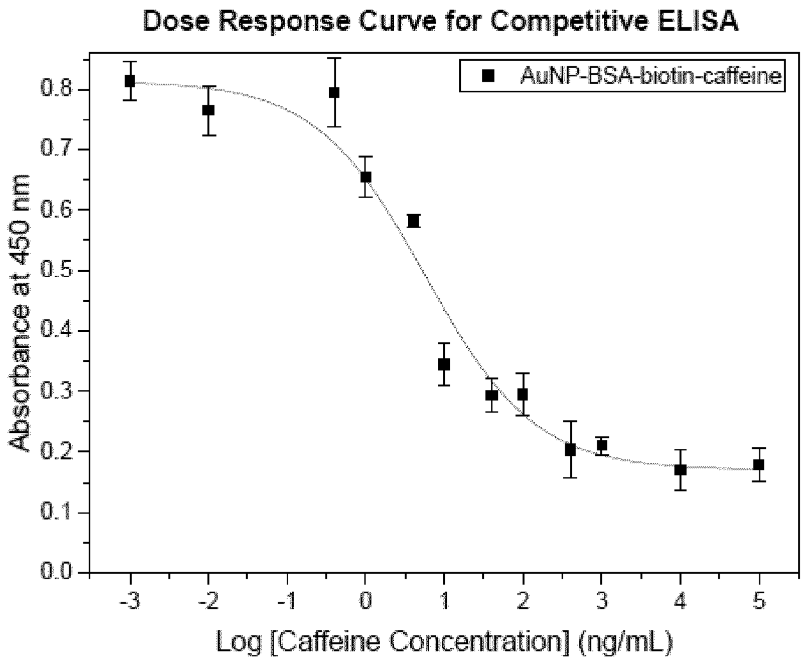
Dose Response Curve for Competitive ELISA
AuNP-BSA-biotin-caffeine
Absorbance at 450 nm
0.0
0.1
0.2
0.3
0.4
0.5
0.6
0.7
0.8
-3
-2
-1
0
1
2
3
4
5
Log [Caffeine Concentration] (ng/mL)

Fig. 6

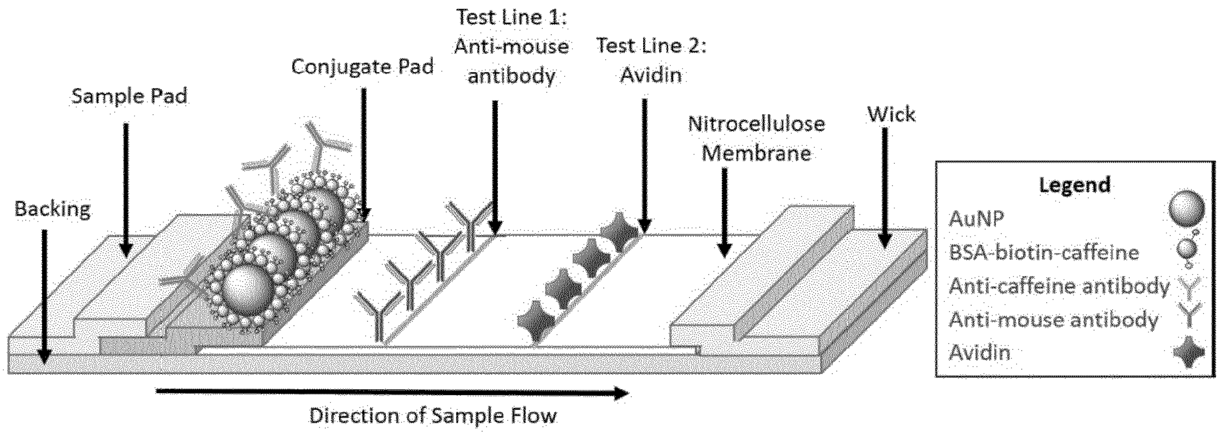
Test Line 1:
Anti-mouse antibody
Test Line 2:
Avidin
Conjugate Pad
Sample Pad
Nitrocellulose Membrane
Wick
Backing
Legend
AuNP
BSA-biotin-caffeine
Anti-caffeine antibody
Anti-mouse antibody
Avidin
Direction of Sample Flow

Fig. 7

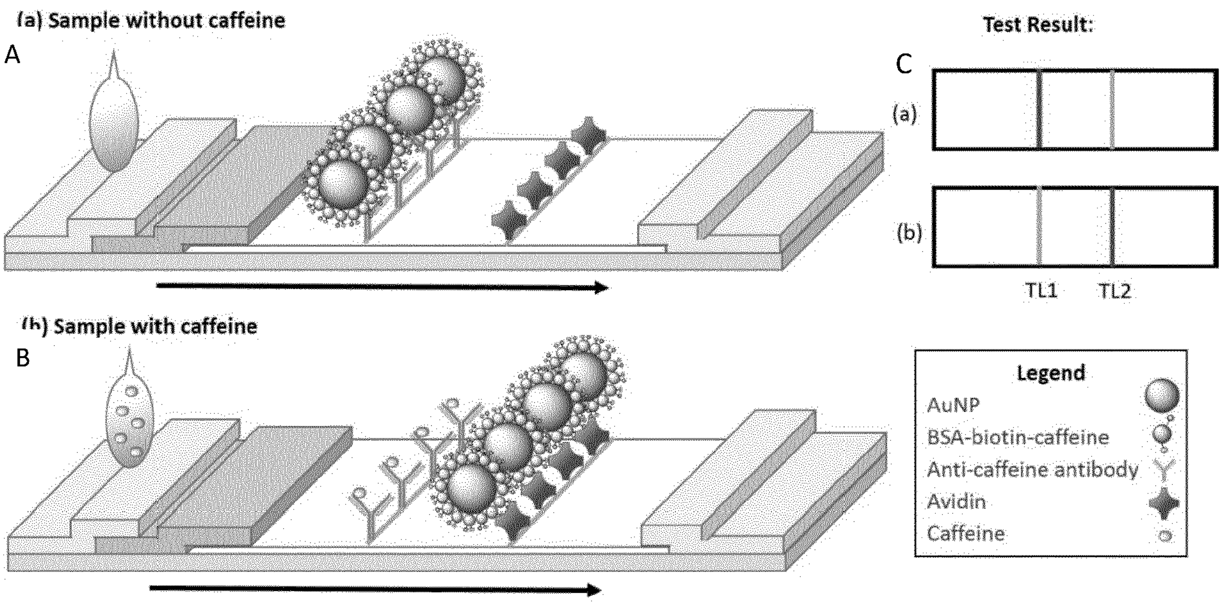
(a) Sample without caffeine
A
Test Result:
C
(a)
(b)
TL1
TL2
(b) Sample with caffeine
B
Legend
AuNP
BSA-biotin-caffeine
Anti-caffeine antibody
Avidin
Caffeine

Fig. 8

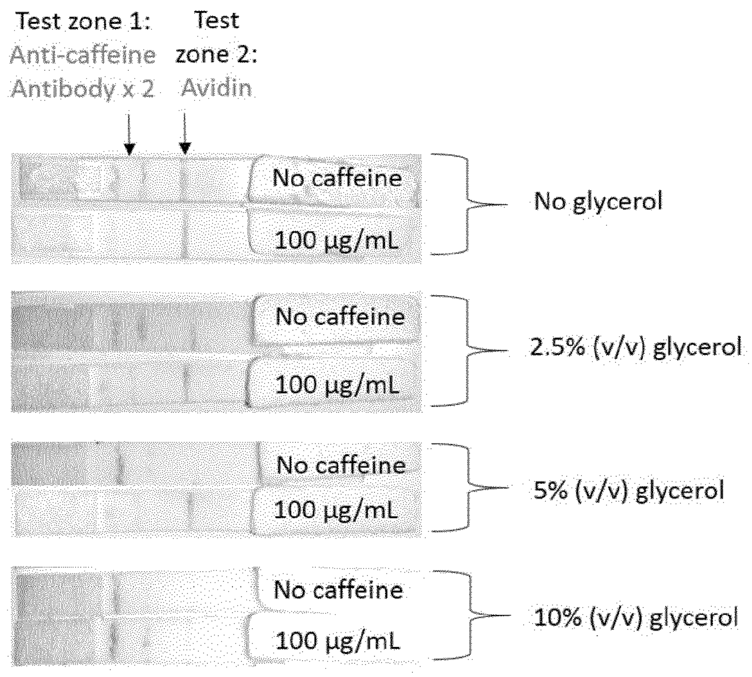
Test zone 1:
Anti-caffeine
Antibody x 2
Test
zone 2:
Avidin
No caffeine
100 µg/mL
No glycerol
No caffeine
100 µg/mL
2.5% (v/v) glycerol
No caffeine
100 µg/mL
5% (v/v) glycerol
No caffeine
100 µg/mL
10% (v/v) glycerol

Fig. 9

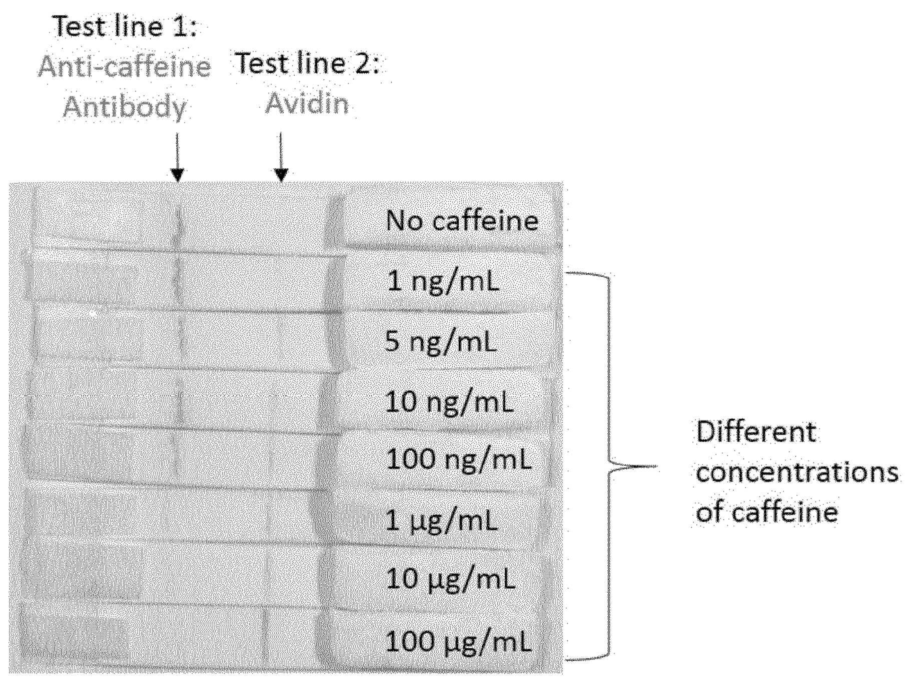
Test line 1:
Anti-caffeine
Antibody
Test line 2:
Avidin
No caffeine
1 ng/mL
5 ng/mL
10 ng/mL
100 ng/mL
1 µg/mL
10 µg/mL
100 µg/mL
Different
concentrations
of caffeine

Fig. 10

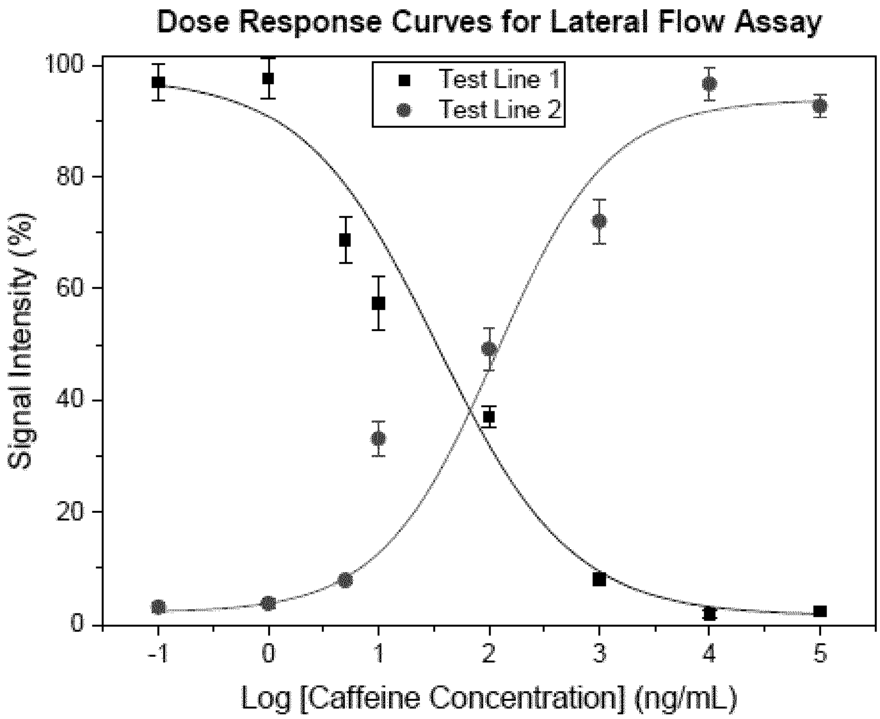
Dose Response Curves for Lateral Flow Assay
Test Line 1
Test Line 2
Signal Intensity (%)
0
20
40
60
80
100
-1
0
1
2
3
4
5
Log [Caffeine Concentration] (ng/mL)

Fig. 11

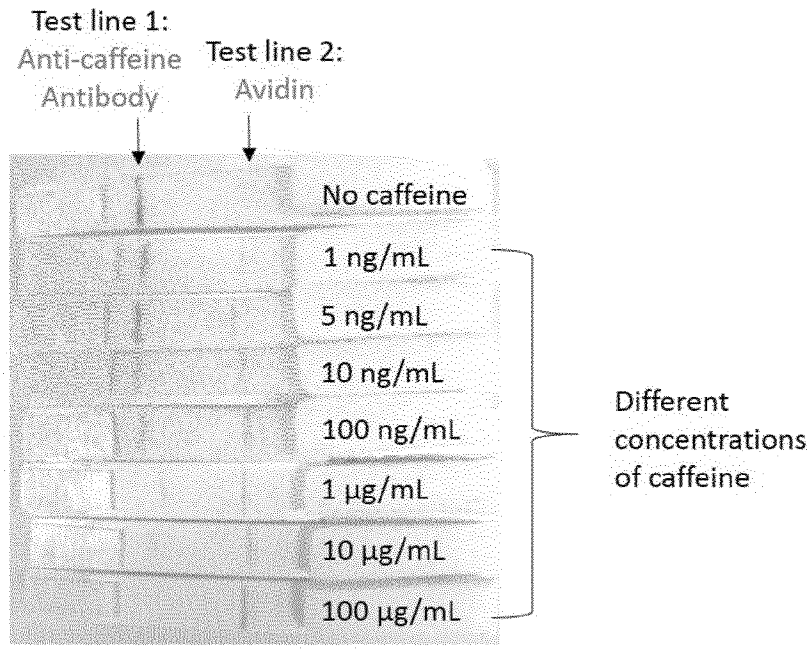
Test line 1:
Anti-caffeine
Antibody
Test line 2:
Avidin
No caffeine
1 ng/mL
5 ng/mL
10 ng/mL
100 ng/mL
1 µg/mL
10 µg/mL
100 µg/mL
Different
concentrations
of caffeine

Fig. 12

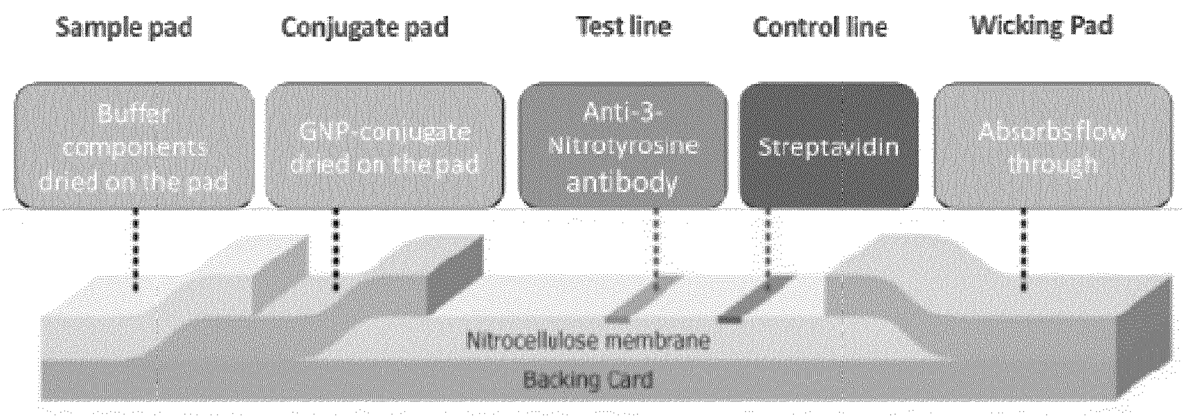
Sample pad
Conjugate pad
Test line
Control line
Wicking Pad
Buffer components dried on the pad
GNP-conjugate dried on the pad
Anti-3-Nitrotyrosine antibody
Streptavidin
Absorbs flow through
Nitrocellulose membrane
Backing Card

Fig. 13

| NHPA, ng/ml | 0 | 50 | 100 | 250 |
|---|---|---|---|---|

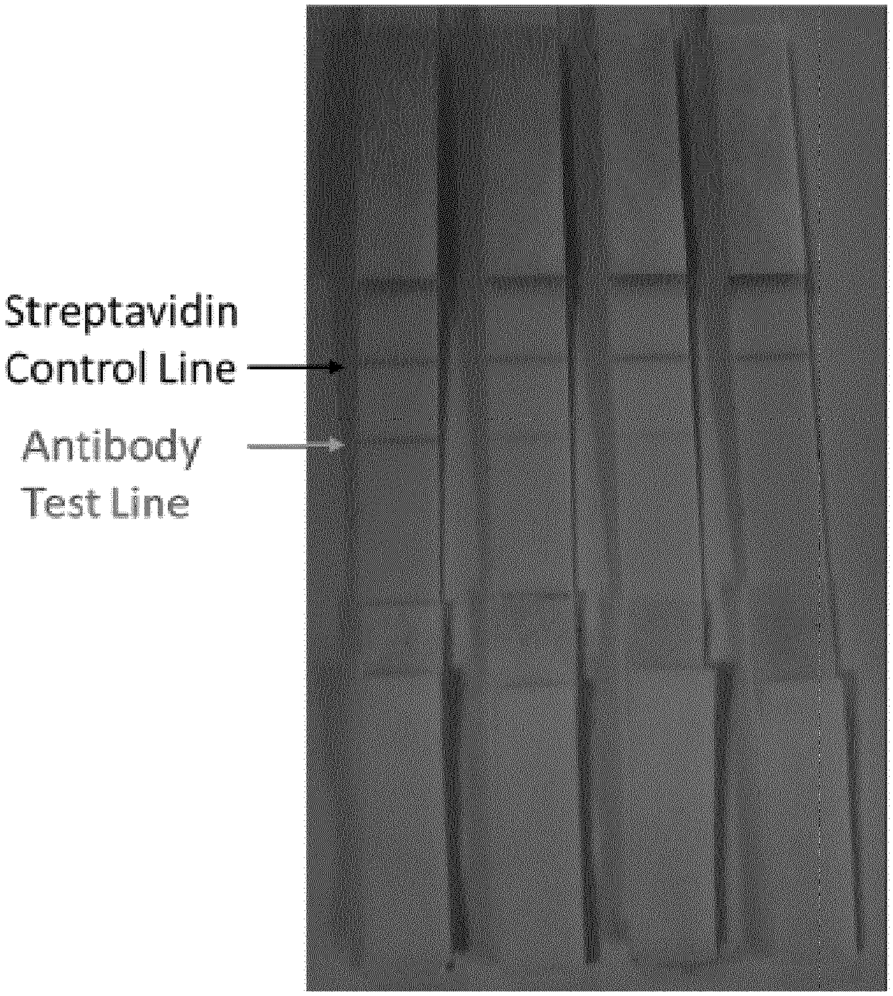


Fig. 14

A

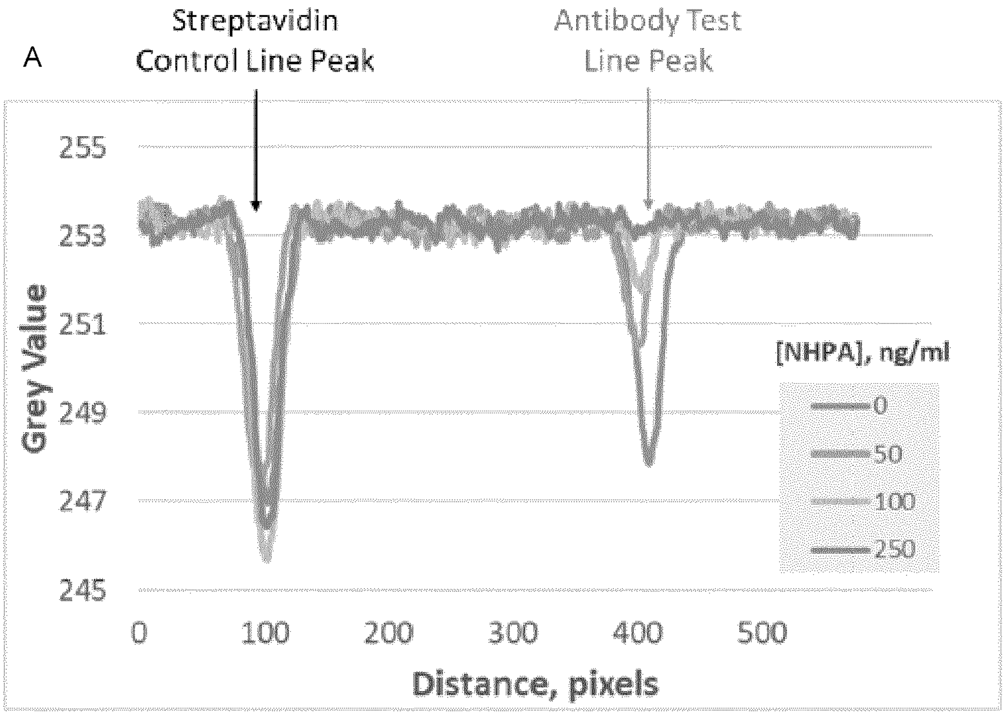
Streptavidin
Control Line Peak
Antibody Test
Line Peak
255
253
251
249
247
245
Grey Value
0
100
200
300
400
500
Distance, pixels
[NHPA], ng/ml
0
50
100
250

B

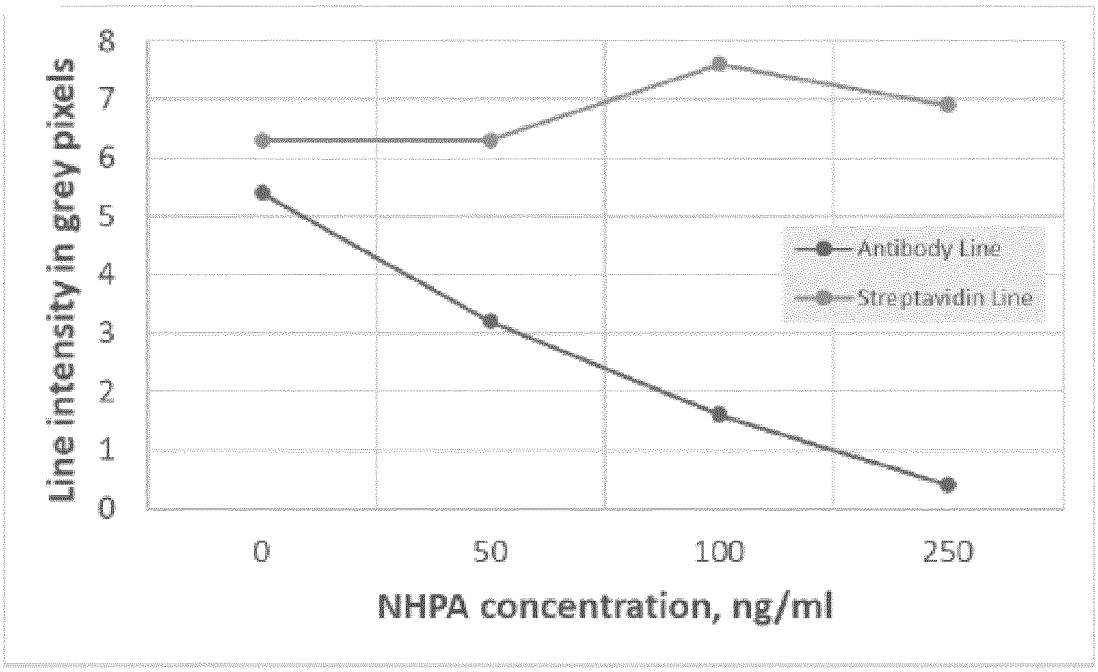
8
7
6
5
4
3
2
1
0
Line intensity in grey pixels
0
50
100
250
NHPA concentration, ng/ml
Antibody Line
Streptavidin Line

Fig. 15

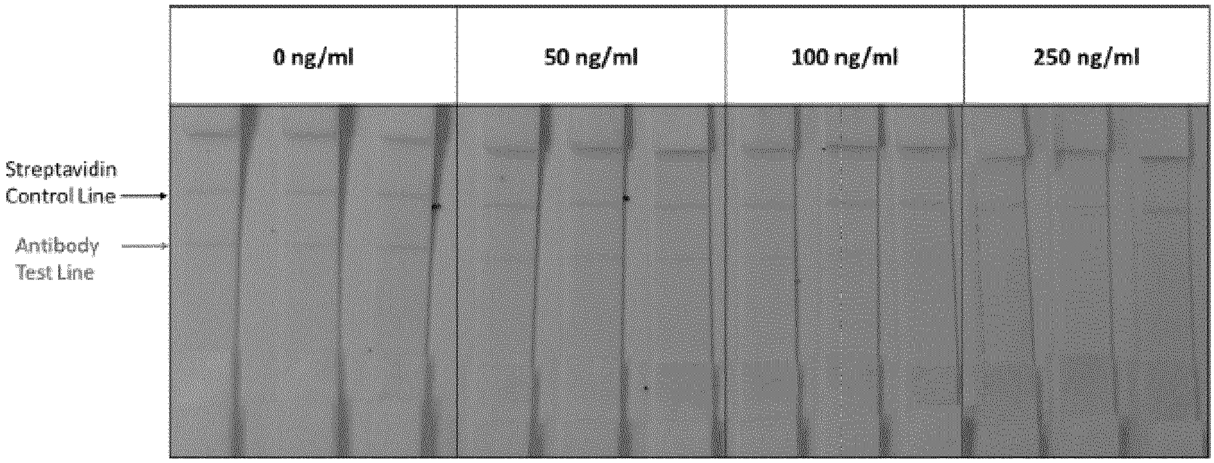
0 ng/ml
50 ng/ml
100 ng/ml
250 ng/ml
Streptavidin Control Line
Antibody Test Line

Fig. 16

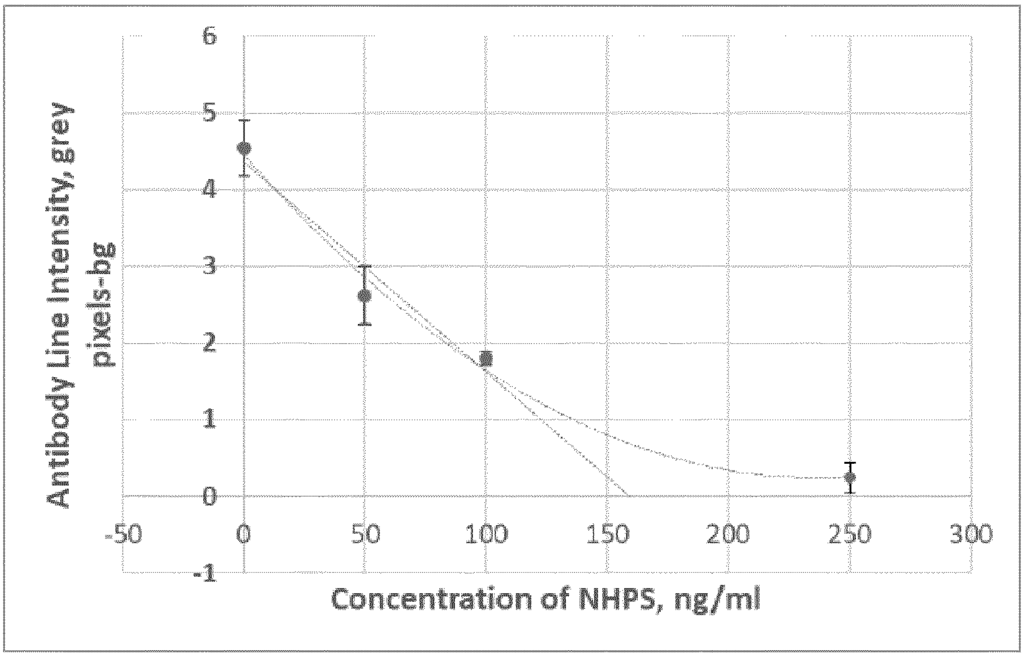
Antibody Line Intensity, grey pixels-bg
6
5
4
3
2
1
0
-1
-50
0
50
100
150
200
250
300
Concentration of NHPS, ng/ml

Fig. 17

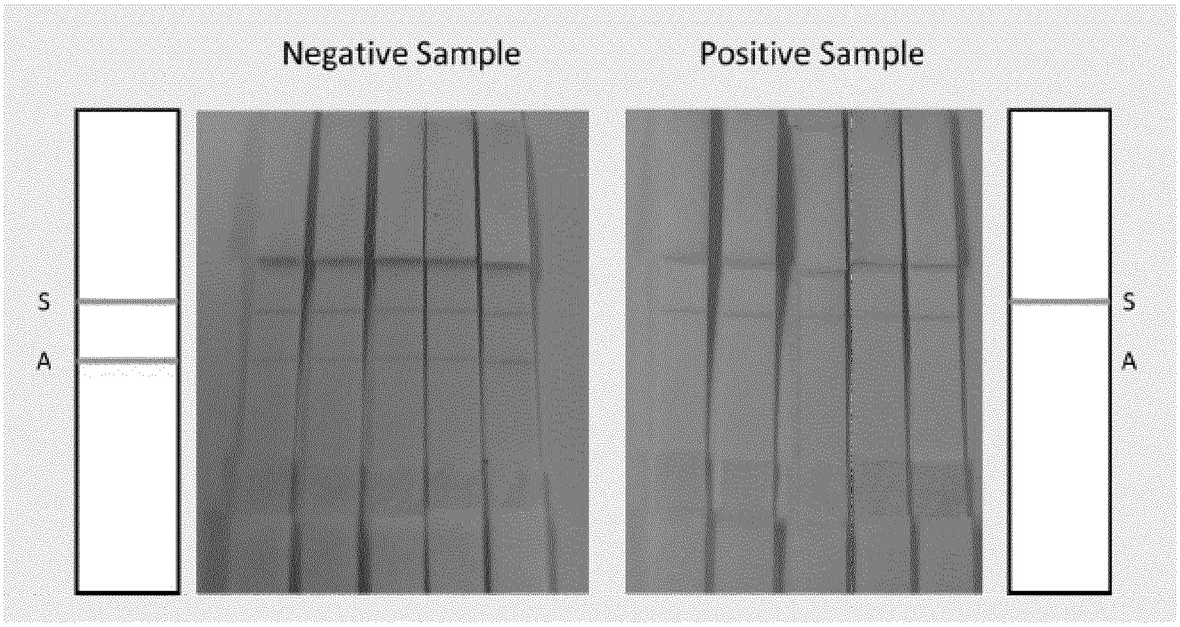
Negative Sample
Positive Sample
S
A
S
A

Fig. 18

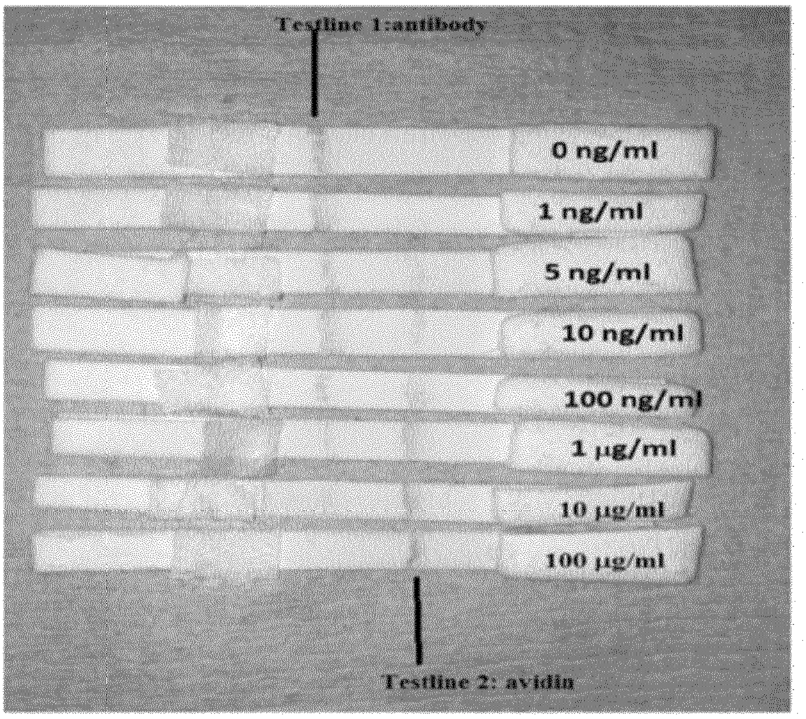
Testline 1:antibody
0 ng/ml
1 ng/ml
5 ng/ml
10 ng/ml
100 ng/ml
1 µg/ml
10 µg/ml
100 µg/ml
Testline 2: avidin

Fig. 19

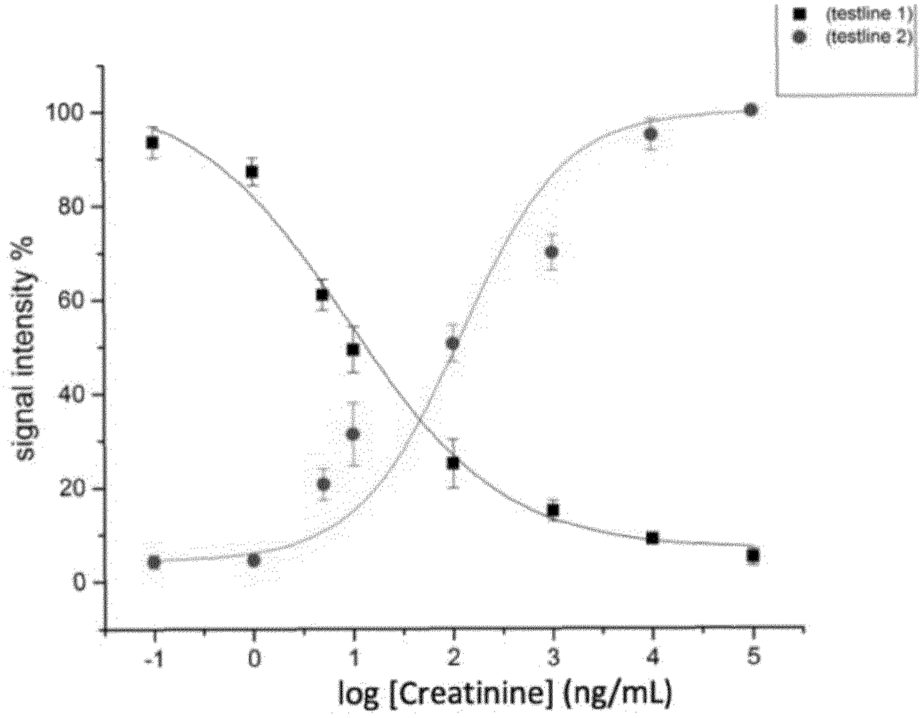
(testline 1)
(testline 2)
signal intensity %
100
80
60
40
20
0
-1
0
1
2
3
4
5
log [Creatinine] (ng/mL)

Fig. 20

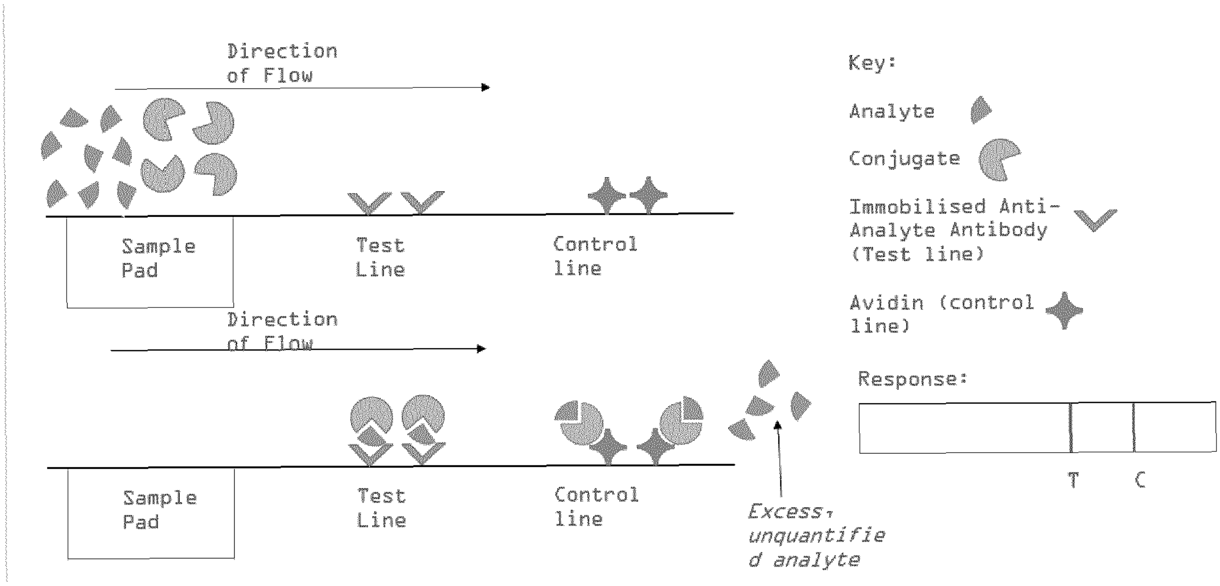
Direction of Flow
Sample Pad
Test Line
Control line
Direction of Flow
Sample Pad
Test Line
Control line
Excess, unquantified analyte
Key:
Analyte
Conjugate
Immobilised Anti-Analyte Antibody (Test line)
Avidin (control line)
Response:
T
C

Fig. 21

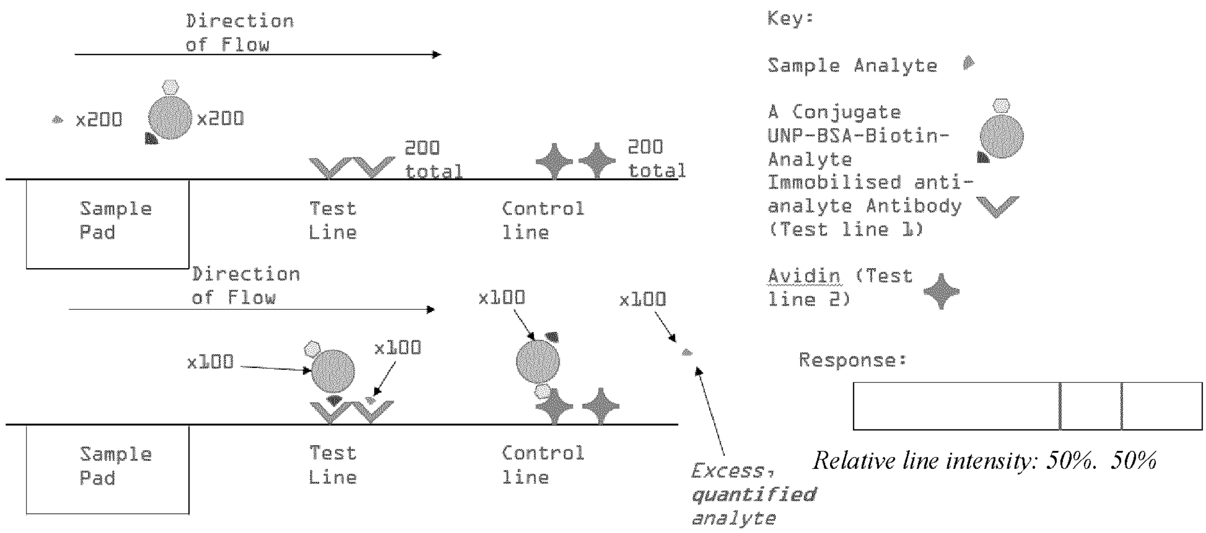
Direction of Flow
x200
x200
200 total
200 total
Sample Pad
Test Line
Control line
Direction of Flow
x100
x100
x100
x100
Sample Pad
Test Line
Control line
Excess, quantified analyte
Key:
Sample Analyte
A Conjugate UNP-BSA-Biotin-Analyte
Immobilised anti-analyte Antibody (Test line 1)
Avidin (Test line 2)
Response:
Relative line intensity: 50%. 50%

Fig. 22

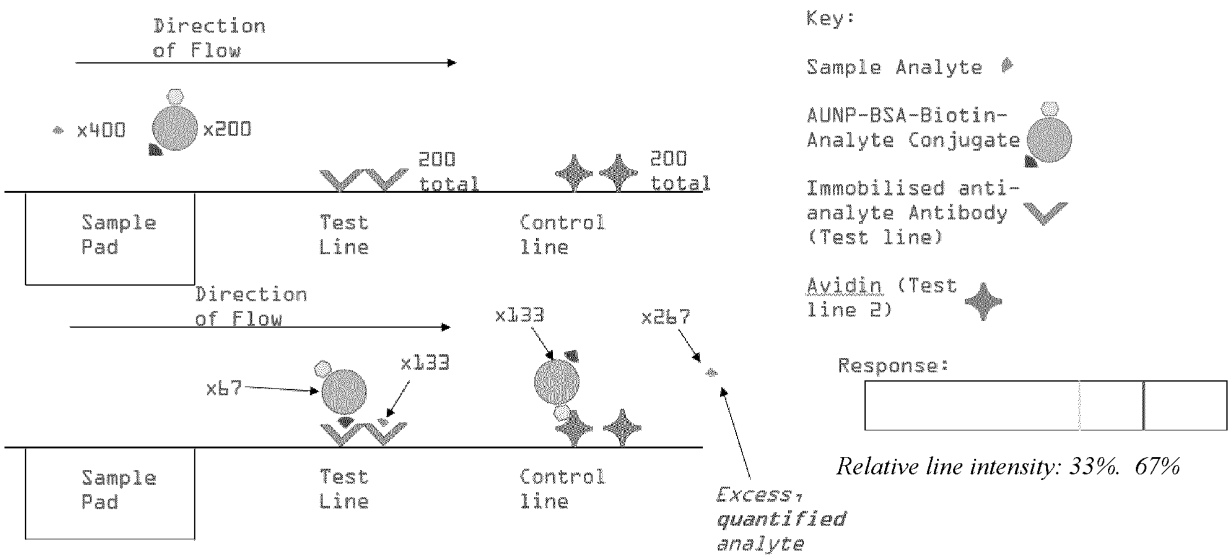
Direction of Flow
x400
x200
200 total
200 total
Sample Pad
Test Line
Control line
Direction of Flow
x133
x267
x133
x67
Sample Pad
Test Line
Control line
Excess, quantified analyte
Key:
Sample Analyte
AUNP-BSA-Biotin-Analyte Conjugate
Immobilised anti-analyte Antibody (Test line)
Avidin (Test line 2)
Response:
Relative line intensity: 33%. 67%

Fig. 23

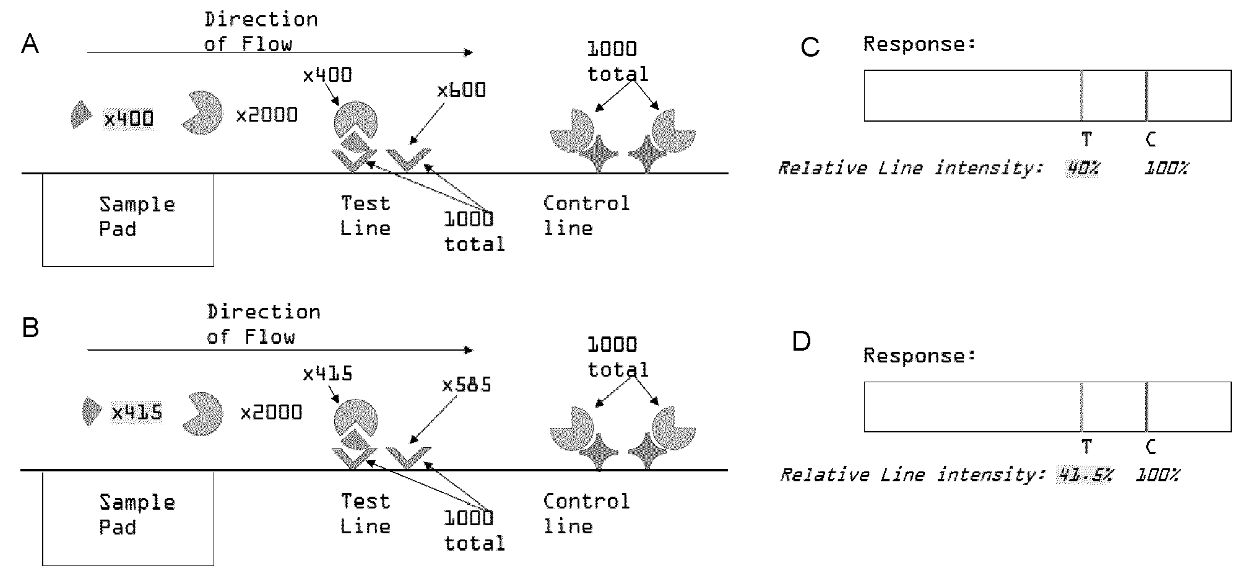
A
Direction of Flow
x400
x2000
x400
x600
1000 total
Sample Pad
Test Line
1000 total
Control line
C
Response:
T
C
Relative Line intensity: 40% 100%
B
Direction of Flow
x415
x2000
x415
x585
1000 total
Sample Pad
Test Line
1000 total
Control line
D
Response:
T
C
Relative Line intensity: 41.5% 100%

Fig. 24

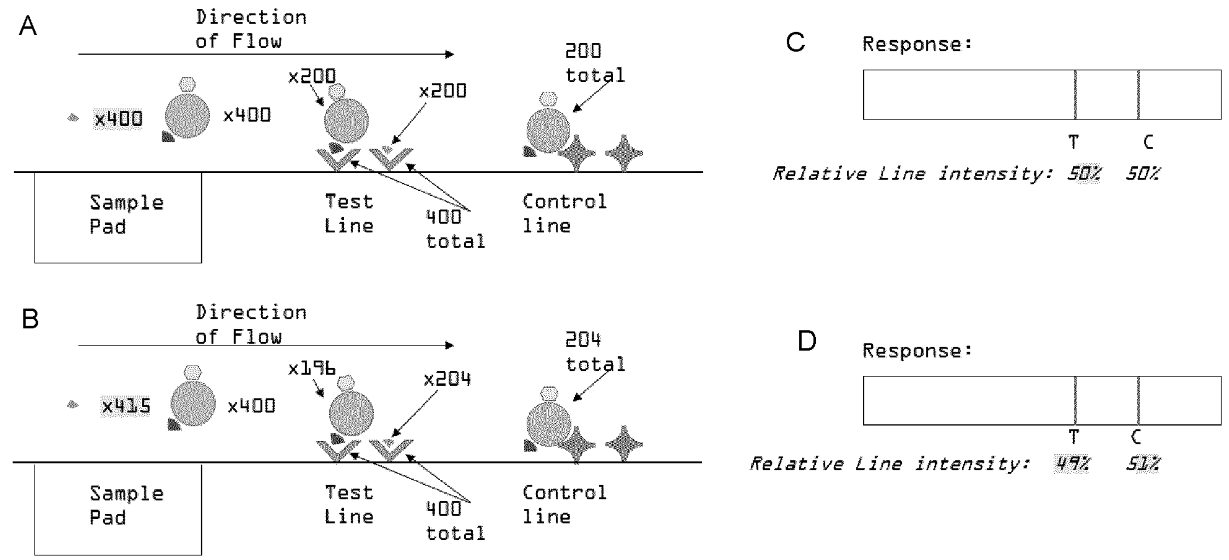
A
Direction of Flow
x400
x400
x200
x200
200 total
Sample Pad
Test Line
400 total
Control line
C
Response:
T
C
Relative Line intensity: 50% 50%
B
Direction of Flow
x415
x400
x196
x204
204 total
Sample Pad
Test Line
400 total
Control line
D
Response:
T
C
Relative Line intensity: 49% 51%

Fig. 25

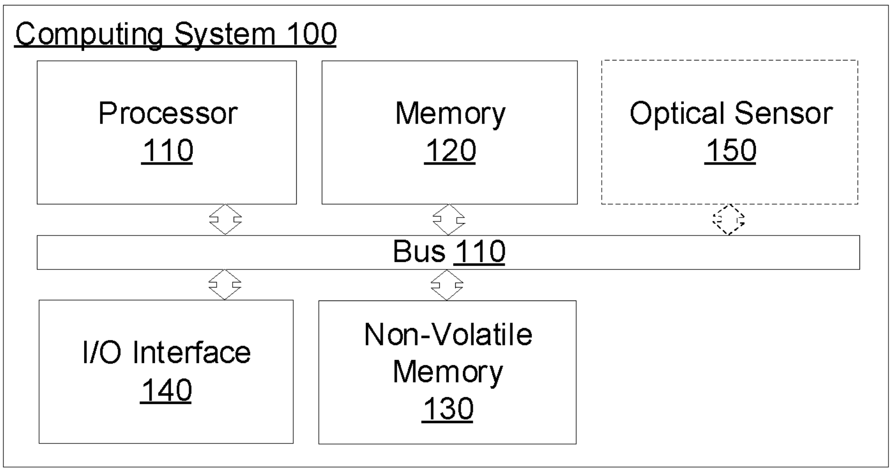
Computing System 100
Processor
110
Memory
120
Optical Sensor
150
Bus 110
I/O Interface
140
Non-Volatile
Memory
130

Fig. 26

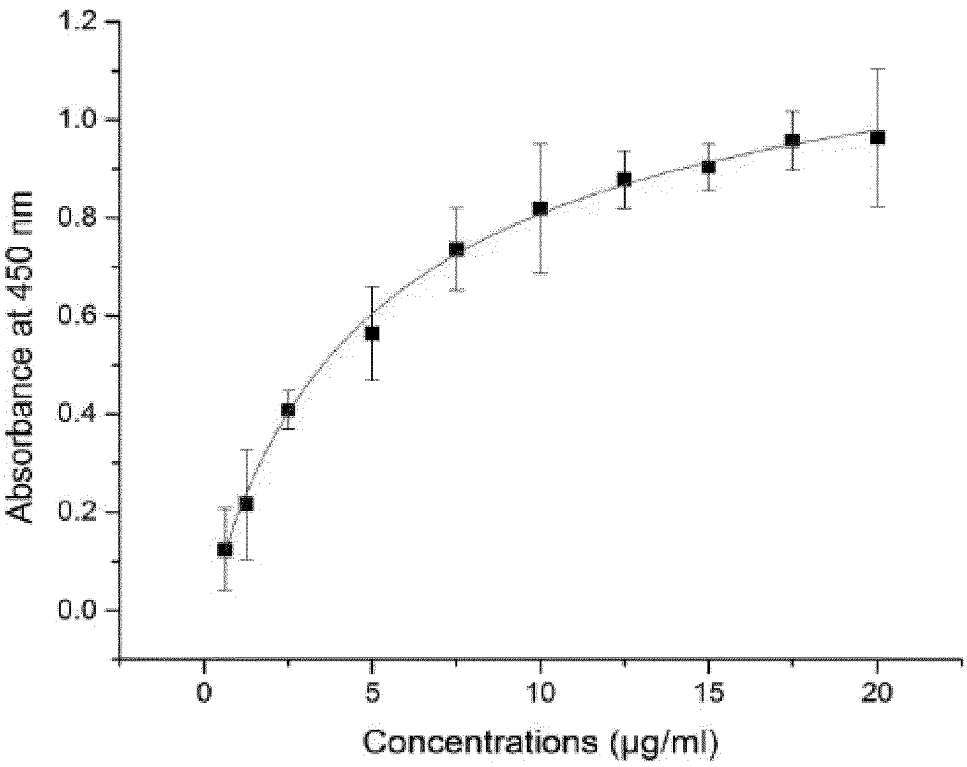
Absorbance at 450 nm
1.2
1.0
0.8
0.6
0.4
0.2
0.0
0
5
10
15
20
Concentrations (μg/ml)

Fig. 27

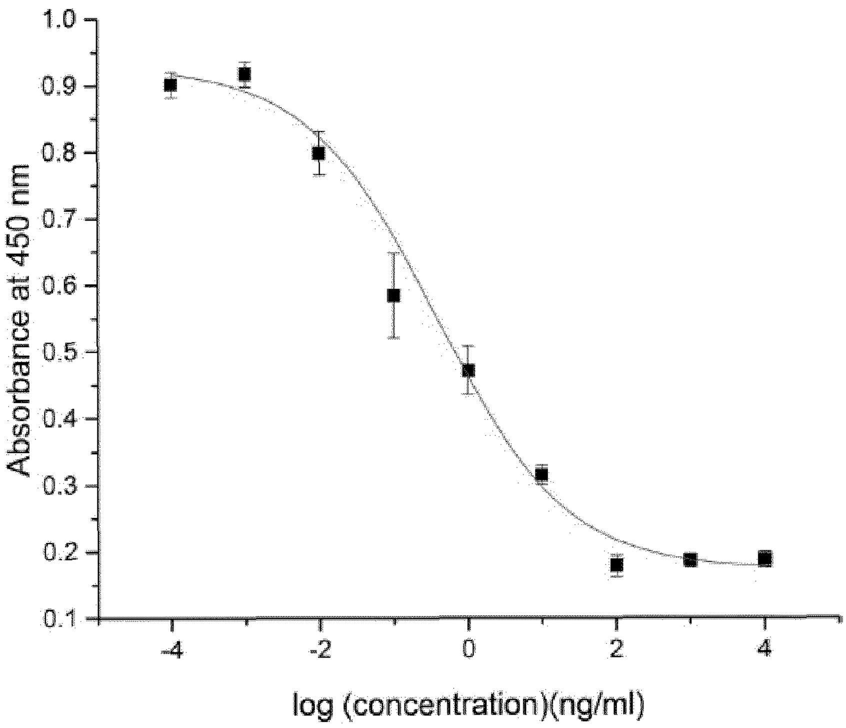
Absorbance at 450 nm
1.0
0.9
0.8
0.7
0.6
0.5
0.4
0.3
0.2
0.1
-4
-2
0
2
4
log (concentration)(ng/ml)

Fig. 28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8137984 B
- US 20220146507 A
- WO 03105899 A
- EP 0203238 A
- WO 2005069007 A1


### Non-patent literature cited in the description


- **DELMULLE et al.** *J. Agric. Food*, 2005, vol. 53, 3364-3368
- Dual recognition element lateral flow assay toward multiplex strain specific influenza virus detection. *Anal. Chem*, 2017, vol. 89, 6781-6786
- **ANG et al.** A colloidal gold-based lateral flow immunoassay for direct determination of haemoglobin Alc in whole blood. *Analytical Methods*, 01 January 2015, vol. 7 (9), 3972-3980
- **WISASTRA et al.** Antibody-free detection of protein tyrosine nitration in tissue sections. *ChemBioChem*, 2011, vol. 12, 2016-2020
- **J. MAJD-ARDEKANI** ; **P. CLOWES** ; **V. MENASH-BONSU** ; **T. O. NUNAN**. *Nucl. Med. Commun*, 2000, vol. 21, 361-364
- **E. REYES** ; **C. Y. LOONG** ; **M. HARBINSON** ; **J. DONOVAN** ; **C. ANAGNOSTOPOULOS** ; **S. R. UNDERWOOD**. *J. Am. Coll. Cardiol.*, 2008, vol. 52, 2008-2016
- **R. B. SASHIDHAR** ; **A. K. CAPOOR** ; **D. RAMANA**. *J. Immunol. Methods*, 1994, vol. 167, 121-127
- **A. AITKEN** ; **M. LEARMONTH**. The Protein Protocols Handbook. Humana Press, 2009
- **B. LAW** ; **W. N. JENNER**. Immunoassay: A Practical Guide. Taylor & Francis, 2005, 16-21
- **D. KIM** ; **A. E. HERR**. *Biomicrofluidics*, 2013, vol. 7, 41501
- **G. T. HERMANSON**. Bioconjugate Techniques. Elsevier, 2013
- **R. LI** ; **Z. WU** ; **Y. WANGB** ; **L. DING** ; **Y. WANG**. *Biotechnol. Reports*, 2016, vol. 9, 46-52
- **R. LEMUS** ; **M. H. KAROL**. Allergy Methods and Protocols. Humana Press, 2008, 167-182
- **J. A. OWEN** ; **K. NAKATSU**. *Clin. Chem.*, 1978, vol. 24, 367-368
- **G. FRENS**. *Nat. Phys. Sci.*, 1973, vol. 241, 20-22
- **J. KIMLING** ; **M. MAIER** ; **B. OKENVE** ; **V. KOTAIDIS** ; **H. BALLOT** ; **A. PLECH**. *J. Phys. Chem. B*, 2006, vol. 110, 15700-15707
- **N. SOSIBO** ; **F. KETER** ; **A. SKEPU** ; **R. TSHIKHUDO** ; **N. REVAPRASADU**. *Nanomaterials*, 2015, vol. 5 (12), 11-1222
- **M. VALCÁRCEL** ; **Á. LÓPEZ-LORENTE**. Gold Nanoparticles in Analytical Chemistry. Elsevier, 2014
- **S. H. BREWER** ; **W. R. GLOMM** ; **M. C. JOHNSON** ; **M. K. KNAG** ; **S. FRANZEN**. *Langmuir*, 2005, vol. 21, 9303-9307
- **C. FANG** ; **Z. CHEN** ; **L. LI** ; **J. XIA**. *J. Pharm. Biomed. Anal.*, 2011, vol. 56, 1035-1040
- **J. KAUR** ; **K. V. SINGH** ; **R. BORO** ; **K. R. THAMPI** ; **M. RAJE** ; **G. C. VARSHNEY** ; **C. R. SURI**. *Environ. Sci. Technol.*, 2007, vol. 41, 5028-5036
- **I. ROMBOUTS** ; **B. LAGRAIN** ; **K. A. SCHERF** ; **M. A. LAMBRECHT** ; **P. KOEHLER** ; **J. A. DELCOUR**. *Sci. Rep*, 2015, vol. 5, 12210
- **C. LAROSA** ; **E. STURA** ; **R. EGGENHÖFFNER** ; **C. NICOLINI**. *Materials (Basel)*, 2009, vol. 2, 1193-1204
- **X. HUANG** ; **M. A. EI-SAYED**. *J. Adv. Res*, 2010, vol. 1, 13-28
- **S. BALOG** ; **L. RODRIGUEZ-LORENZO** ; **C. A. MONNIER** ; **M. OBIOLS-RABASA** ; **B. ROTHEN-RUTISHAUSER** ; **P. SCHURTENBERGER** ; **A. PETRI-FINK**. *Nanoscale*, 2015, vol. 7, 5991-5997
- **S. DOMINGUEZ-MEDINA** ; **S. MCDONOUGH** ; **P. SWANGLAP** ; **C. F. LANDES** ; **S. LINK**. *Langmuir*, 2012, vol. 28, 9131-9139
- **S. LOU** ; **J. YE** ; **K. LI** ; **A. WU**. *Analyst*, 2012, vol. 137, 1174
- **M. SU** ; **C. WANG** ; **C. BAI**. *Chinese Sci. Bull.*, 1998, vol. 43, 1882-1886
- **B. J. FRISKEN**. *Appl. Opt.*, 2001, vol. 40, 4087
- **Y. LI** ; **G. YANG** ; **Z. MEI**. *Acta Pharm. Sin. B*, 2012, vol. 2, 53-59
- **W. HAISS** ; **N. T. K. THANH** ; **J. AVEYARD** ; **D. G. FERNIG**. *Anal. Chem.*, 2007, vol. 79, 4215-4221
- **X. SHI** ; **D. LI** ; **J. XIE** ; **S. WANG** ; **Z. WU** ; **H. CHEN**. *Chinese Sci. Bull*, 2012, vol. 57, 1109-1115

- **W.J. KIM** ; **S.-H. CHOI** ; **Y.-S. RHO** ; **D.-J. YOO**. *Bull. Korean Chem. Soc*, 2011, vol. 32, 4171-4175
- **Y. YANG** ; **S. MATSUBARA** ; **M. NOGAMI** ; **J. SHI**. *Mater. Sci. Eng. B*, 2007, vol. 140, 172-176
- **K. ASLAN** ; **C. C. LUHRS** ; **V. H. PÉREZ-LUNA**. *J. Phys. Chem. B*, 2004, vol. 108, 15631-15639
- **G. A. POSTHUMA-TRUMPIE** ; **J. KORF** ; **A. VAN AMERONGEN**. *Anal. Bioanal. Chem.*, 2008, vol. 393, 569-582
- **B. LUTZ** ; **T. LIANG** ; **E. FU** ; **S. RAMACHANDRAN** ; **P. KAUFFMAN** ; **P. YAGER**. *16th International Conference on Miniaturized Systems for Chemistry and Life Sciences*, 2012, vol. 20, 788-790
- **F. HUA** ; **P. ZHANG** ; **F. ZHANG** ; **Y. ZHAO** ; **C. LI** ; **C. SUN** ; **X. WANG** ; **R. YANG** ; **C. WANG** ; **A. YU**. *Sci. Rep.*, 2015, vol. 5, 17178
- **Y. A. KIM** ; **E. H. LEE** ; **K. O. KIM** ; **Y. T. LEE** ; **B. D. HAMMOCK** ; **H. S. LEE**. *Anal. Chim. Acta*, 2011, vol. 693, 106-113
- **R.-H. SHYU** ; **H.-F. SHYU** ; **H.-W. LIU** ; **S.-S. TANG**. *Toxicon*, 2002, vol. 40, 255-258